# EUROPEAN PATENT APPLICATION

(11) **EP 4 549 468 A2**
(43) Date of publication of application: **07.05.2025**
(21) Application number: 25151971.6
(22) Date of filing: 21.01.2022
(51) Int. Cl.: C07K 16/28

(54) **METHOD**

(30) Priority: 21.01.2021 SG 10202100687X; 25.08.2021 SG 10202109307T
(62) Divisional of application: 22704308.0
(71) Applicant: Agency for Science, Technology and Research, Singapore 138632 (SG); Tan Tock Seng Hospital Pte Ltd, Singapore 308433 (SG)
(72) Inventor: BARD, Frederic, 138673 SINGAPORE (SG); TRAN, Le Son, 138673 SINGAPORE (SG); LEONG, Khai Pang, 308433 SINGAPORE (SG); CHIA, Joanne Zhi Hui, 138632 SINGAPORE (SG)
(74) Representative: Mewburn Ellis LLP

(57) **Abstract**

Disclosed herein are methods and treatments for cartilage degradation, wherein the method comprises administration of a therapeutic agent which inhibits the GalNAc-T activation (GALA) pathway.

## Description

This application claims priority from SG 10202100687X, filed 21 January 2021, and SG 10202109307T, filed 25 August 2021, the contents and elements of which are herein incorporated by reference for all purposes.

### FIELD OF THE INVENTION

The present invention relates generally to the field of molecular biology. In particular, the present invention relates to methods of detecting and treating arthritis.

### BACKGROUND OF THE INVENTION

Arthritis is the leading cause of joint pain and disability affecting more than one in four adults world-wide, with rheumatoid arthritis (RA) and osteoarthritis (OA) being the two most common types of arthritis.

Currently, there is no effective cure for either osteoarthritis or rheumatoid arthritis patients. Rheumatoid arthritis patients are subject to various anti-rheumatic drugs or immunomodulatory agents (eg. anti-TNFa and anti-IL-6) that primarily target the immune system.

A major problem is the immunodeficiency induced by these treatments. In addition, the treatments are usually unable to completely halt disease progression and recurrence. Patients with severe end-stage symptomatic osteoarthritis or rheumatoid arthritis who failed to respond to such drugs will have to undergo joint replacement, which comes with its own risks of serious adverse events.

Thus, there is an unmet need for a method of detecting arthritis and/or treating arthritis in a subject.

### SUMMARY OF THE INVENTION

In one aspect, the present disclosure refers to a method of treating arthritis in a subject, wherein the method comprises administration of a therapeutic agent which inhibits the GalNAc-T activation (GALA) pathway.

In another aspect, the present disclosure refers to a method of detecting the presence or absence of arthritis in a subject, wherein the method comprises the steps of obtaining a synovial fibroblast sample from a subject; detecting the level of Tn antigen/Tn glycan in the sample obtained in step (i); comparing the level of Tn antigen/Tn glycan in step (ii) with the level of Tn antigen/Tn glycan in a synovial fibroblast sample from a control group; wherein an increase in the level of Tn antigen/Tn glycan present in the sample compared to the control group is indicative of the presence of arthritis; wherein the control group consists of arthritis-free subjects.

In one aspect, the present disclosure refers to the use of an anti-calnexin (anti-CNX) antibody for the manufacture of a medicament for the treatment or prevention of cartilage degradation in a subject.

In another aspect, the present disclosure refers to an anti-calnexin (anti-CNX) antibody for use in the treatment or prevention of cartilage degradation in a subject

In another aspect, the present disclosure refers to a method of treating or preventing cartilage degradation, wherein the method involves the administration of an anti-calnexin (anti-CNX) antibody to a subject.

In some aspects, the subject has osteoarthritis, rheumatoid arthritis, psoriasis arthritis, juvenile idiopathic arthritis (JIA), arthritic flares, bursitis, gout, chondrocalcinosis, fibromyalgia, costochondritis, cartilage damage, or polychondritis.

In some aspects, the subject has arthritis.

In some aspects, the subject has osteoarthritis or rheumatoid arthritis.

In some aspects, the cartilage degradation is in one or more joints.

In some aspects, the cartilage degradation is characterised by increased O-glycosylation and/or GALNT Activation (GALA) in joint tissues, cartilage tissue, and/or synovial fibroblasts.

In some aspects, the cartilage degradation is characterised by increased O-glycosylation of calnexin in joint tissues, cartilage tissue, and/or synovial fibroblasts.

In some aspects, the cartilage degradation is characterised by increased cell surface expression of calnexin in joint tissues, cartilage tissue, and/or synovial fibroblasts.

In some aspects, the cartilage degradation is characterised by extracellular matrix (ECM) degradation.

In some aspects, the cartilage degradation is mediated by the activity of synovial fibroblasts.

In some aspects, the antibody against Cnx is capable of reducing:
(a) ECM degradation; or
(b) an oxireductase activity; or
(c) a disulphide bond reductase activity.

In some aspects, the antibody is capable of reducing cartilage degradation.

In some aspects, the antibody is capable of reducing ECM degradation.

In some aspects, the antibody is capable of reducing an ECM degradation activity.

In some aspects, the antibody is capable of reducing an ECM degradation activity of CNX:PDIA3 (also known as Cnx/ERp57).

In some aspects, the antibody is capable of reducing an ECM degradation activity of CNX.

In some aspects, the antibody is capable of reducing an ECM degradation activity of fibroblasts.

In some aspects, the antibody is capable of reducing an ECM degradation activity of synovial fibroblasts.

In some aspects, the antibody is capable of reducing an oxireductase activity.

In some aspects, the antibody is capable of reducing an oxireductase activity of CNX:PDIA3 (also known as Cnx/ERp57).

In some aspects, the antibody is capable of reducing an oxireductase activity of CNX.

In some aspects, the antibody is capable of reducing disulphide bond reductase activity.

In some aspects, the antibody is capable of reducing a disulphide bond reductase activity of CNX:PDIA3 (also known as Cnx/ERp57).

In some aspects, the antibody is capable of reducing a disulphide bond reductase activity of CNX.

In some aspects, the antibody is capable of reducing O-glycosylation.

In some aspects, the antibody is capable of reducing O-glycosylation of CNX.

In some aspects, the antibody is capable of reducing glycosylation of CNX.

In some aspects, the antibody is capable of reducing GALA mediated O-glycosylation of CNX.

In some aspects, the antibody is capable of reducing CNX activity.

In some aspects, the antibody is capable of reducing PDIA3 activity.

In some aspects, the antibody is capable of reducing PDIA4 activity.

In some aspects, the antibody is capable of reducing the number or proportion of cells expressing CNX.

In some aspects, the antibody is capable of reducing the number or proportion of cells expressing PDIA3.

In some aspects, the antibody is capable of reducing the number or proportion of cells expressing PDIA4.

In some aspects, the antibody is capable of reducing the number or proportion of cells expressing CNX:PDIA3.

In some aspects, the antibody is antagonistic.

In some aspects, the antibody is monoclonal.

In some aspects, the therapeutic agent is to be administered together, separately, or sequentially with a further therapeutic agent, wherein the further therapeutic agent is an anti-rheumatic drug.

In some aspects, the method comprises intravenous, subcutaneous or intraperitoneal administration of the anti-cnx antibody.

In some aspects, the treatment or prevention is in a human subject.

In some aspects, a subject is determined to be suitable for treatment with the anti-cnx antibody through the following steps:
i. detecting the level of Tn antigen, Tn glycan, CNX cell surface expression, CNX glycosylation, and/or ECM degradation in a synovial fibroblast sample;
ii. comparing the level of Tn antigen, Tn glycan, CNX cell surface expression, CNX glycosylation, and/or ECM degradation in step (i) with the level of Tn antigen/Tn glycan in a synovial fibroblast sample from a control group;
wherein an increase in the level of Tn antigen, Tn glycan, CNX expression, CNX glycosylation, and/or ECM degradation present in the sample compared to the control group is indicative of the suitability for treatment.

In one aspect there is a method provided for detecting the presence or absence of a disorder associated with cartilage degradation in a subject, wherein the method comprises the steps of:
i. detecting the level of Tn antigen, Tn glycan, CNX cell surface expression, CNX glycosylation, and/or ECM degradation in a synovial fibroblast sample;
ii. comparing the level of Tn antigen, Tn glycan, CNX cell surface expression, CNX glycosylation, and/or ECM degradation in step (i) with the level of Tn antigen/Tn glycan in a synovial fibroblast sample from a control group;
wherein an increase in the level of Tn antigen, Tn glycan, CNX expression, CNX glycosylation, and/or ECM degradation present in the sample compared to the control group is indicative of the suitability for treatment.

In some aspects, the method is for detecting the presence or absence of arthritis in a subject. In some aspects, the method is for detecting the presence or absence of osteoarthritis or rheumatoid arthritis in a subject.

***Numbered statements***
1. A method of treating arthritis in a subject, wherein the method comprises administration of a therapeutic agent which inhibits the GalNAc-T activation (GALA) pathway.
2. The method of statement 1, wherein the therapeutic agent inhibits any one or more of the cell surface markers involved in the GalNAc-T activation (GALA) pathway.
3. The method of any one of the preceding statements, wherein the therapeutic agent inhibits one or more of the following targets of the GalNAc-T activation (GALA) pathway selected from the group consisting of Calnexin (CNX), Protein disulfide-isomerase A4 (PDIA4), Protein disulfide-isomerase A3 (PDIA3), a Calnexin-PDIA3 complex, and O-glycosylated matrix metalloproteinase-14 (MMP14).
4. The method of statement 3, wherein the target of the GALA pathway is Calnexin (CNX) and/or Protein disulfide-isomerase A3 (PDIA3).
5. The method of any one of the preceding statements, wherein the therapeutic agent is selected from the group consisting of an antibody, a gene, a fusion protein, and an expression vector.
6. The method of statement 5, wherein the therapeutic agent is to be administered together, separately or sequentially with a further therapeutic agent, wherein the further therapeutic agent is an anti-rheumatic drug.
7. The method of statement 5, wherein the fusion protein is ER-2Lec.
8. The method of statement 5, wherein the antibody is an anti-Calnexin antibody
9. The method of statement 5, wherein the expression vector is an Adeno-associated virus (AAV) vector.
10. The method of statement 5 or 9, wherein the expression vector expresses the protein ER-2Lec.
11. The method of any one of the preceding statements, wherein one or more of the therapeutic agents are to be administered as a gene therapy.
12. A method of detecting the presence or absence of arthritis in a subject, wherein the method comprises the steps of:
   (i) obtaining a synovial fibroblast sample from a subject;
   (ii) detecting the level of Tn antigen/Tn glycan in the sample obtained in step (i);
   (iii) comparing the level of Tn antigen/Tn glycan in step (ii) with the level of Tn antigen/Tnglycan in a synovial fibroblast sample from a control group;
   wherein an increase in the level of Tn antigen/Tn glycan present in the sample compared to the control group is indicative of the presence of arthritis; wherein the control group consists of arthritis-free subjects.
13. The method of any one of the preceding statements, wherein the arthritis is selected from the group consisting of rheumatoid arthritis, osteoarthritis, psoriasis arthritis, juvenile idiopathic arthritis (JIA), and arthritic flares/flare up.

### BRIEF DESCRIPTION OF THE DRAWINGS

The invention will be better understood with reference to the detailed description when considered in conjunction with the non-limiting examples and the accompanying drawings, in which:

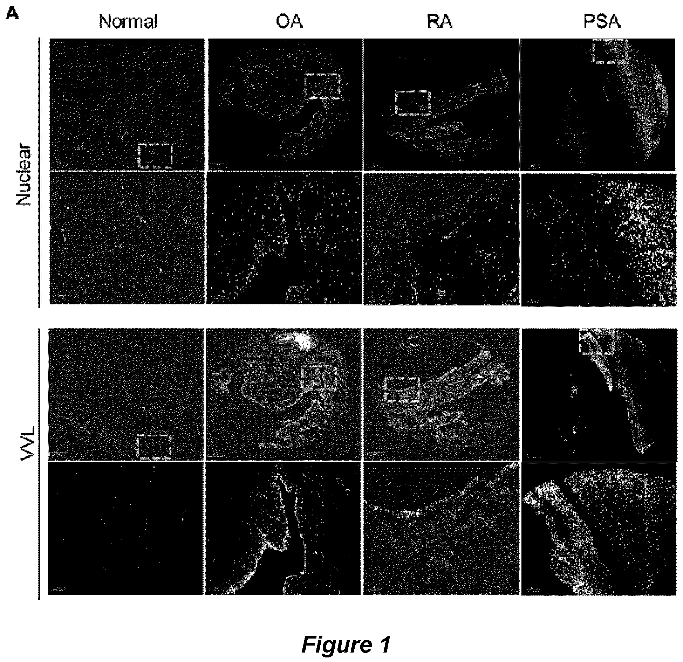

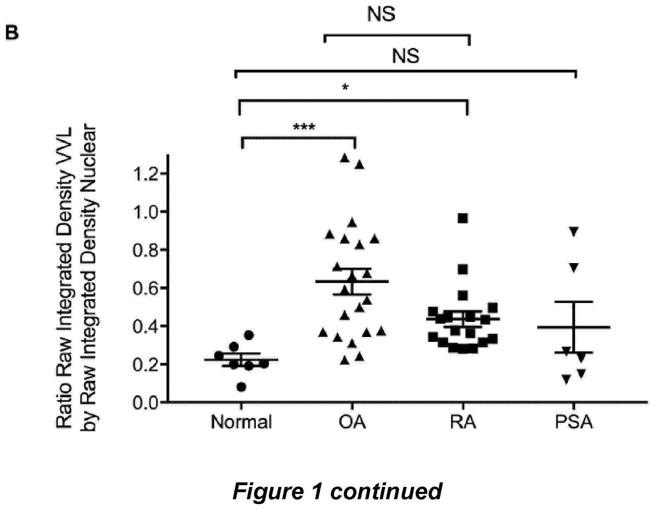

**Fig. 1** shows that O-glycosylation is enhanced in human samples of rheumatoid arthritis and osteoarthritis, indicating that high levels of O-glycosylation correlate with a diseased state. Panel A shows representative images of immunohistofluorescence staining of nuclei with Hoechst (upper panel) and O-GalNAc glycans (Tn glycans) stained with *Vicia Villosa* lectin (VVL, lower panel) on human tissue microarray (TMA) containing joint tissues from healthy subjects (Normal) and patients with osteoarthritis (OA), rheumatoid arthritis (RA) or Psoriasis Arthritis (PSA). Scale bar, 5 µm. Panel B shows a quantification graph of Tn glycan levels in individual tissue cores. Osteoarthritis patients displayed higher O-GalNAc glycan levels than healthy subjects, while most rheumatoid arthritis patients and two Psoriasis arthritis patients showed higher O-GalNAc glycan levels than healthy individuals. Data are mean ± SEM and combined from two different tissue microarray (TMA) slides consisting of tissue sections from 21 osteoarthritis patients, 18 rheumatoid arthritis patients, 6 psoriasis arthritis patients and 7 healthy subjects. Individual data points represent the raw integrated density of Vicia Villosa lectin (VVL) staining normalized to that of nuclear staining of individual subjects. Box and whisker plots show all values, boxes extend from the 25^{th} to 75^{th} percentiles, and error bars span max to min values *, p < 0.05, ****, p < 0.0001, NS: not significant (one-way ANOVA, Kruskal-Wallis test).

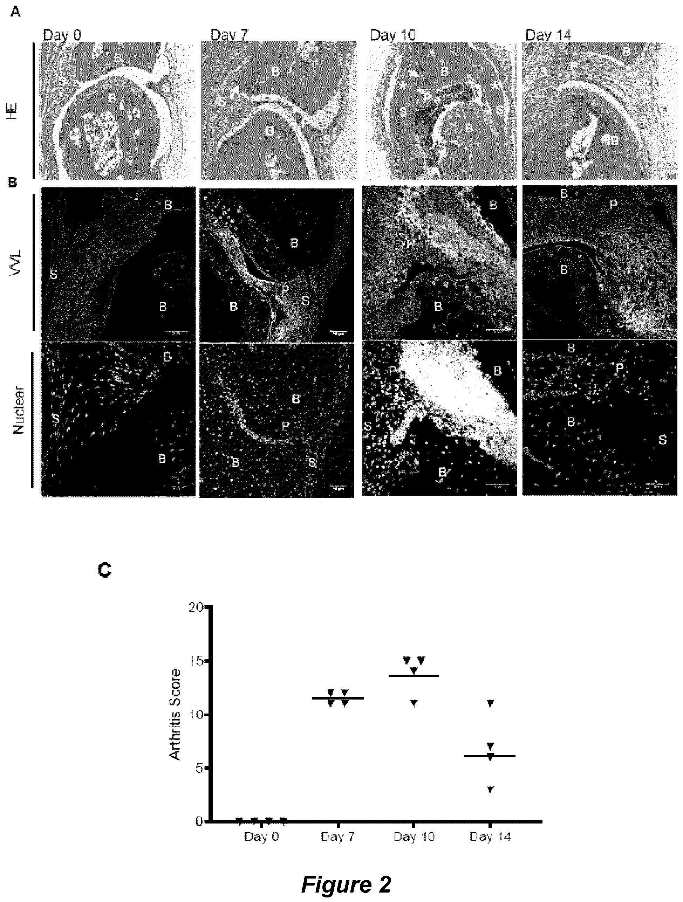

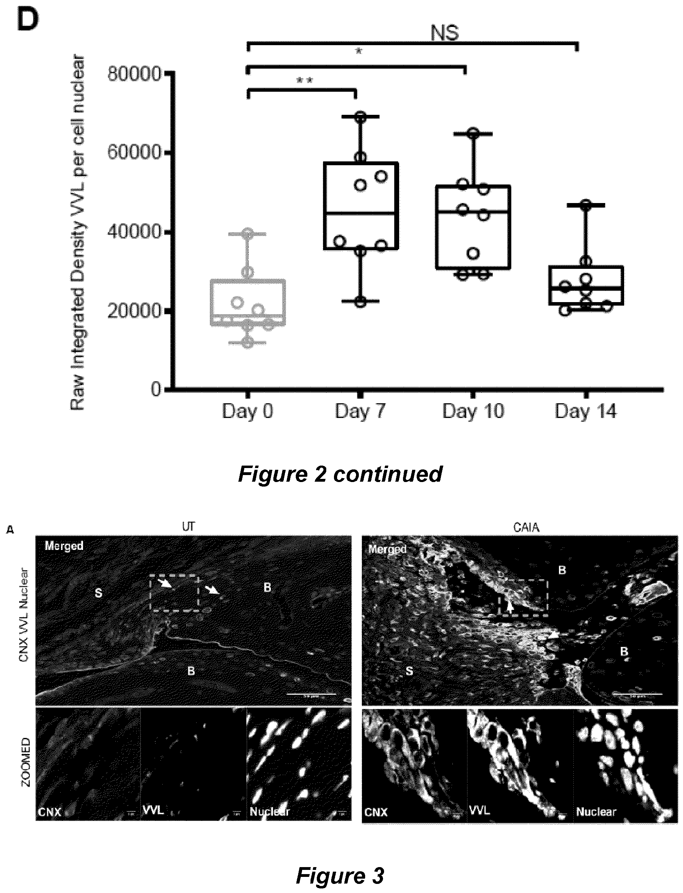

**Fig. 2** shows that Tn glycan levels are enhanced in arthritis induced mice and correlated with disease severity. This indicates that high Tn glycan levels correlate with a diseased state. Panel A shows the results of haematoxylin and eosin (HE) histology of synovial tissues obtained from control mice (day 0) or those injected with Collagen type II antibody induced arthritis (CAIA) at day 7, 10 and 14. S: synovium; B: bone, P: pannus, (*): infiltration of immune cells in the synovial sub-lining, arrows: bone erosion. Panel B shows representative immunofluorescence staining images with *Vicia Villosa* lectin (VVL; upper panel) and nuclei (lower panel) showing the time-course dependent increase of VVL staining in the pannus tissues of CAIA mice from day 7 to day 10. Scale bar, 50 µm. Panels C and D show the results of evaluation of clinical scores (C) and quantification of total Tn levels in the synovium (D) of CAIA mice from day 0 to day 14. In panel C, data are mean of arthritic scores of 4 mice per time point. In panel D, individual data points represent mean Tn levels of individual joints, two joints on the front paws of each animal are computed. Box and whisker plots show all values, boxes extend from the 25^{th} to 75^{th} percentiles, and error bars span max to min values. *, p < 0.05; **, p<0.01; NS, not significant (one-way ANOVA).

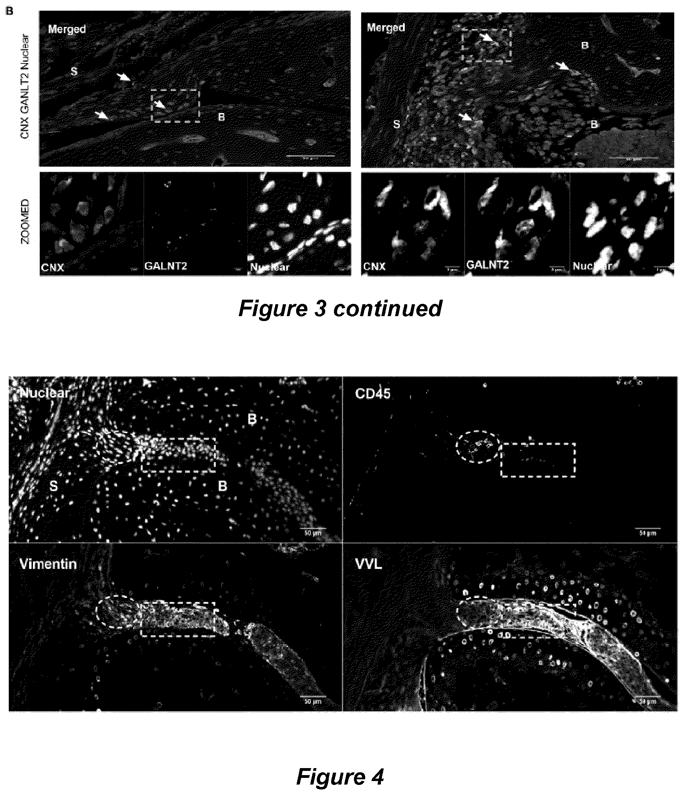

**Fig. 3** shows data indicating that synovial cells in collagen type II antibody induced arthritis (CAIA) mice display signs of GALA pathway activation. This indicates in a diseased model, the GALA pathway is active in synovial cells, thereby making the GALA pathway a therapeutic target. Panels A and B show images of co-staining of *Vicia Villosa* lectin (VVL; A) or GALNT2 (B) with endoplasmic reticulum (ER) resident protein Calnexin (CNX), showing markedly increased Tn glycan levels in the synovium of arthritis induced mice. Scale bar 50 µm. Zoomed images show VVL staining or GALNT2 enzymes co-localized with CNX in arthritis joints, indicating the GALA activation state. Zoom scale 4x; S: synovial membrane; B: bone; arrows show either Golgi (in untreated mice) or ER staining patterns (in CAIA mice) of VVL or GALNT2.
**Fig. 4** shows images showing that synovial fibroblasts (SF) are the major cell type displaying GALA activation in Collagen type II antibody induced arthritis (CAIA) mice, indicating that in a diseased model, the GALA pathway is active in synovial cells and thereby making the GALA pathway a therapeutic target. Co-staining of *Vicia Villosa* lectin (bottom right panel) with fibroblast marker vimentin (bottom left panel), immune cell marker anti-CD45 (top right panel) and nuclei (top left panel) shows relative distribution of immune cells (circles), fibroblasts (rectangles) in the pannus tissues of CAIA mice and their Tn expression levels. Scale bar, 50 µm.

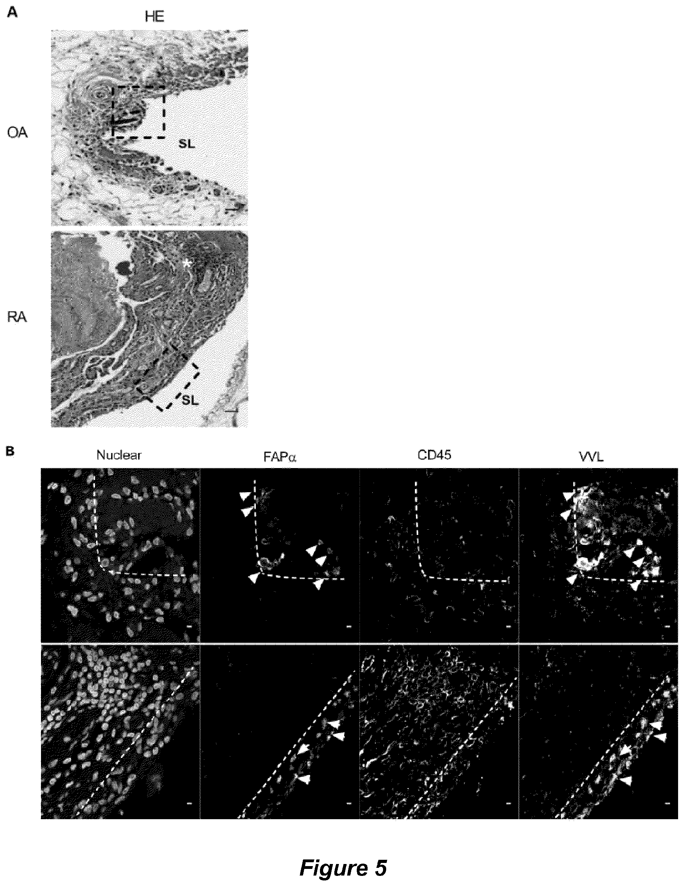

**Fig. 5** shows images showing that synovial lining fibroblasts from both osteoarthritis and rheumatoid arthritis patients display strong GALA activation, indicating that in a diseased model, the GALA pathway is active in synovial cells and thereby making the GALA pathway a therapeutic target. Panel A shows the results of haematoxylin and eosin (H&E) histology of synovial tissues obtained from rheumatoid arthritis and osteoarthritis patients. "*" indicates Infiltration of immune cells in the sub-lining of RA synovium; SL: synovial lining. Scale bar, 100 µm. Panel B shows representative immunofluorescence images of osteoarthritis (top panel) and rheumatoid arthritis (bottom panel) synovium showing strong Tn glycan levels in the lining synovial fibroblasts (SF), as identified by FAPα (arrowheads) and sparse Tn staining in immune cells identified by CD45 in the sub-lining (divided by a dashed line). Scale bar, 50 µm.

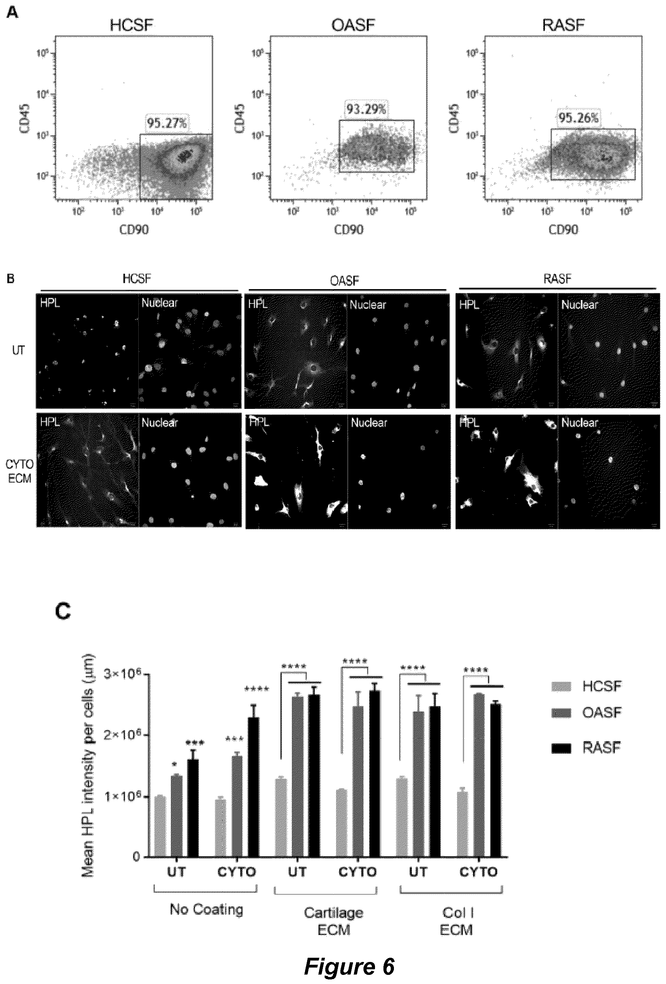

**Fig. 6** shows data indicating that primary synovial fibroblasts (SF) from osteoarthritis and rheumatoid arthritis patients induces strong GALA activation in response to stimulation with arthritis-driving cytokines and cartilage extracellular matrix (ECM). This shows that the GALA pathway is responsible for the symptoms and disease progression of arthritis. Panel A shows fluorescence-activated cell sorting (FACS) dot plots showing high purity (>90%) of primary synovial fibroblasts cultures established from healthy subjects (HCSF), osteoarthritis (OASF) and rheumatoid arthritis (RASF) patients. Panel B shows representative images of *Helix pomatia* lectin (HPL) staining show higher Tn glycan levels in both osteoarthritis synovial fibroblasts and rheumatoid arthritis compared synovial fibroblasts to HCSF under basal conditions. The magnitudes are accelerated after priming those cells with arthritis driven cytokines including IL1β and TNFα (CYTO) and cartilage extracellular matrix. Scare bar, 20 µm. Panel C shows the results of the quantification of *Helix pomatia* lectin (HPL) levels in HCSF, OASF and RASF under basal conditions and stimulation with CYTO only (no coating) or in combination with cartilage extracellular matrix or collagen type I extracellular matrix. Data are mean ± SEM of two 2 independent experiments. *p < 0.05, ***< 0.001, ****p < 0.0001, NS: not significant (Two-way ANOVA).

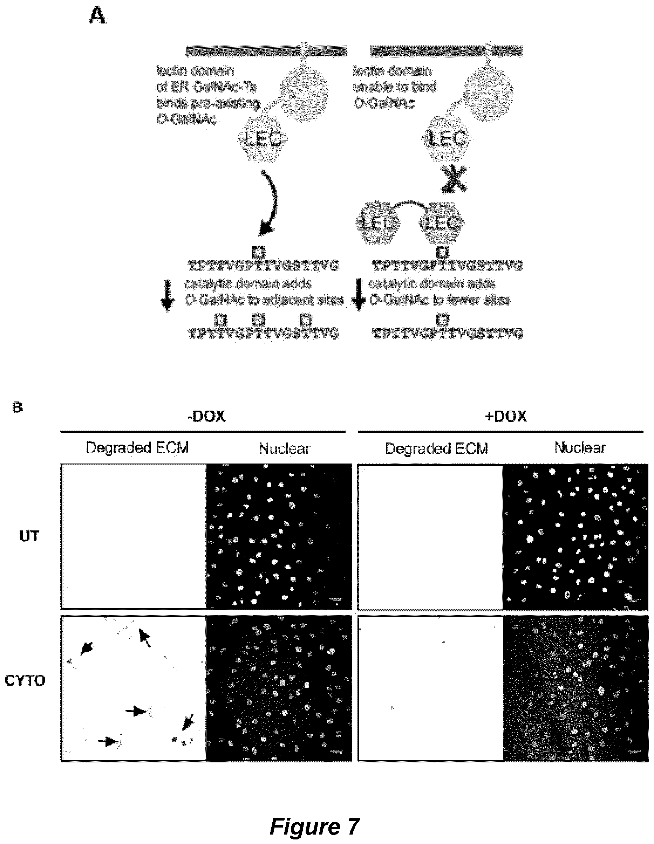

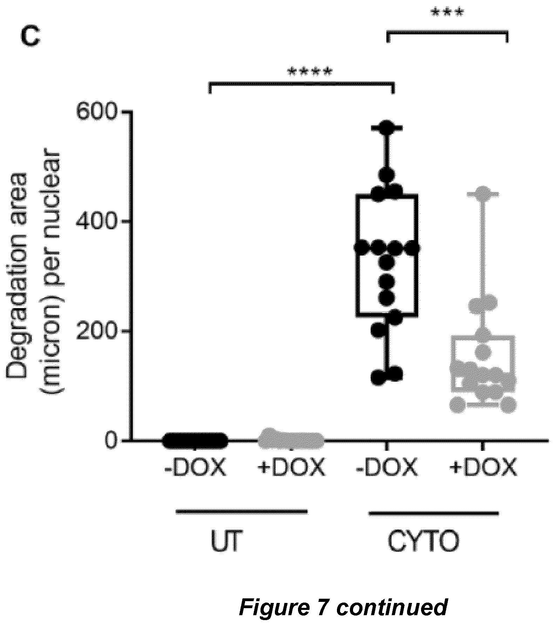

**Fig. 7** shows data indicating that GALA activation in synovial fibroblasts drives cartilage extracellular matrix degradation, showing that the GALA pathway is responsible for the symptoms and disease progression of arthritis. Panel A shows a strategy to inhibit GALA in synovial fibroblasts by stably transfecting SW982 synovial fibroblasts with a construct expressing doxycycline (DOX) inducible 2 lectin domains of GALNT2 in the ER (ER-2Lec). Panel B shows representative images of the results of matrix degradation activity of SW982 cells and ER-2Lec expressing SW982 cells after stimulation with rheumatoid arthritis associated cytokines (CYTO; IL1β and TNFα). Arrows show degraded matrices. Panel C shows a quantification graph showing decreased matrix degradation activity in GALA inhibited SW982 cells after stimulation with CYTO (IL1β and TNFα). Data correspond to the mean ± SEM and are representative for three independent experiments. Each data point represents the total degraded area (µm) per nuclear per well. ***, p<0.001, ****p < 0.0001 (One-way ANOVA).

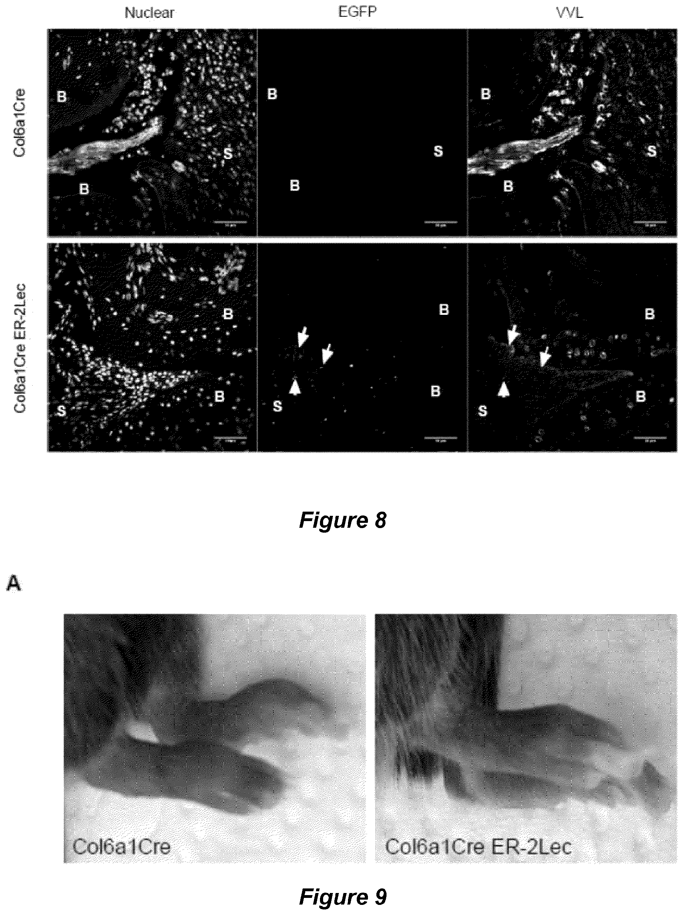

**Fig. 8** shows data indicating that ER-2Lec expression in synovial fibroblasts *in vivo* suppresses GALA activation, showing the efficacy of ER-2Lec expression in treating arthritis or alleviating a symptoms associated with arthritis. Representative images are shown indicating that ER-2Lec is mainly expressed in synovial fibroblasts identified by EGFP positive stains (arrowheads) in the synovium of Col6a1Cre ER-2Lec mice and that such cells display reduced VVL staining, indicating the inhibition of GALA. In other words, Panel B shows the results of VVL staining showing loss of O-GalNAc glycans in fibroblasts (EGFP positive cells) of GALA inhibition joints after arthritis induction. B: bone, S: synovium. Scale bar, 50 µm.

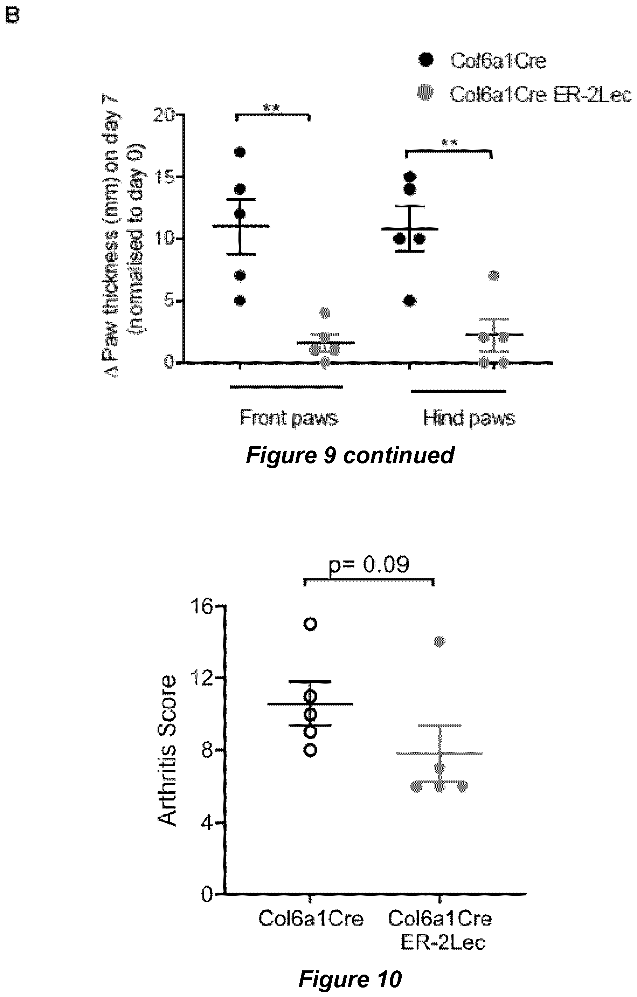

**Fig. 9** shows images (panel A) and graphs (panel B) illustrating the effect of GALA inhibition by ER-2Lec expression in reducing paw swelling in CAIA mice, showing the efficacy of ER-2Lec expression in treating arthritis or alleviating symptoms associated with arthritis. Paw thickness analysis shows alleviated swelling levels on both front and hind paws of Col6a1Cre ER-2Lec mice at day 7 post arthritis induction as compared to Col6a1Cre control mice. Data are mean ± SEM of two 2 independent experiments, n=5 mice per group. **, p<0.01 (one way ANOVA).
**Fig. 10** shows data indicating that GALA inhibition by ER-2Lec expression in synovial fibroblasts (SF) reduces clinical scores in CAIA mice, which in turn indicates that ER-2Lec expression in effective in treating arthritis on a clinical scale. Measurement of clinical scores shows reduced arthritis severity in Col6a1Cre ER-2Lec mice at day 7 post arthritis induction as compared to Col6a1Cre control mice. Data are mean ± SEM of two 2 independent experiments, n=5 mice per group. P= 0.09 (non-parametric t test, Mann-Whitney test).

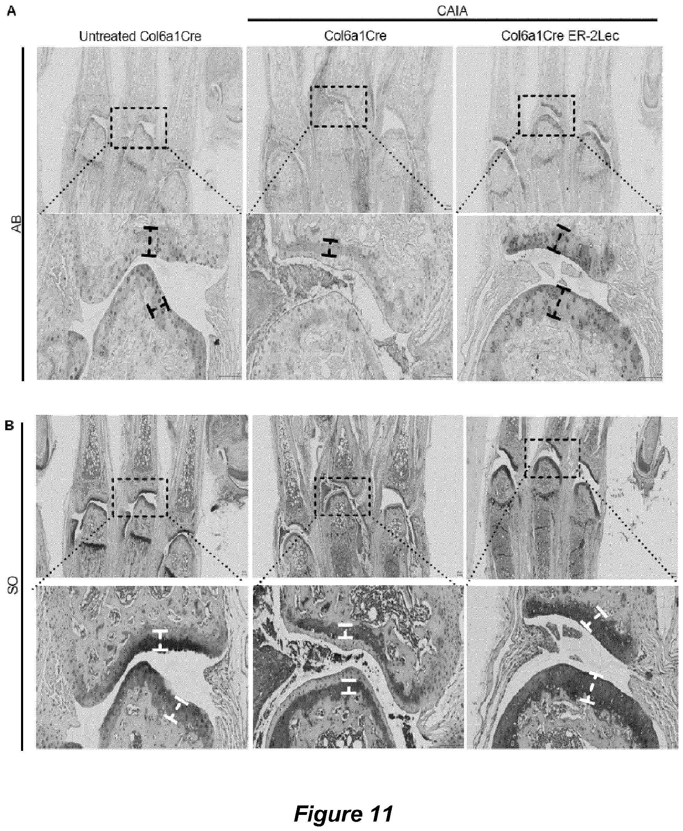

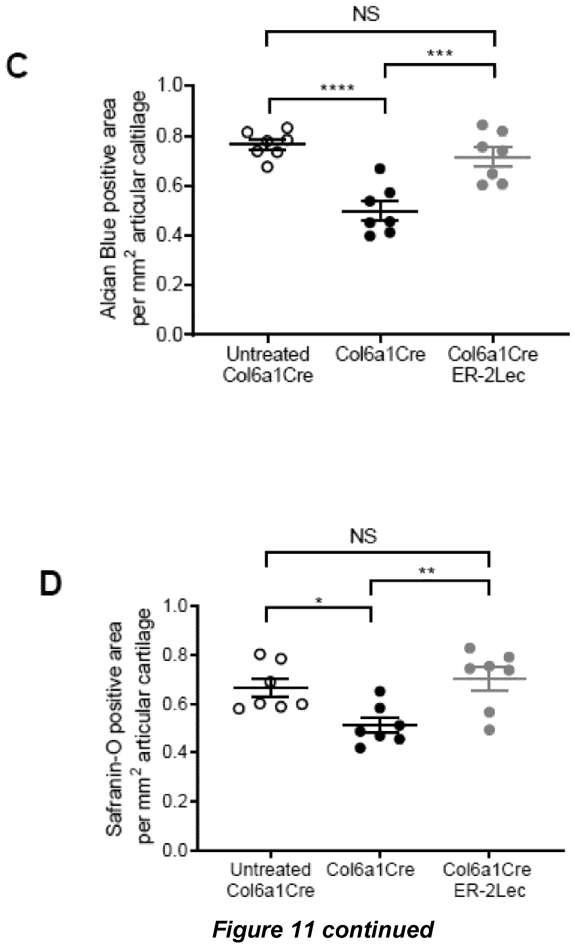

**Fig. 11** shows images and data indicating that GALA inhibition by ER-2Lec expression in synovial fibroblasts protects CAIA mice from cartilage degradation, showing the efficacy of ER-2Lec expression in treating arthritis or alleviating symptoms associated with arthritis. Panels A and B show representative images showing Alcian blue (AB) (A) and Safranin-O (SO) (B) staining in untreated Col6a1Cre mice or arthritis induced Col6a1Cre and Col6a1Cre ER-2Lec mice at day 7. Panels C and D show results of the quantification of positive AB (C) and SO (D) staining areas (scale bars). The data showed that arthritis induced cartilage matrix degradation is restored in GALA inhibition mice (Col6aCre ER-2Lec). Each data point represents an average of positive staining area per mm2 articular cartilage from three different metacarpophalangeal joints of one animal. Data are shown as mean ± SEM. *, p<0.05, **, p<0.01; **, p<0.001 (one way ANOVA test).

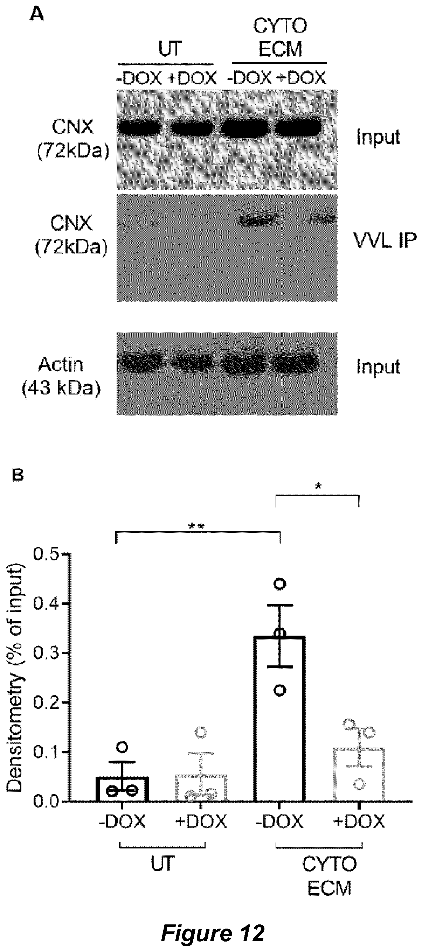

**Fig. 12** shows data indicating that GALA activates O-glycosylation of Calnexin (CNX) in arthritis-primed synovial fibroblasts. This shows the effect of the GALA pathway in the diseased state, and identifies Calnexin as a therapeutic target. Panel A and B shows the blot results of co-immunoprecipitation using VVL lectin (A) and a quantification graph (B) showing that levels of O-GalNAc glycosylated Calnexin (CNX) are enhanced in SW982 cells after stimulation with arthritis associated cytokines (CYTO) and cartilage extracellular matrix (ECM) but reduced in doxycycline- (DOX) inducible ER-2Lec expressing SW982 cells. Actin was used as a loading control. In Panel B, data are shown as mean ± SEM and representative of 3 independent experiments. *, p<0.05; **, p<0.01 (one way ANOVA test).

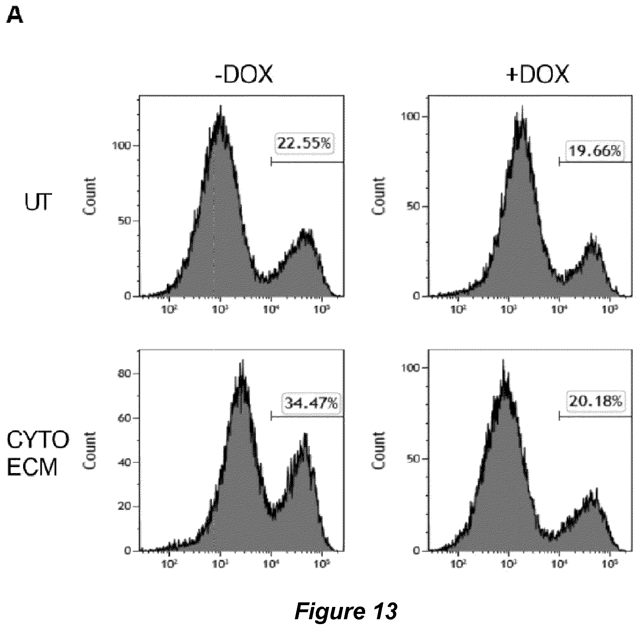

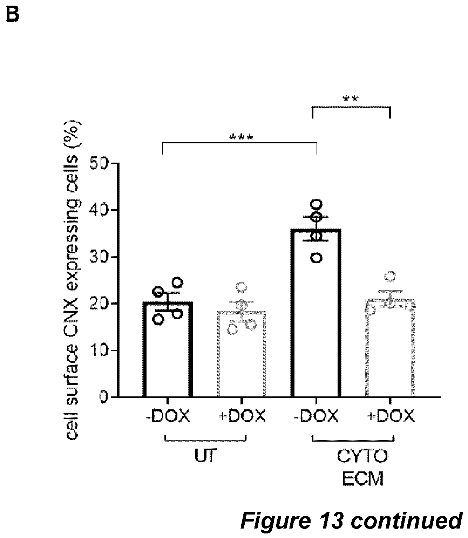

**Fig. 13** shows data indicating that GALA induces cell surface exposure of Calnexin (CNX) in arthritic-primed synovial fibroblasts. This shows the effect of the GALA pathway in the diseased state, and identifies Calnexin as a therapeutic target. Panels A and B show flow cytometry histogram plots (A) and a quantification graph (B) showing an increased proportion of synovial fibroblasts cells expressing surface Calnexin (CNX) after stimulation with arthritis induced cytokines (CYTO) and cartilage extracellular matrix (ECM). The induction of cell surface CNX is diminished in ER-2Lec expressing SF cells. In B, data are shown as mean ± SEM and representative of 2 independent experiments. **, p<0.01; ***, p<0.001 (one-way ANOVA test).

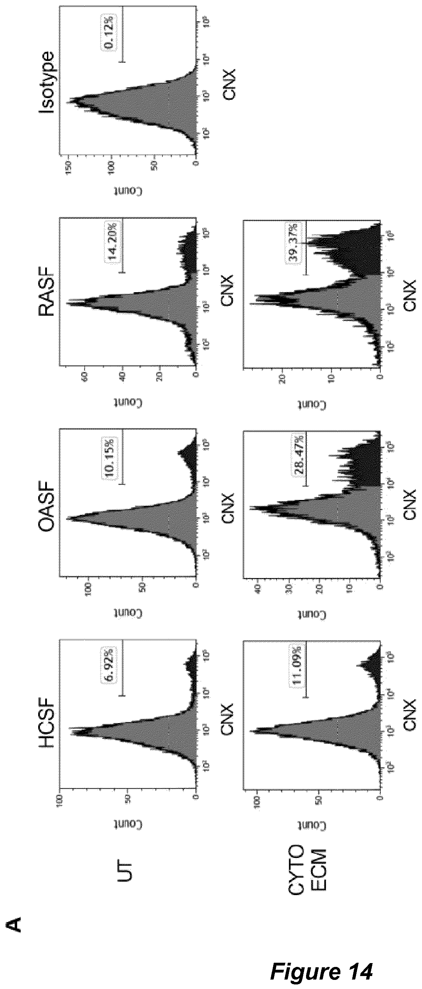

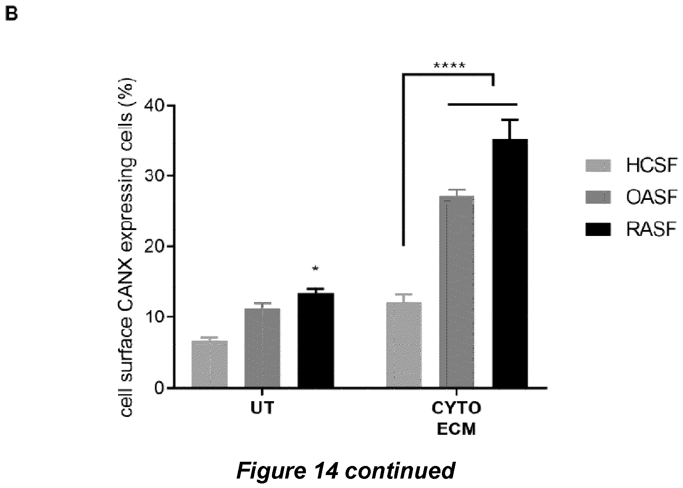

**Fig. 14** shows data indicating that Calnexin (CNX) surface exposure is enhanced in primary synovial fibroblasts from both osteoarthritis (OA) and rheumatoid arthritis (RA) patients, thus identifying Calnexin as a therapeutic target in the treatment of arthritis. Panels A and B show flow cytometry histogram plots (A) and a quantification graph (B) showing higher proportions of osteoarthritis synovial fibroblasts (OASF) or rheumatoid arthritis synovial fibroblasts (RASF) cells positive for surface CNX compared to that of healthy control synovial fibroblasts (HCSF) under basal condition or stimulation with arthritis driven cytokines (CYTO) and cartilage extracellular matrix (ECM). In Panel B, data are shown as mean ± SEM and representative of 2 independent experiments. *, p<0.05; ***, p<0.001 (one-way ANOVA test).

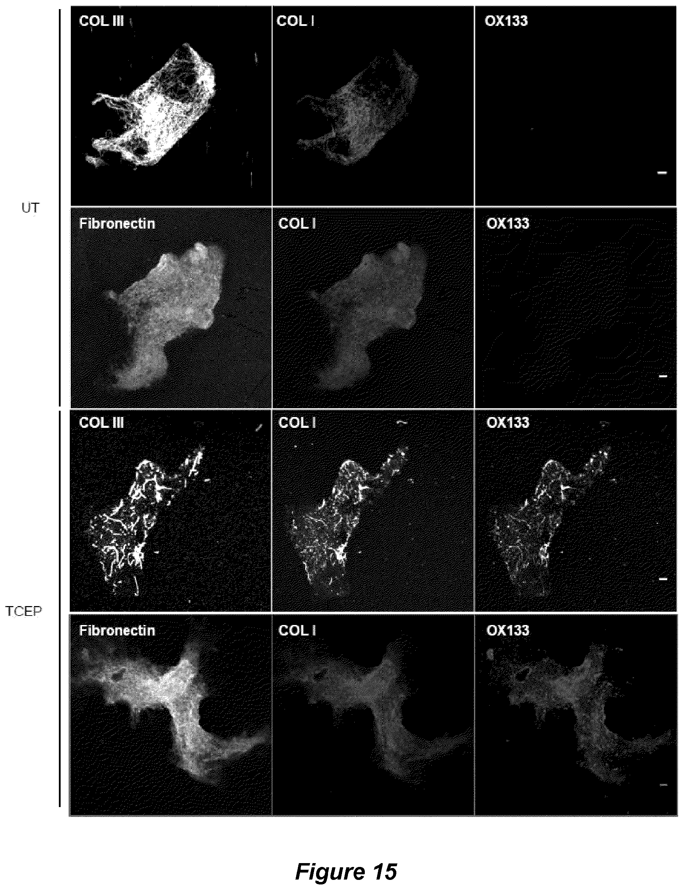

**Fig. 15** shows images indicating that disulfide bonds are abundantly present in cartilage extracellular matrix (ECM). This identifies the CNX:PDIA3 complex (that is the reducing effect of the complex on disulfide bonds) as a therapeutic target. Representative immunofluorescence staining images show staining of collagen fibres containing Collagen type III (Col III), Collagen type I (Col I) and Fibronectin in cartilage extracellular matrix (ECM). Collagen disulfide bonds are chemically reduced using TCEP which can be detected by staining with OX133 antibodies or left untreated (UT). Scale bar, 5 µm.

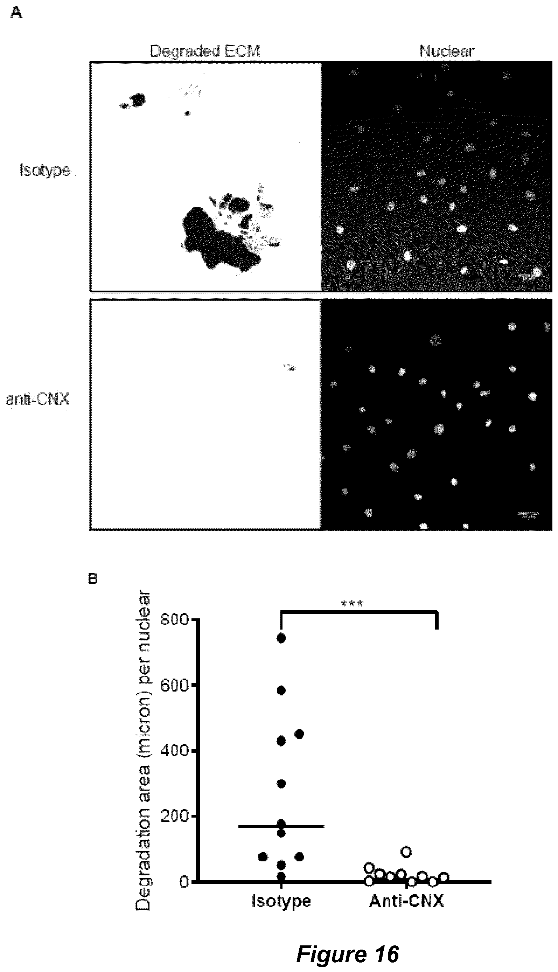

**Fig. 16** shows data indicating that a blockage of Calnexin (CNX) reduces cartilage degradation activity of primary synovial fibroblasts from osteoarthritis patients, thus identifying Calnexin as a therapeutic target in the treatment of arthritis. Panel A and B show representative images (A) and a quantification graph (B) showing decreased matrix degradation activity in primary fibroblasts isolated from osteoarthritis patients after incubation with anti-CNX antibodies or isotype control antibodies. Data correspond to the mean ± SEM and representative data for three independent experiments. Each data point represents the total degraded area (µm) per nuclear per well. ***, p<0.001 (one-way ANOVA).

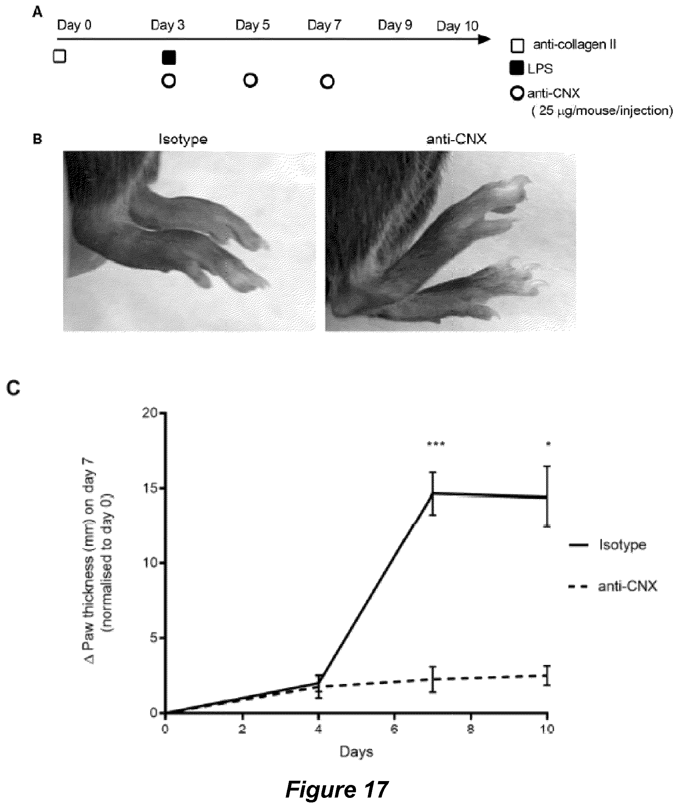

**Fig. 17** shows data indicating that treatment with antibodies against Calnexin (CNX) reduces paw swelling in CAIA mice, showing the efficacy of the use of anti-Calnexin antibodies in treating arthritis. Panel A shows a schematic of the antibody treatment scheme. Panel B shows representative photographs of CAIA mice treated with anti-CNX antibodies or left untreated at day 10. Panel C shows a line graph plotting paw thickness measurement, which shows reduced paw swelling in CAIA animals after injecting with anti-CNX antibodies. Data represent mean ± SEM and n=4-5 mice per group. *, p<0.05 (two-way ANOVA test).

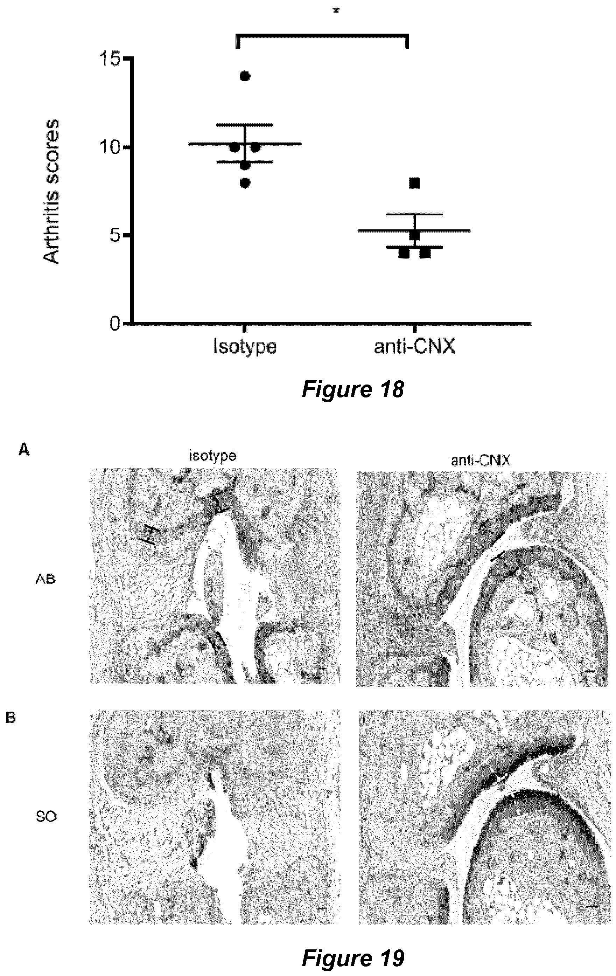

**Fig. 18** shows a graph indicating that treatment with antibodies against Calnexin (CNX) reduces arthritis severity in CAIA mice, thus showing the efficacy of the use of anti-Calnexin antibodies in treating arthritis. Clinical scores of CAIA mice at day 10 following treatment with isotype control antibodies or anti-CNX antibodies. Data are mean ± SEM, n=4 -5 mice per group. P-value is estimated by non-parametric t-test (Mann-Whitney test).

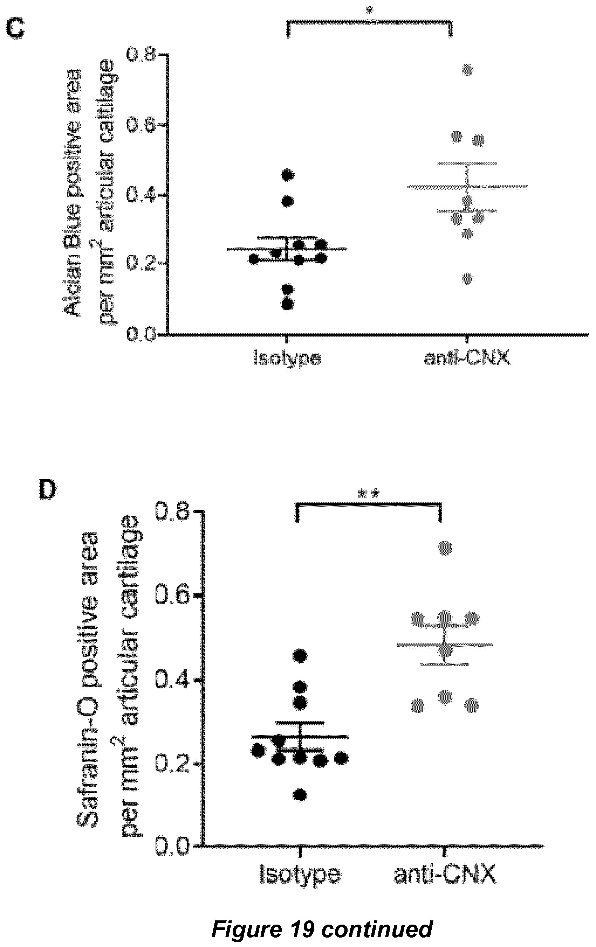

**Fig. 19** shows data indicating that treatment with antibodies against Calnexin (CNX) protects CAIA mice from cartilage degradation, thus showing the efficacy of the use of anti-Calnexin antibodies in treating arthritis or preventing the worsening of arthritis or a symptom of arthritis. Panels A and B show representative histological images showing Alcian blue (AB) (A) and Safranin-O (SO) (B) staining (arrow bars) in CAIA mice treated with isotype control or anti-CNX antibodies. Panels C and D show quantification graphs show increased AB and SO staining areas in CAIA mice treated with anti-CNX antibodies as compared to those treated with isotype control antibodies. Individual data points represent average of positive staining area per mm² articular cartilage from individual metacarpophalangeal joints of one animal, n= 4-5 mice per group. Data are shown as mean ± SEM. *, p<0.05; **, p<0.01(Mann-Whitney test).

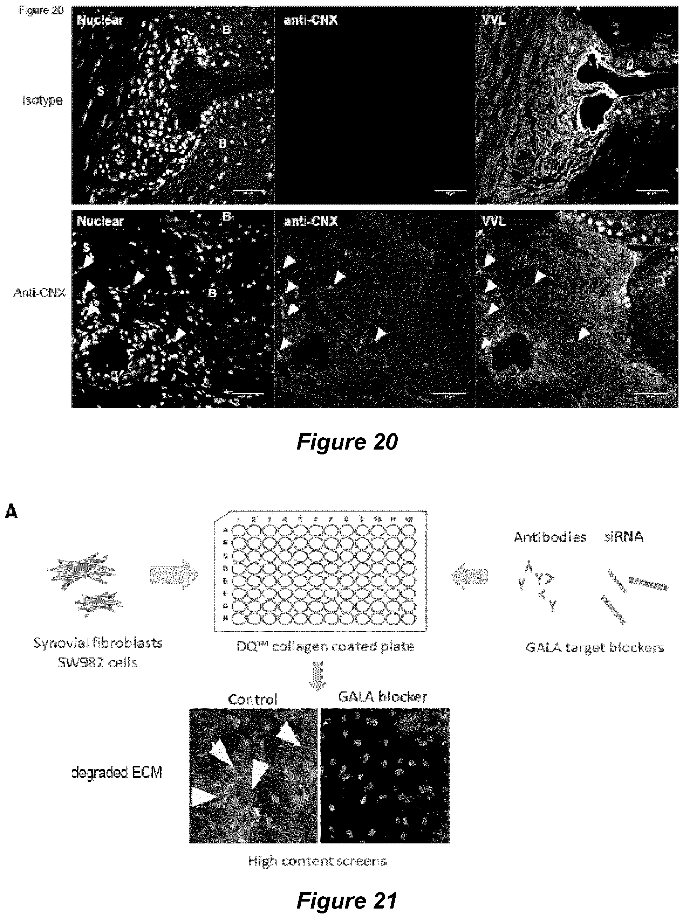

**Fig. 20** shows images indicating that anti-Calnexin (CNX) antibodies are accumulated in the synovium of CAIA mice. Representative immunofluorescence images show co-staining of VVL and anti-CNX antibodies (arrow heads) in CAIA mice injected with anti-CNX antibodies or isotype control antibodies at day 10. This shows that the accumulation of anti-CNX antibodies indicates that their ability to bind and target the CAIA mice synovium, while, in contrast, there is no binding with the control isotype antibodies. This indicates that Calnexin is expressed by cells in the synovium of CAIA mice, and that the cells in the synovium can specifically be targeted by anti-CNX antibodies for therapy. B: bone, S: synovium. Scale bar, 50 µm.

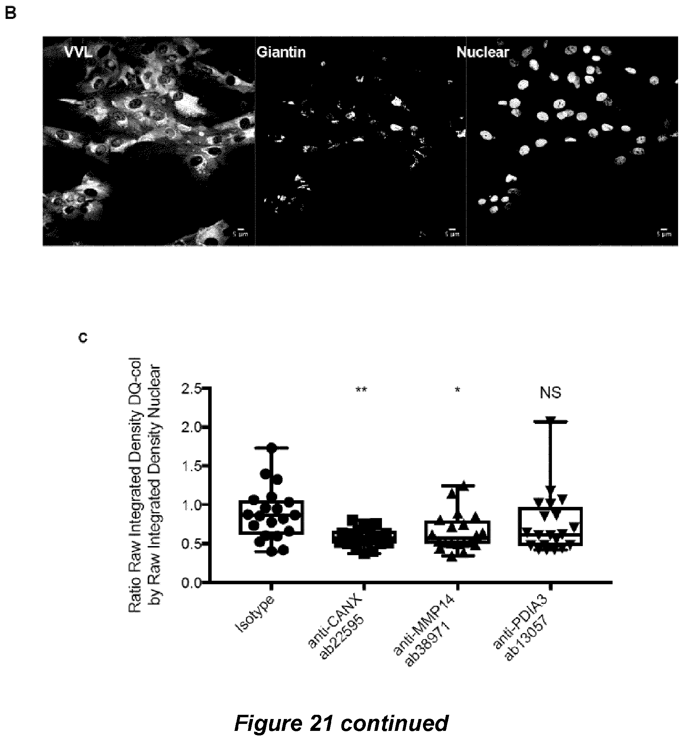

**Fig. 21** shows data indicating the results of screening for GALA targets and validation of its effect on primary arthritic synovial fibroblasts. Panel A shows a schematic diagram of high content screen to select GALA targets and their blocking modalities. Synovial fibroblast (SF) cells (SW982) were cultured on a quenched fluorescent cartilage matrix component (DQ-Collagen) in the presence of GALA target blockers (antibodies or siRNA). The degradation activity of GALA in synovial fibroblasts results in fluorescence signal increases (control panel), while synovial fibroblasts treated with blockers could inhibit degradation activity. Panel B shows the results of staining with VVL showing high Tn glycan expression outside Golgi (identified by Golgi marker, Giantin), indicating GALA activation in primary synovial fibroblasts isolated from an osteoarthritis patient (OASF). Scale bar, 5 µm. Panel C shows a quantification graph showing decreased matrix degradation activity in primary synovial fibroblasts, isolated from an osteoarthritis patient (OASF), treated with anti-Calnexin and anti-MMP14 antibodies. Each data point represents the ratio of raw integrated density of degraded DQ-Collagen to nuclear per field of view. Data are mean ± SEM, n = 20 field of views per group. *, p<0.05, **, p<0.01; NS, not significant (one way ANOVA test).

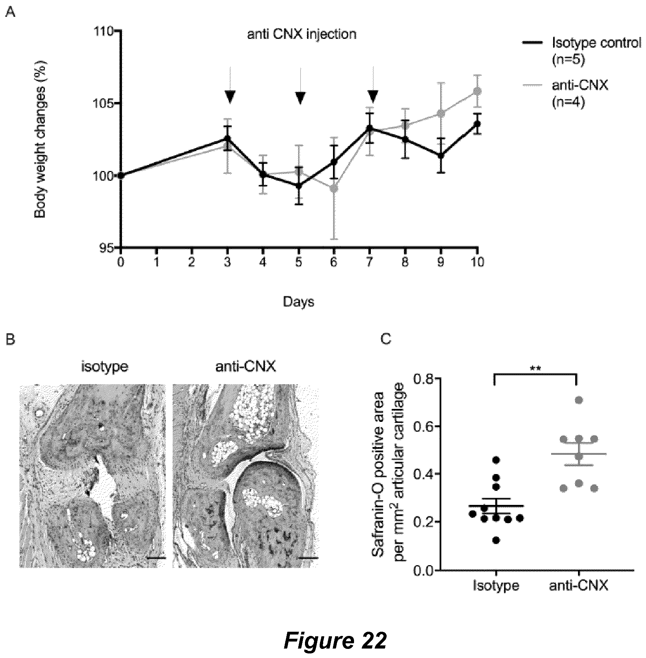

**Fig. 22** shows data of mice injected with anti-CNX or isotype control antibodies. This data shows comparable body weight changes between the two. A: Body weight changes of CAIA mice treated with isotype control or anti-CNX antibodies. B and C: Representative histological images and quantification of Safranin-O (SO) staining areas (B) in CAIA mice treated with isotype control or anti-CNX antibodies. Individual data points represent average of positive staining area per mm2 articular cartilage from individual metacarpophalangeal joints of one animal, n= 4-5 mice per group. Data are shown as mean ± SEM. *, p<0.05; **, p<0.01(Mann-Whitney test).

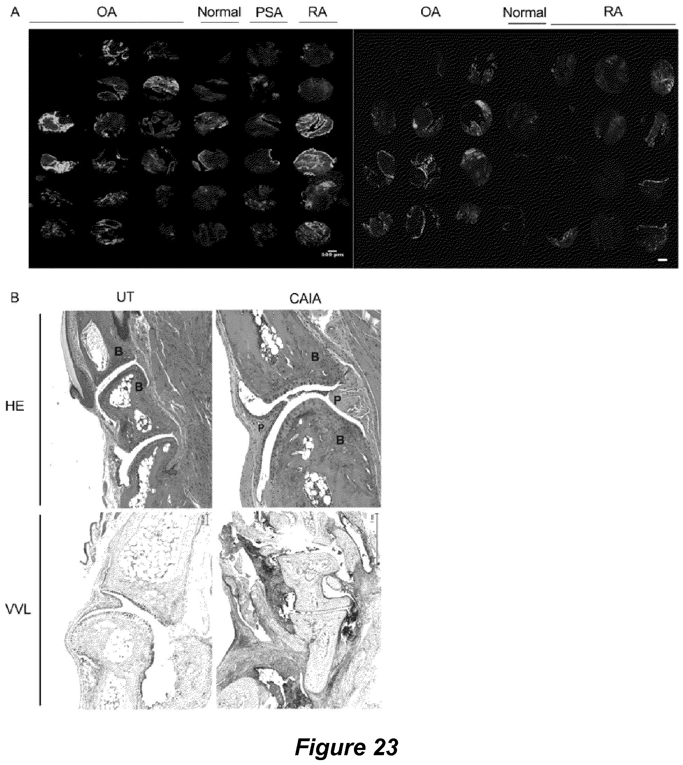

**Fig. 23** shows O-GalNAc (Tn) glycans tissue analysis in synovial tissues from both arthritis patients and arthritis induced animals. A: Representative Immunohistofluorescence staining of O-GalNAc glycans with VVL lectin on human tissue microarray (TMA) sections from Osteoarthritis (OA), psoriasis (PSA), rheumatoid arthritis (RA) and health subjects (Normal). Magnification 10X, scale bar, 500 µm. B: HE histology (upper panel) and immunohistochemistry staining with VVL lectin (lower panel) on collagen type II antibody induced arthritis (CAIA) mice at day 7 or those left untreated (UNT).

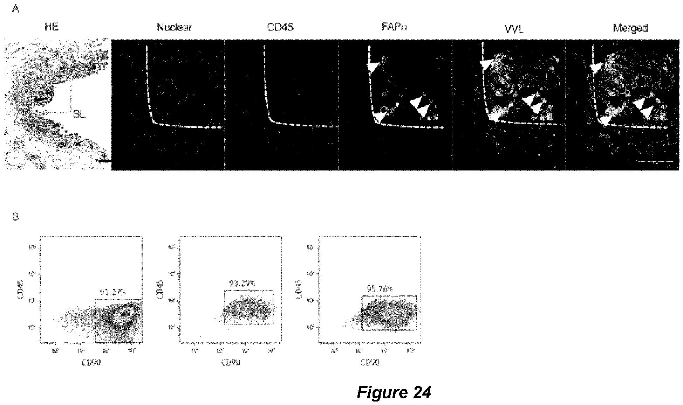

**Fig 24** shows OA synovial tissue analysis and primary synovial fibroblasts purification metric. A: (Left) H&E histology of synovial tissues obtained from OA patients. Scale bar, 100 µm; SL: synovial lining. (Right) Representative immunofluorescence images of OA synovium stained with VVL/CD45 and FAP***α***. Lining synovial fibroblasts are identified by FAP***α*** (arrowheads). Immune cells are identified by CD45. Sublining layer and lining layers boundaries are defined with dash line. Scale bar, 50 µm. B: FACS dot plots showing high purity (>90%) of primary SF cultures established from healthy subjects (HCSF), OA (OASF) and RA (RASF) patients.

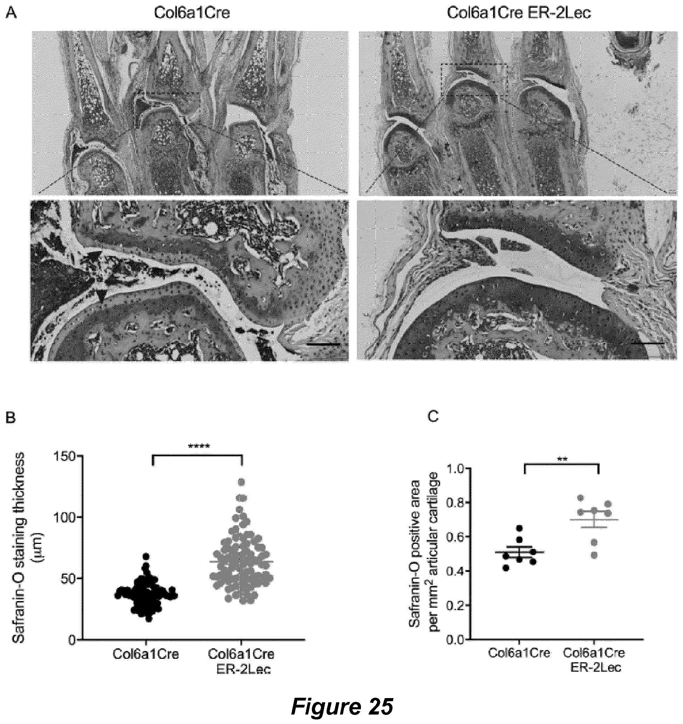

**Fig 25** shows that GALA activation causes damage to cartilage in CAIA mice. A: Representative images of Safranin-O (SO) (B) staining in untreated Col6a1Cre mice or arthritis induced Col6a1Cre and Col6a1Cre ER-2Lec mice at day 7. Scale bars, 100 µm. B and C: Quantification of SO staining thickness (B) and total positive staining area (C). Arthritis induced cartilage matrix degradation is indicated with arrowheads . Each data point represents an average of positive staining area per mm2 articular cartilage from three different metacarpophalangeal joints of one animal. Data are shown as mean ± SEM. p<0.01; ****, p<0.0001 (one way ANOVA test).

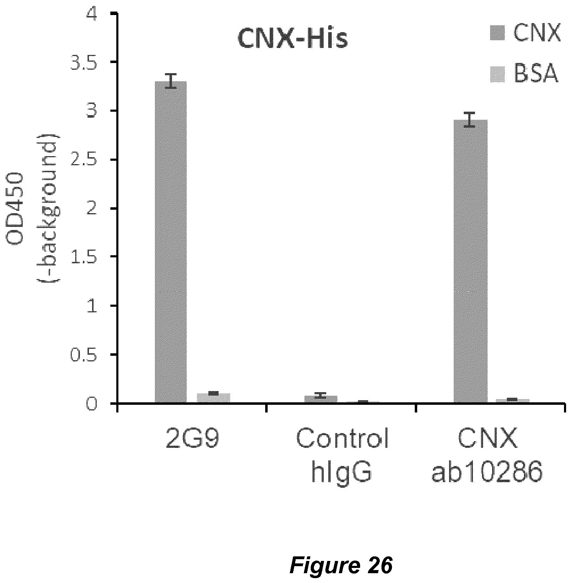

**Fig 26** shows binding data for monoclonal anti-calnexin antibody clone 2G9. Data were generated using an ELISA assay. The assay revealed high specificity for Cnx (left bar) for clone 2G9 and the commercial monoclonal antibody ab10286, with little binding to BSA coated wells (right bar). The negative control hIgG did not bind significantly to Calnexin. Data correspond to the mean ± SEM and representative data for three independent experiments.

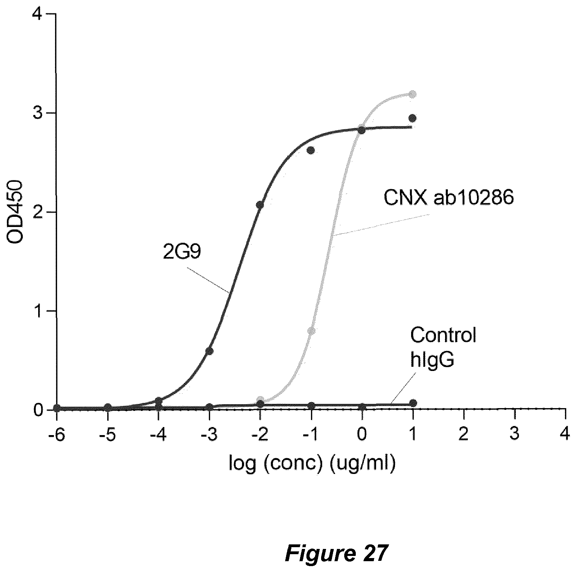

**Fig 27** shows further binding data for monoclonal anti-calnexin antibody clone 2G9, based on ELISA essays performed with serial dilutions of antibodies 2G9 and ab10286. Data suggest higher affinity and/or avidity for 2GP compared to commercial monoclonal antibody ab10286.

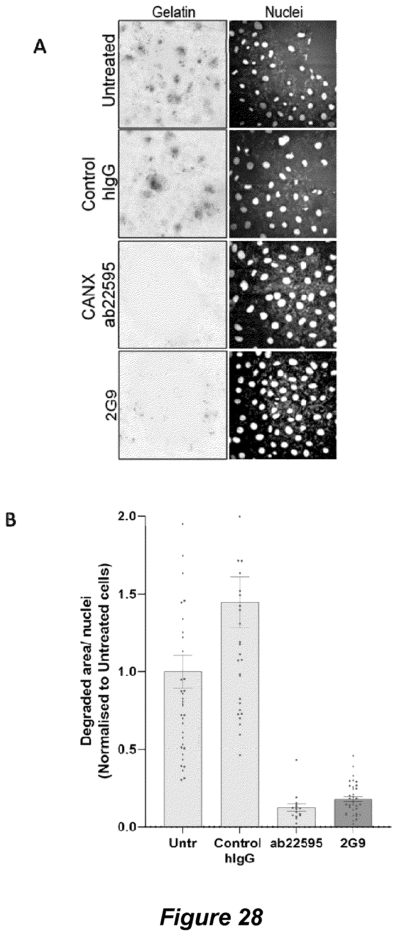

**Fig 28** shows ECM degradation assay data which demonstrate the ECM degradation ability of monoclonal anti-calnexin antibody clone 2G9. Panels A and B show representative images (A) and a quantification graph (B) showing 2G9 was able to reduce by 90% ECM degradation compared to untreated controls. Data correspond to the mean ± SEM and representative data for three independent experiments.

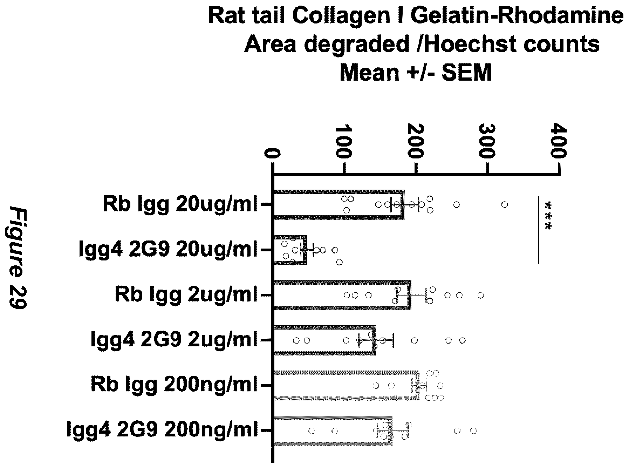

**Fig 29** shows further ECM degradation assay data. 2G9 in IgG4 format is also able to block ECM degradation. Data correspond to the mean ± SEM and representative data for three independent experiments. ***, p<0.001 (one-way ANOVA).

### DETAILED DESCRIPTION OF THE PRESENT INVENTION

### GalNAc-T activation (GALA) inhibitors in the treatment of cartilage degradation

The degradation of extracellular matrix is a key factor in cartilage degeneration, cartilage degradation, disorders associated with cartilage degradation, and joint disorders. Specifically, extracellular matrix degradation can lead to the development, progression or worsening of disorders such as osteoarthritis, psoriasis arthritis, rheumatoid arthritis, bursitis, gout, chondrocalcinosis, fibromyalgia, costochondritis, osteochondritis dissecans, cartilage damage, and polychondritis.

In most tissues, fibroblasts are the key cell type involved in producing extracellular matrices. However, fibroblasts can also degrade the matrix, allowing the turn-over of this essential component of tissues. How fibroblasts regulate these two opposite activities remains unclear.

Synovial fibroblasts (SF) also called synoviocytes are the prototypical Janus-faced cells. In healthy individuals, SFs contribute to the viscosity of the synovial fluid by secreting proteins such as hyaluronic acid and lubricin (Jay et al., J. Rheumatol. 27, 594-600, 2000). In arthritic diseases, SFs adhere and degrade the cartilage, specifically the extracellular matrix (ECM) of the cartilage. Understanding this change in activity during arthritis has been a major focus of research in recent years (Ospelt. RMD Open. 3, e000471, 2017).

The GALNTs Activation pathway (GALA) regulates ECM degradation in cancer cells through glycosylation of MMP14 and Calnexin. Inventors demonstrate here for the first time that cartilage degradation, disorders associated with cartilage degradation, and joint disorders are also associated with increased levels of GALA and O-glycosylation.

GALA induces matrix degradation through at least two mechanisms. First, it stimulates glycosylation of MMP14, which is required for its proteolytic activity (Nguyen et al., Cancer Cell. 32, 639-653.e6, 2017). Second, GALA induces the glycosylation of the ER-resident protein Calnexin, which forms a complex with ERp57, alias PDIA3 (Ros et al., Nat. Cell Biol. 22, 1371-1381. 2020). Following GALA-glycosylation, a fraction of the Cnx-PDIA3 complex is translocated to the surface of cancer cells. The complex accumulates in invadosomes and reduces disulfide bridges in the ECM (Ros et al., Nat. Cell Biol. 22, 1371-1381. 2020). This reduction is essential for the effective degradation of ECM (Ros et al., Nat. Cell Biol. 22, 1371-1381. 2020).

Inventors also demonstrate the treatment of cartilage degradation with a GALA inhibitor. Specifically, it is shown that the inhibition of GALA through the use of anti-cnx antibodies leads to a reduction of ECM degradation and arthritis symptoms in vivo.

Inventors results indicate that GALA O-glycosylation as a key switch in the pathologic, cartilage degradative activation of fibroblasts.

### Cartilage degradation

Cartilage degradation can occur through and the progression of certain disorders (e.g. arthritis, chondrocalcinosis, and polychondritis), and through mechanical damage (e.g. sports injuries). People with cartilage degradation commonly experience joint pain, stiffness, and inflammation, which can impact quality of life.

Certain disorders and diseases are associated with cartilage degradation. Furthermore, cartilage degradation can be caused by the mechanisms of certain disorders/diseases (e.g. arthritis, chondrocalcinosis, and polychondritis).

Inventors have demonstrated that cartilage degradation can be treated with GALA inhibitors, such as anti-cnx antibodies.

There are a number of different disorders associated with cartilage degradation, many of which are difficult to treat. It is noted by Krishnan and Grodzinsky (2018) in their publication titled "Cartilage Diseases" that most diseases involving cartilage lead to dramatic changes in the ECM which can (1) govern disease progression (e.g., in osteoarthritis), (2) cause the main symptoms of the disease (e.g., dwarfism caused by genetically inherited mutations) or (3) occur as collateral damage in pathological processes occurring in other nearby tissues (e.g., osteochondritis dissecans and inflammatory arthropathies). It is further noted that challenges associated with cartilage diseases include poor understanding of the etiology and pathogenesis (Krishnan and Grodzinsky. Matrix Biol. 2018 Oct; 71-72: 51-69).

Cartilage is an avascular, aneural, alymphatic connective tissue found in the synovial joints, spine, ribs, external ears, nose, and airways, and in the growth plates of children and adolescents. There are three major types of cartilage found in humans: hyaline, fibrous and elastic (Wachsmuth et al., Histol Histopathol. 2006 May; 21(5):477-85). Therefore, ECM degradation in cartillage can lead to many different disorders.

Cartilage degradation may occur in hyaline cartilage, fibrous cartilage or elastic cartilage. A disorder associated with cartilage degradation may be a disorder involving hyaline cartilage degradation, fibrous cartilage degradation or elastic cartilage degradation.

Hyaline cartilage is the most widespread type of cartilage and is the type that makes up the embryonic skeleton. It persists in human adults at the ends of bones in free-moving joints as articular cartilage, at the ends of the ribs, and in the nose, larynx, trachea, and bronchi.

Fibrocartilage is tough, strong tissue found predominantly in the intervertebral disks and at the insertions of ligaments and tendons; it is similar to other fibrous tissues but contains cartilage ground substance and chondrocytes.

Elastic cartilage, is more pliable than the other two forms because it contains elastic fibres in addition to collagen. In humans it makes up the external ear, the auditory tube of the middle ear, and the epiglottis.

A subject with cartilage degradation may have arthritis, osteoarthritis, psoriasis arthritis, rheumatoid arthritis, gout, chondrocalcinosis, fibromyalgia, costochondritis, osteochondritis dissecans, cartilage damage, and/or polychondritis.

A subject with cartilage degradation may have arthritis. The cartilage degradation may be caused by, or otherwise associated with, the arthritis of the subject.

A subject with cartilage degradation may have osteoarthritis or rheumatoid arthritis.

Arthritis is a group of diseases affecting joints (Barbour et al., Morbidity and Mortality Weekly Report. 65. 2016, pp. 1052-1056). Rheumatoid arthritis (RA) and Osteoarthritis (OA) are two of the most common types (Murphy and Nagase. Nat. Clin. Pract. Rheumatol. 4, 128-135. 2008). RA is an immune-mediated inflammatory disease, where autoreactive B and T cells produce autoantibodies, resulting in the formation of immune complexes in the joint (van Delft, et al., J. Autoimmun. 110, 102392. 2020). This drives inflammation and the recruitment of neutrophils, macrophages and other immune cells to the area (Hirota et al., Immunity. 48, 1220-1232.e5. 2018; Kuo et al., Sci. Transl. Med. 11. 2019, doi:10.1126/scitranslmed.aau8587; Takemura et al., J. Immunol. 167, 4710-4718. 2001; Smolen et al., Nature Reviews Disease Primers. 4. 2018. doi:10.1038/nrdp.2018.1). These immune cells secretes cytokines such as IL-1Beta and TNFalpha that activate synoviocytes. These cytokines are now therapeutically targeted, providing significant relief in most patients. OA is the most frequent and less well understood form of arthritis; it is a gradually evolving disease with a less prominent immune component (Martel-Pelletier et al., Nat Rev Dis Primers. 2, 16072. 2016; Kapoor et al., Nat. Rev. Rheumatol. 7, 33-42. 2011; Goldring and Otero. Curr. Opin. Rheumatol. 23, 471-478. 2011). A commonly accepted hypothesis is that mechanical damage of the cartilage leads to a low grade inflammatory condition that mediates progressive cartilage loss (Kapoor et al., Nat. Rev. Rheumatol. 7, 33-42. 2011; Pap and Korb-Pap. Rheumatol. 11, 606-615. 2015).

In healthy synovial joints, the synovial membrane surrounds and isolates the joint cavity, secreting extracellular matrix proteins in the synovial fluid. Synovial fibroblasts are the main stromal cells of the synovial membrane, interspaced with resident macrophages (Barbour et al., Morbidity and Mortality Weekly Report. 65. 2016, pp. 1052-1056). During the active phases of RA, SFs become activated, expressing the Fibroblast Activation Protein alpha and proliferate. SF cells, as other stromal cells, express innate immune receptors such as Toll-Like Receptors. They can detect local pathogens and molecular damage, secreting cytokines that activate immune cells (Ospelt et al., Arthritis Rheum. 58, 3684-3692. 2008). During inflammation, SF proliferate, forming, together with infiltrating immune cells, an enlarged synovial membrane called a pannus (Choy. Rheumatology . 51 Suppl 5, v3-11. 2012). The pannus invades the joint cavity and degrades cartilage (Pap and Korb-Pap. Rheumatol. 11, 606-615. 2015). In particular, SF in the synovial lining layer have been shown to mediate cartilage degradation, while SF in the sub-lining tend to mediate inflammation (Croft et al., Nature. 570, 246-251. 2019). The ECM degrading activity is due to increased production of matrix metalloproteinases (MMPs), A Disintegrin And Metalloproteinase with Thrombospondin motifs (ADAMTs) and cathepsins (Rengel and Ospelt. Arthritis Res. Ther. 9, 221 2007). Arthritic synovial fibroblasts express both secreted (Jay et al., J. Rheumatol. 27, 594-600, 2000; Barbour et al., Morbidity and Mortality Weekly Report. 65. 2016, pp. 1052-1056; Smolen et al., Nature Reviews Disease Primers. 4. 2018. doi:10.1038/nrdp.2018.1) and cell surface MMPs (Lange-Brokaar et al., Osteoarthritis Cartilage. 20, 1484-1499. 2012; Nygaard and Firestein. Nat. Rev. Rheumatol. 16, 316-333. 2020; Bauer et al., Arthritis Res. Ther. 8, R171; 2006) MMPs. MMP14 (MT1-MMP) in particular is essential for the invasive properties of SFs.

The acquisition of aberrant matrix degradation is also characteristic of SFs in OA (Fuchs et al., Osteoarthritis Cartilage. 12, 409-418. 2004). While the OA synovial membrane typically has less immune cells than in RA, it drives cartilage degradation as in RA. What controls the switch to ECM-degradation mode of SFs is not well understood. Change in gene expressions are obviously suspected and similar transcriptional signatures have been detected in both diseases (Cai et al., J Immunol Res. 2019, 4080735. 2019). Epigenetic changes have been detected and proposed to drive the phenotype of arthritic SFs (Nakano et al., Ann. Rheum. Dis. 72, 110-117. 2013). Whether these alterations are sufficient remains unclear.

The phenotype of SFs during arthritis has been compared to that of malignant cancer cells. Indeed, cancer growth requires a profound remodelling of the ECM in the tissue of origin, with degradation of the original tissue ECM (Hotary et al., Cell. 114, 33-45. 2003). MMPs and other matrix degradation enzymes are particularly active in malignant cells (Castro-Castro et al., Cell Dev. Biol. 32, 555-576. 2016).

Gout is an inflammatory type of arthritis, also known as gouty arthritis. Gout is the most common inflammatory arthritis with a prevalence of 2.5% in the UK. Although it has the potential to be cured, its treatment remains suboptimal (Abhishek et al., Clin Med (Lond). 2017 Feb; 17(1): 54-59). The ultrasonographic findings of gout include double contour sign (MSU crystal deposition on surface of hyaline articular cartilage). Normal adult articular cartilage is made up of an abundant ECM composed mainly of type II collagen fibrils interspersed with types IX and XI collagens. Cartilage loss tends to be a late feature of gouty arthropathy and, similar to bone erosion, is localized rather than diffuse. Cartilage damage is often associated with erosion and has been described as occurring in regions of biomechanical stress. Treatment to prevent, reduce, or reverse of ECM degradation and/or cartilage loss would benefit patients with gout. In some cases, the gout to be treated is associated with ECM degradation and/or cartilage loss.

Chondrocalcinosis, or cartilage calcification, is calcification (accumulation of calcium salts) in hyaline cartilage and/or fibrocartilage. Build-up of calcium phosphate in the ankle joints has been found in about 50% of the general population, and may be associated with osteoarthritis (Hubert et al., BMC Musculoskelet Disord. 2018; 19: 169). It is often found in weight bearing joints such as the hip, ankle and knee. The molecular structure of calcium pyrophosphate has the potential of triggering inflammatory responses. The presence of chondrocalcinosis has associations with the degradation of cartilage menisci and synovial tissue. It has been reported that presence of calcium-containing crystals, which are associated with chondrocalcinosis, was associated with higher prevalence of cartilage and meniscal damage (Gersing et al., Eur Radiol. 2017 Jun;27(6):2497-2506. doi: 10.1007/s00330-016-4608-8. Epub 2016 Oct 4). Treatment to prevent, reduce, or reverse of ECM degradation and/or cartilage loss would benefit patients with chondrocalcinosis. In some cases, the chondrocalcinosis to be treated is associated with ECM degradation and/or cartilage loss.

Fibromyalgia (FM) is a medical condition characterized by chronic widespread pain and a heightened pain response to pressure. FM is common in rheumatoid arthritis, axial spondyloarthritis and psoriatic arthritis, and could therefore influence management of these rheumatic conditions. FM is also associated with costochondritis.

Costochondritis is an inflammation of the cartilage in the rib cage. The condition usually affects the cartilage where the upper ribs attach to the breastbone, or sternum, an area known as the costosternal joint or costosternal junction. Costochondritis can be caused by mechanical stress, potentially leading to cartilage loss and/or ECM degradation. Therefore, treatment to prevent, reduce, or reverse of ECM degradation and/or cartilage loss could benefit patients with costochondritis. In some cases, the costochondritis to be treated is associated with ECM degradation and/or cartilage loss.

Osteochondritis dissecans (OCD or OD) is a disorder in which cracks form in the articular cartilage and the underlying subchondral bone. OCD usually causes pain during and after sports. In later stages of the disorder there will be swelling of the affected joint which catches and locks during movement. Physical examination in the early stages can identify pain as symptom, in later stages there could be an effusion, tenderness, and a crackling sound with joint movement. Treatment to prevent, reduce, or reverse of ECM degradation and/or cartilage loss would benefit patients with osteochondritis dissecans. In some cases, the osteochondritis dissecans to be treated is associated with ECM degradation and/or cartilage loss.

Polychondritis, or relapsing polychondritis (RP), is an immune-mediated systemic disease characterized by recurrent inflammatory episodes of cartilaginous and proteoglycan-rich tissues, including the elastic cartilage of the ear and nose, the hyaline cartilage of peripheral joints, the fibrocartilage at axial sites and the cartilage of the tracheobronchial tree, which result in progressive anatomical deformation and functional impairment of the involved structures (Borgio et al., Biomedicines. 2018 Sep; 6(3): 84). Mono- or, more frequently, bilateral auricular chondritis is the most common feature of RP, which is observed in up to 90% of patients during the course of the disease, and is the inaugural symptom in 20% of cases. The onset is abrupt, with painful red to violaceous erythema and edema confined to the cartilaginous part of the ear, typically sparing the lobe, which lacks cartilage. Acute inflammatory episodes tend to resolve spontaneously within few days or weeks, with recurrence at variable intervals. As a long-term consequence of repeated flares, the cartilage matrix is severely damaged and replaced by fibrous connective tissue (Borgio et al., Biomedicines. 2018 Sep; 6(3): 84). Therefore, treatment to prevent, reduce, or reverse of ECM degradation and/or cartilage loss could benefit patients with polychondritis. In some cases, the polychondritis to be treated is associated with ECM degradation and/or cartilage loss.

Cartilage damage can arise through a number of processes, for example through the mechanisms of specific diseases such as arthritis, gout, chondrocalcinosis, fibromyalgia, costochondritis, osteochondritis dissecans, and polychondritis. Cartilage damage can also result from mechanical injury, for example through a collision or hyperextension whilst playing sport. In some cases cartilage damage requires surgery to remove damaged tissue. Isolated chondral and osteochondral defects of joints (such as the knee) are a difficult clinical challenge, particularly in younger patients for whom alternatives such as partial or total knee arthroplasty are rarely advised. Numerous surgical techniques have been developed to address focal cartilage defects. Cartilage treatment strategies are characterized as palliation (e.g., chondroplasty and debridement), repair (e.g., drilling and microfracture [MF]), or restoration (e.g., autologous chondrocyte implantation [ACI], osteochondral autograft [OAT], and osteochondral allograft [OCA]) (Richter et al., Sports Health. Mar-Apr 2016;8(2):153-60. doi: 10.1177/1941738115611350. Epub 2015 Oct 12). In some cases, treatment to prevent, reduce, or reverse of ECM degradation and/or cartilage loss could be an alternative to surgery for patients with cartilage damage. Treatment to prevent, reduce, or reverse of ECM degradation and/or cartilage loss could benefit patients with cartilage damage.

In some cases, cartilage damage can result in arthritis (e.g. osteoarthritis). Post-traumatic arthritis (PTA) develops after an acute direct trauma to the joints. PTA causes about 12% of all osteoarthritis cases, and a history of physical trauma may also be found in patients with chronic inflammatory arthritis. Treatment with a GALA inhibitor after cartilage damage could prevent the development of arthritis after physical trauma.

### Disorders associated with cartilage degradation

Inventors have demonstrated that disorders associated with cartilage degradation can be treated with GALA inhibitors, such as anti-cnx antibodies.

The disorder associated with cartilage degradation may be arthritis, osteoarthritis, psoriasis arthritis, rheumatoid arthritis, gout, chondrocalcinosis, fibromyalgia, costochondritis, osteochondritis dissecans, cartilage damage, or polychondritis.

The disorder associated with cartilage degradation may be arthritis.

The disorder associated with cartilage degradation may be osteoarthritis or rheumatoid arthritis.

### Joint disorders

Inventors have demonstrated that joint disorders can be treated with GALA inhibitors, such as anti-cnx antibodies.

A joint is defined as a connection between two bones in the skeletal system. Joints can be classified by the type of the tissue present (fibrous, cartilaginous or synovial), or by the degree of movement permitted (synarthrosis, amphiarthrosis or diarthrosis). Therefore, a joint disorder is defined as a condition which affects a connection between two bones in the skeletal system. Definitions of specific joints and related aspects of anatomy can be found in "Netter, F. H. (2006). Atlas of human anatomy. Philadelphia, PA: Saunders/Elsevier", which is incorporated by reference in its entirety.

The joint disorder may affect a fibrous, cartilaginous or synovial joint.

Fibrous joints are connected by dense connective tissue consisting mainly of collagen. These joints are also called fixed or immovable joints because they do not move. Fibrous joints have no joint cavity and are connected via fibrous connective tissue. The skull bones are connected by fibrous joints called sutures.

Cartilaginous joints are a type of joint where the bones are entirely joined by cartilage, either hyaline cartilage or fibrocartilage. These joints generally allow more movement than fibrous joints but less movement than synovial joints.

A synovial joint is characterised by the presence of a fluid-filled joint cavity contained within a fibrous capsule. It is the most common type of joint found in the human body and contains several structures which are not seen in fibrous or cartilaginous joints. The three main features of a synovial joint are: (i) articular capsule, (ii) articular cartilage, (iii) synovial fluid. The articular capsule surrounds the joint and is continuous with the periosteum of articulating bones. The articulating surfaces of a synovial joint (i.e. the surfaces that directly contact each other as the bones move) are covered by a thin layer of hyaline cartilage. The articular cartilage has two main roles: (i) minimising friction upon joint movement, and (ii) absorbing shock. The synovial fluid is located within the joint cavity of a synovial joint. It has three primary functions. Synovial joints can include accessory structures such as tendons, ligaments, bursae, and vasculature.

There are numerous types of synovial joints. In some cases, the joint disorder is of a gliding joint, a hinge joint, a pivot joint, an ellipsoid joint, saddle joint, or a ball and socket joint.

A gliding joint, also known as a plane joint or planar joint, is a common type of synovial joint formed between bones that meet at flat or nearly flat articular surfaces. Gliding joints allow the bones to glide past one another in any direction along the plane of the joint - up and down, left and right, and diagonally. Slight rotations can also occur at these joints, but are limited by the shape of the bones and the elasticity of the joint capsule surrounding them.

A hinge joint (ginglymus) is a bone joint in which the articular surfaces are molded to each other in such a manner as to permit motion only in one plane. According to one classification system they are said to be uniaxial (having one degree of freedom) (Platzer, Werner (2008) Color Atlas of Human Anatomy, Volume 1). The direction which the distal bone takes in this motion is seldom in the same plane as that of the axis of the proximal bone; there is usually a certain amount of deviation from the straight line during flexion. The articular surfaces of the bones are connected by strong collateral ligaments. The best examples of ginglymoid joints are the Interphalangeal joints of the hand and those of the foot and the joint between the humerus and ulna. The knee joints and ankle joints are less typical, as they allow a slight degree of rotation or of side-to-side movement in certain positions of the limb. The knee is the largest hinge joint in the human body.

A pivot joint (trochoid joint, rotary joint or lateral ginglymus) is a type of synovial joint whose movement axis is parallel to the long axis of the proximal bone, which typically has a convex articular surface. According to one classification system, a pivot joint has one degree of freedom (Platzer, Werner (2008) Color Atlas of Human Anatomy, Volume 1).

An ellipsoid joint (also called a condyloid joint) is is an ovoid articular surface, or condyle that is received into an elliptical cavity. This permits movement in two planes, allowing flexion, extension, adduction, abduction, and circumduction, as seen in the wrist joint.

A saddle joint is a type of synovial joint in which the opposing surfaces are reciprocally concave and convex. It is found in the thumb, the thorax, and the middle ear, and the heel.

A ball and socket joint (or spheroid joint) is a type of synovial joint in which the ball-shaped surface of one rounded bone fits into the cup-like depression of another bone. The distal bone is capable of motion around an indefinite number of axes, which have one common center. This enables the joint to move in many directions. The joint disorder may affect a synarthrosis, amphiarthrosis or diarthrosis joint. The hip and shoulder are ball and socket joints.

A synarthrosis is a type of joint which allows no movement under normal conditions. Sutures and gomphoses are both synarthroses.

An amphiarthrosis is a joint that has limited mobility. An example of this type of joint is the cartilaginous joint that unites the bodies of adjacent vertebrae.

A diarthrosis joint is a freely moveable joint. Sometimes the terms diarthrosis joints and synovial joints are used interchangeably.

The joint disorder may affect any joint. In some cases, the joint disorder may affect any mammalian joint. In some cases, the joint disorder may affect any human joint.

In some cases, the joint disorder affects the hip, knee, ankle, foot, toe, shoulder, elbow, wrist, hand, finger, neck, spine, ribs, or sacroiliac joint.

The joint disorder may be osteoarthritis, psoriasis arthritis, rheumatoid arthritis, bursitis, gout, chondrocalcinosis, fibromyalgia, costochondritis, osteochondritis dissecans, polychondritis, cartilage damage, tendon damage, or ligament damage.

Bursitis is inflammation of a bursa, a small fluid-filled sac that acts as a cushion between bone and muscle, skin or tendon. The type of bursitis depends on where the affected bursa is located. This soft tissue condition commonly affects the shoulder, elbow, hip, buttocks, knees and calf. Athletes, the elderly and people who do repetitive movements like manual laborers and musicians are more likely to get bursitis. Bursitis is sometimes mistaken for arthritis because the pain can occur in a joint.

Tendon damage, or tendinopathy, can be caused in a number of ways, for example from overuse, aging, wear and tear, or a mechanical injury. Tendon damage may be tendinitis or tendonosis. Tendinitis refers to inflammation of a tendon, and tendonosis relates to tears in the tissue in and around the tendon. The tendon damage may be a strained tendon, a sprained tendon, a torn tendon, a partially ruptured tendon or a completely ruptured tendon. Treatment to prevent, reduce, or reverse of ECM degradation and/or tendon loss could benefit patients with tendon damage. In some cases, the tendon damage to be treated is associated with ECM degradation and/or cartilage loss.

Ligament damage can be caused in a number of ways for example from overuse, aging, wear and tear, or a mechanical injury. The ligament damage may be a strained ligament, a sprained ligament, a torn ligament, a partially ruptured ligament or a completely ruptured ligament. Treatment to prevent, reduce, or reverse of ECM degradation and/or ligament loss could benefit patients with ligament damage. In some cases, the tendon damage to be treated is associated with ECM degradation and/or cartilage loss.

### Characteristics of disorders

Disorders described herein (e.g. disorders associated with cartilage degradation, and joint disorders) may be characterised by a number of features. In some cases, the disorder is characterised by ECM degradation. In some cases, the disorder is characterised by increased ECM degradation. In some cases, the disorder to be treated is characterised by increased expression of calnexin. In some cases, the disorder to be treated is characterised by increased calnexin glycosylation. In some cases, the disorder is characterised by increased calnexin O-glycosylation. In some cases, the disorder is characterised by increased O-glycosylation. In some cases, the disorder is characterised by GALNT Activation (GALA). In some cases, the disorder is characterised by increased GALNT Activation (GALA). In some cases, the disorder is characterised by inflammation.

The ECM is a highly dynamic structure, constantly undergoing a remodelling process where ECM components are deposited, degraded, or otherwise modified. Extracellular matrix (ECM) degradation is mediated by cell surface and secreted proteases, in particular matrix metalloproteases (MMP), such as, but not limited to, MMP14, and O-glycosylated MMP14.

Calnexin (CNX) is a 90 kDa calcium binding protein of the ER membrane, which forms a complex with PDIA3. Calnexin is a target of GALA. Following glycosylation, the CNX-PDIA3 complex has previously been shown to translocate to the surface of cancer cells, where it reduces disulfide bridges in the extracellular matrix (Ros et al., Nat. Cell Biol. 22, 1371-1381, 2020). The reduction of disulfide bridges has been shown to be essential for the effective activity of matrix metalloproteinases (MMPs) and thus for the degradation of the extracellular matrix in cancer.

Where reference is made to a CNX polypeptide, this should be taken as a reference to any member of the CNX family of polypeptides. Of particular interest are CNX polypeptides derived from the genes in the group consisting of: Mouse Gene ID: 12330, Rat Gene ID: 29144, Dog Gene ID: 403908, Cat Gene ID: 101085686 and Horse Gene ID: 100067402.

For example, the Cnx polypeptide may comprise a human CNX sequence having GenBank Accession Number NP_001350929.1, NP_001350926.1, NP_001350923.1, NP_001350922.1, NP_001350924.1, NP_001350928.1, NP_001350927.1 or NP_001019820.1.

PDIA4 is one of the largest PDI members comprising 645 amino acids and with three classical CGHC active sites. Together with ER Hsp70 (BiP), Grp94, PDI and ERp29 they form a multiprotein chaperone complex which is an ER network that can bind to unfolded protein substrates (Meunier et al., Mol. Biol. Cell, 13 (2002), pp. 4456-4469, 10.1091/mbc.e02-05-0311).

Where reference is made to a PDIA4 polypeptide, this should be taken as a reference to any member of the PDIA4 family of polypeptides. Of particular interest are PDIA4 polypeptides derived from the genes in the group consisting of: Homo sapiens GeneID: 9601, Mus musculus GeneID: 12304, Rattus norvegicus GeneID: 116598, Bos taurus GeneID: 415110, Equus caballus GeneID: 100060535, Felis catus GeneID: 101087568 and Canis lupus familiaris GeneID: 482715.

For example, the PDIA4 polypeptide may comprise a human PDIA4 sequence having GenBank Accession Number NP_001358173.1, NP_001358174.1, or NP_004902.1.

PDIA3, also known as ERp57, is an isomerase enzyme. This protein localizes to the endoplasmic reticulum (ER) and interacts with lectin chaperones calreticulin and calnexin (CNX) to modulate folding of newly synthesized glycoproteins. It is thought that complexes of lectins and this protein mediate protein folding by promoting formation of disulfide bonds in their glycoprotein substrates.

Where reference is made to a PDIA3 (ERp57) polypeptide, this should be taken as a reference to any member of the PDIA3 (ERp57) family of polypeptides. Of particular interest are PDIA3 (ERp57) polypeptides derived from the genes in the group consisting of: Homo sapiens GeneID: 2923, Mus musculus GeneID: 14827, Rattus norvegicus GeneID: 29468, Bos taurus GeneID: 281803, Equus caballus GeneID: 100056198, Felis catus GeneID: 101097245 and Canis lupus familiaris GeneID: 478279.

For example, the ERp57 polypeptide may comprise a human PDIA3 (ERp57) sequence having GenBank Accession Number NP_005304.3.

Glycosylation is an enzymatic process that attaches glycans to proteins, or other biological molecules. O-glycosylation is the addition of O-linked glycan to the hydroxyl group of the side chain of e.g. a serine, threonine, tyrosine, hydroxylysine, or hydroxyproline residue of a protein.

The N-Acetylgalactosaminyltransferases (GALNTs) Activation (GALA) pathway is regulated by the relocation of O-glycosylation initiation enzymes GALNTs from the Golgi to the ER. GALNTs catalyse the formation of the Tn glycan, which consist of a single GalNac residue. Tn can be detected with lectins such as Vicia Villosa Lectin (VVL). GALNTs ER relocation dramatically increases the total cellular staining levels of Tn, with a cytoplasmic instead of perinuclear appearance (Gill, D. J. et al. Initiation of GalNAc-type O-glycosylation in the endoplasmic reticulum promotes cancer cell invasiveness. Proc. Natl. Acad. Sci. U. S. A. 110, E3152-61 (2013).

Inflammation is part of the complex biological response of body tissues to harmful stimuli, such as pathogens, damaged cells, or irritants. The function of inflammation is to eliminate the initial cause of cell injury, clear out necrotic cells and tissues damaged from the original insult and the inflammatory process, and initiate tissue repair. Clinically and epidemiologically, inflammation is usually estimated by measurement of circulating substances that are released as a cause or consequence of an inflammatory response, and the most widely used biomarker for inflammation is C-reactive protein (CRP) (Sproston and Ashworth. Front Immunol. 2018; 9: 754).

In some cases the disorder to be treated is characterised by increased expression of calnexin, increased expression of PDIA4, increased expression of PDIA3, increased calnexin glycosylation, increased calnexin O-glycosylation, increased O-glycosylation, GALA, increased GALA, inflammation, and/or increased inflammation.

If a disorder is characterised by an increase in expression, activity, or another characteristic, the characteristic is increased compared to a subject unaffected by the disorder. The increase may be greater than 1 times, e.g. one of ≥1.1 times, ≥1.2 times, ≥1.3 times, ≥1.4 times, ≥1.5 times, ≥1.6 times, ≥1.7 times, ≥1.8 times, ≥1.9 times, ≥2.0 times, ≥2.5 times, ≥3.0 times, ≥3.5 times, ≥4.0 times, ≥4.5 times, ≥5.0 times, ≥5.5 times, ≥6.0 times, ≥6.5 times, ≥7.0 times, ≥7.5 times, ≥8.0 times, ≥8.5 times, ≥9.0 times, ≥9.5 times, or ≥10 times compared to a subject unaffected by the disorder.

If a disorder associated with cartilage degradation is characterised by increase in a characteristic, the affected subject has a greater level of the characteristic when compared to a corresponding subject unaffected by the characteristic.

If a joint disorder is characterised by an increase in a characteristic, the affected subject has a greater level of the characteristic in a joint compared to a corresponding control (e.g. a subject unaffected by the joint disorder, or an unaffected joint).

If the arthritis is characterised by an increase in a characteristic, the affected subject has a greater level of the characteristic in a joint compared to a corresponding control (e.g. to a corresponding subject unaffected by arthritis, or a corresponding joint unaffected by arthritis).

If a disorder is characterised by increased expression of calnexin, increased expression of PDIA4, increased expression of PDIA3, increased calnexin glycosylation, increased calnexin O-glycosylation, increased O-glycosylation, increased GALA, and/or increased inflammation, the characteristic is increased compared to a corresponding control (e.g. to a subject unaffected by the disorder).

If a disorder (e.g. a disorder associated with cartilage degradation, a joint disorder, and/or arthritis) is characterised by increased o-glycosylation, the affected subject has a greater level of o-glycosylation when compared to a corresponding to a corresponding control (e.g. subject unaffected by the disorder). The level of o-glycosylation may be greater than 1 times, e.g. one of ≥1.1 times, ≥1.2 times, ≥1.3 times, ≥1.4 times, ≥1.5 times, ≥1.6 times, ≥1.7 times, ≥1.8 times, ≥1.9 times, ≥2.0 times, ≥2.5 times, ≥3.0 times, ≥3.5 times, ≥4.0 times, ≥4.5 times, ≥5.0 times, ≥5.5 times, ≥6.0 times, ≥6.5 times, ≥7.0 times, ≥7.5 times, ≥8.0 times, ≥8.5 times, ≥9.0 times, ≥9.5 times, or ≥10 times compared to a subject unaffected by the disorder.

If a disorder (e.g. a disorder associated with cartilage degradation, a joint disorder, and/or arthritis) is characterised by increased o-glycosylation in joint tissues, cartilage tissue, and/or synovial fibroblasts, the affected subject has a greater level of o-glycosylation in joint tissues, cartilage tissue, and/or synovial fibroblasts when compared to a corresponding to a corresponding control (e.g. subject unaffected by the disorder). The level of o-glycosylation in joint tissues, cartilage tissue, and/or synovial fibroblasts may be greater than 1 times, e.g. one of ≥1.1 times, ≥1.2 times, ≥1.3 times, ≥1.4 times, ≥1.5 times, ≥1.6 times, ≥1.7 times, ≥1.8 times, ≥1.9 times, ≥2.0 times, ≥2.5 times, ≥3.0 times, ≥3.5 times, ≥4.0 times, ≥4.5 times, ≥5.0 times, ≥5.5 times, ≥6.0 times, ≥6.5 times, ≥7.0 times, ≥7.5 times, ≥8.0 times, ≥8.5 times, ≥9.0 times, ≥9.5 times, or ≥10 times compared to a subject unaffected by the disorder.

If a disorder (e.g. a disorder associated with cartilage degradation, a joint disorder, and/or arthritis) is characterised by increased cellular levels of Tn (T nouvelle), the affected subject has a greater level of Tn (T nouvelle) when compared to a corresponding to a corresponding control (e.g. subject unaffected by the disorder). The level of Tn (T nouvelle) may be greater than 1 times, e.g. one of ≥1.1 times, ≥1.2 times, ≥1.3 times, ≥1.4 times, ≥1.5 times, ≥1.6 times, ≥1.7 times, ≥1.8 times, ≥1.9 times, ≥2.0 times, ≥2.5 times, ≥3.0 times, ≥3.5 times, ≥4.0 times, ≥4.5 times, ≥5.0 times, ≥5.5 times, ≥6.0 times, ≥6.5 times, ≥7.0 times, ≥7.5 times, ≥8.0 times, ≥8.5 times, ≥9.0 times, ≥9.5 times, or ≥10 times compared to a subject unaffected by the disorder.

If a disorder (e.g. a disorder associated with cartilage degradation, a joint disorder, and/or arthritis) is characterised by increased cellular levels of Tn (T nouvelle) in joint tissues, cartilage tissue, and/or synovial fibroblasts, the affected subject has a greater level of Tn (T nouvelle) in joint tissues and/or synovial fibroblasts when compared to a corresponding to a corresponding control (e.g. subject unaffected by the disorder). The level of Tn (T nouvelle) in joint tissues and/or synovial fibroblasts may be greater than 1 times, e.g. one of ≥1.1 times, ≥1.2 times, ≥1.3 times, ≥1.4 times, ≥1.5 times, ≥1.6 times, ≥1.7 times, ≥1.8 times, ≥1.9 times, ≥2.0 times, ≥2.5 times, ≥3.0 times, ≥3.5 times, ≥4.0 times, ≥4.5 times, ≥5.0 times, ≥5.5 times, ≥6.0 times, ≥6.5 times, ≥7.0 times, ≥7.5 times, ≥8.0 times, ≥8.5 times, ≥9.0 times, ≥9.5 times, or ≥10 times compared to a subject unaffected by the disorder.

If a disorder (e.g. a disorder associated with cartilage degradation, a joint disorder, and/or arthritis) is characterised by increased levels of CNX glycosylation, the affected subject has a greater level of CNX glycosylation when compared to a corresponding to a corresponding control (e.g. subject unaffected by the disorder). The level of CNX glycosylation may be greater than 1 times, e.g. one of ≥1.1 times, ≥1.2 times, ≥1.3 times, ≥1.4 times, ≥1.5 times, ≥1.6 times, ≥1.7 times, ≥1.8 times, ≥1.9 times, ≥2.0 times, ≥2.5 times, ≥3.0 times, ≥3.5 times, ≥4.0 times, ≥4.5 times, ≥5.0 times, ≥5.5 times, ≥6.0 times, ≥6.5 times, ≥7.0 times, ≥7.5 times, ≥8.0 times, ≥8.5 times, ≥9.0 times, ≥9.5 times, or ≥10 times compared to a subject unaffected by the disorder.

If a disorder (e.g. a disorder associated with cartilage degradation, a joint disorder, and/or arthritis) is characterised by increased levels of CNX glycosylation in synovial fibroblasts, the affected subject has a greater level of CNX glycosylation in synovial fibroblasts when compared to a corresponding to a corresponding control (e.g. subject unaffected by the disorder). The level of CNX glycosylation in synovial fibroblasts may be greater than 1 times, e.g. one of ≥1.1 times, ≥1.2 times, ≥1.3 times, ≥1.4 times, ≥1.5 times, ≥1.6 times, ≥1.7 times, ≥1.8 times, ≥1.9 times, ≥2.0 times, ≥2.5 times, ≥3.0 times, ≥3.5 times, ≥4.0 times, ≥4.5 times, ≥5.0 times, ≥5.5 times, ≥6.0 times, ≥6.5 times, ≥7.0 times, ≥7.5 times, ≥8.0 times, ≥8.5 times, ≥9.0 times, ≥9.5 times, or ≥10 times compared to a subject unaffected by the disorder.

If a disorder (e.g. a disorder associated with cartilage degradation, a joint disorder, and/or arthritis) is characterised by increased levels of CNX cell surface expression, the affected subject has a greater level of CNX cell surface expression when compared to a corresponding to a corresponding control (e.g. subject unaffected by the disorder). The level of CNX cell surface expression may be greater than 1 times, e.g. one of ≥1.1 times, ≥1.2 times, ≥1.3 times, ≥1.4 times, ≥1.5 times, ≥1.6 times, ≥1.7 times, ≥1.8 times, ≥1.9 times, ≥2.0 times, ≥2.5 times, ≥3.0 times, ≥3.5 times, ≥4.0 times, ≥4.5 times, ≥5.0 times, ≥5.5 times, ≥6.0 times, ≥6.5 times, ≥7.0 times, ≥7.5 times, ≥8.0 times, ≥8.5 times, ≥9.0 times, ≥9.5 times, or ≥10 times compared to a subject unaffected by the disorder.

If a disorder (e.g. a disorder associated with cartilage degradation, a joint disorder, and/or arthritis) is characterised by increased levels of CNX cell surface expression in synovial fibroblasts, the affected subject has a greater level of CNX cell surface expression in synovial fibroblasts when compared to a corresponding to a corresponding control (e.g. subject unaffected by the disorder). The level of CNX cell surface expression in synovial fibroblasts may be greater than 1 times, e.g. one of ≥1.1 times, ≥1.2 times, ≥1.3 times, ≥1.4 times, ≥1.5 times, ≥1.6 times, ≥1.7 times, ≥1.8 times, ≥1.9 times, ≥2.0 times, ≥2.5 times, ≥3.0 times, ≥3.5 times, ≥4.0 times, ≥4.5 times, ≥5.0 times, ≥5.5 times, ≥6.0 times, ≥6.5 times, ≥7.0 times, ≥7.5 times, ≥8.0 times, ≥8.5 times, ≥9.0 times, ≥9.5 times, or ≥10 times compared to a subject unaffected by the disorder.

If a disorder (e.g. a disorder associated with cartilage degradation, a joint disorder, and/or arthritis) is characterised by increased levels of cartilage ECM degradation, the affected subject has a greater level of ECM degradation when compared to a corresponding to a corresponding control (e.g. subject unaffected by the disorder). The level of cartilage ECM degradation may be greater than 1 times, e.g. one of ≥1.1 times, ≥1.2 times, ≥1.3 times, ≥1.4 times, ≥1.5 times, ≥1.6 times, ≥1.7 times, ≥1.8 times, ≥1.9 times, ≥2.0 times, ≥2.5 times, ≥3.0 times, ≥3.5 times, ≥4.0 times, ≥4.5 times, ≥5.0 times, ≥5.5 times, ≥6.0 times, ≥6.5 times, ≥7.0 times, ≥7.5 times, ≥8.0 times, ≥8.5 times, ≥9.0 times, ≥9.5 times, ≥10 times, ≥20 times, ≥30 times, ≥40 times, ≥50 times, ≥60 times, ≥70 times, ≥80 times, ≥90 times, or ≥100 times compared to a subject unaffected by the disorder.

If a disorder (e.g. a disorder associated with cartilage degradation, a joint disorder, and/or arthritis) is characterised by increased levels of ECM degradation activity, the affected subject has a greater level of ECM degradation activity when compared to a corresponding to a corresponding control (e.g. subject unaffected by the disorder). The level of ECM degradation activity may be greater than 1 times, e.g. one of ≥1.1 times, ≥1.2 times, ≥1.3 times, ≥1.4 times, ≥1.5 times, ≥1.6 times, ≥1.7 times, ≥1.8 times, ≥1.9 times, ≥2.0 times, ≥2.5 times, ≥3.0 times, ≥3.5 times, ≥4.0 times, ≥4.5 times, ≥5.0 times, ≥5.5 times, ≥6.0 times, ≥6.5 times, ≥7.0 times, ≥7.5 times, ≥8.0 times, ≥8.5 times, ≥9.0 times, ≥9.5 times, ≥10 times, ≥20 times, ≥30 times, ≥40 times, ≥50 times, ≥60 times, ≥70 times, ≥80 times, ≥90 times, or ≥100 times compared to a subject unaffected by the disorder.

If a disorder (e.g. a disorder associated with cartilage degradation, a joint disorder, and/or arthritis) is characterised by increased levels of an ECM degradation activity of CNX, CNX/ERP57, and/or synovial fibroblasts, the affected subject has a greater level of an ECM degradation activity of CNX, CNX/ERP57, and/or synovial fibroblasts when compared to a corresponding to a corresponding control (e.g. subject unaffected by the disorder). The level of an ECM degradation activity of CNX, CNX/ERP57, and/or synovial fibroblasts may be greater than 1 times, e.g. one of ≥1.1 times, ≥1.2 times, ≥1.3 times, ≥1.4 times, ≥1.5 times, ≥1.6 times, ≥1.7 times, ≥1.8 times, ≥1.9 times, ≥2.0 times, ≥2.5 times, ≥3.0 times, ≥3.5 times, ≥4.0 times, ≥4.5 times, ≥5.0 times, ≥5.5 times, ≥6.0 times, ≥6.5 times, ≥7.0 times, ≥7.5 times, ≥8.0 times, ≥8.5 times, ≥9.0 times, ≥9.5 times, or ≥10 times compared to a subject unaffected by the disorder.

When comparing a patient or subject with a disorder to a subject without the disorder, an assay or test can be used to determine the level of the characteristic of interest in both subjects and determine if the characteristic of interest is increased in the subject with the disorder. Many assays are known in the art which can determine relative levels of biological characteristics.

The skilled person would be able to determine the relative levels of characteristics such as ECM degradation, glycosylation, O-glycosylation, gene expression (e.g. calnexin, PDIA4, and/or PDIA3 expression), protein expression, GALA, and inflammation through well known methods in the art.

The skilled person could also use methods that are well known in the art to determine whether a characteristic is present in a disorder. For example, the skilled person could use well known methods to determine whether a disorder is associated with the presence of ECM degradation, glycosylation, O-glycosylation, the expression of a specific gene (e.g. calnexin expression), the expression of a specific protein (e.g. calnexin), GALA, and inflammation through methods known in the art.

The presence and level of ECM degradation can be determined through a number of methods, such as ECM degradation assays and histological analysis. The skilled person is aware of a number of ways in which ECM degradation activity can be measured, for example those shown in the examples of this specification. Such methods could readily determine whether a Cnx/ERp57 inhibitor is capable of inhibiting ECM degradation activity, an ECM degradation activity of Cnx/ERp57, and/or an ECM degradation activity of Cnx.

The following steps are one way in which the skilled person can assay for ECM degradation activity.

A commercial solution of gelatin (2%) can be labeled with 5-Carboxy-X-Rhodamine, Succinimidyl Ester. The labeled gelatin can then be transferred on sterile coverslips to create a thin layer, before being stabilised by glutaraldehyde fixation. Finally, a solution of rat tail collagen can be used to coat the coverslips, creating a thin layer of collagen on top of the gelatin.

Then, the coverslips can be transferred in culture vessels and cells with degradative activity (for instance human hepatocellular carcinoma Huh7) are seeded and incubated for 48h to allow for degradation to occur.

After fixation, the coverslips can be stained with Hoescht to permit the counting of cells. The coverslips can then be imaged on a confocal microscope. The images can then be analysed using ImageJ. A threshold can be defined manually to reveal the surface of degraded gelatin and the total area per field was measured. In parallel, the number of nuclei can be calculated and the final result can be normalised to the cells in each field.

The presence and level of glycosylation can be determined by methods well known in the art, such as methods used in the examples and commercial assay kits. Methods to detect and analyze glycosylation and glycoproteins include: glycan staining using lectins, glycan labelling, glycoprotein purification or enrichment, high-performance liquid chromatography (HPLC) analysis, and analysis by mass spectrometry. The skilled person would be aware of such methods.

The presence and level of O-glycosylation can be determined through methods known in the art, such as those used in the examples of this specification. A hallmark of O-glycosylation is increased cellular levels of Tn (T nouvelle), the O-glycan formed by the addition of a GalNac to a Ser or Thr residue. Tn can be detected by Tn-binding proteins such as Vicia Villosa Lectin (VVL) and Helix Pomatia Lectin (HPL) (Gill, et al., Proc. Natl. Acad. Sci. U. S. A. 110, E3152-61 2013).

The presence and level of gene expression (e.g. calnexin expression) can be determined through a number of different methods known by the skilled person. The level of RNA encoding a given gene can be determined e.g. by techniques such as RNAseq, RT-PCR, and RT-qPCR amongst other well known methods.

The presence and level of protein expression (e.g. calnexin expression) can be determined by means well known to the skilled person. The level of a given protein/isoform thereof can be determined e.g. by antibody-based methods including western blot, immunohisto/cytochemistry, flow cytometry, ELISA, etc.

The presence and level of GALA can be determined through methods known in the art, such as those used in the examples of this specification. A hallmark of GALA is increased cellular levels of Tn (T nouvelle), the O-glycan formed by the addition of a GalNac to a Ser or Thr residue. Tn can be detected by Tn-binding proteins such as Vicia Villosa Lectin (VVL) and Helix Pomatia Lectin (HPL) (Gill, et al., Proc. Natl. Acad. Sci. U. S. A. 110, E3152-61 2013).

The presence and level of inflammation can be determined through methods known in the art. For example, through the analysis of biomarkers such as cytokines, chemokines, and immune cells. Such analysis can be performed through the use of techniques known in the art, such as mass spectrometry.

### Mediators of disorders (e.g. fibroblasts, synovial fibroblasts)

Disorders described herein may be mediated by fibroblast activity. Fibroblasts are cells that synthesise collagen and the ECM. However, fibroblasts can also degrade the ECM, allowing the turn-over of this essential component of tissues. Fibroblasts contribute to the health of joints and cartilage. In healthy individuals, fibroblasts contribute to the viscosity of the synovial fluid by secreting proteins such as hyaluronic acid and lubricin. In joint disorders and cartilage disorders, such as arthritis, the fibroblasts adhere and degrade the ECM of the cartilage.

In some cases, the disorders described herein may be mediated by synovial fibroblasts (SF). SFs, also called synoviocytes, are the primary fibroblasts in synovial joints.

The skilled person can determine whether a disease is mediated by fibroblast activity through methods known in the art. For example, the skilled person would be aware of biochemical assays suitable to determine the level of fibroblast activity. The fibroblast activity of known diseases could be assayed to determine whether the disease is associated with increased fibroblast activity when compared to a suitable control (e.g. a non-affected subject), indicating that the disease is mediated by fibroblast activity. Alternatively, animal models of a certain disease could be utilised to determine whether a disease is mediated by fibroblast activity. For example, a mouse model of a disease of interest could be modified to ablate the fibroblast population (e.g. a synovial fibroblast population). Analysis of the modified model compared to the unmodified model could be used to determine whether a disease is mediated by fibroblast activity. For example, a reduction of disease characteristics observed after ablation of the fibroblast population would indicate that the disease is mediated by fibroblast activity.

### Arthritis

Rheumatoid arthritis (RA), an auto-immune disease, and osteoarthritis (OA), a degenerative disease, are both characterised by the degradation of joint cartilage.

Rheumatoid arthritis is understood as an autoimmune, inflammatory condition. The affected joint of rheumatoid arthritis patients is infiltrated by immune cells such as, but not limited to, macrophages, T-cells, and B-cells. These cells are enriched in an enlarged synovial membrane called a pannus. Consistent with an autoimmune disease, autoantibodies are found in the serum of patients suffering rheumatoid arthritis. These autoantibodies are presumed to form immune complexes in the joint, resulting in the activation of immune cells. However, not all rheumatoid arthritis patients present detectable autoantibodies, suggesting a complex aetiology.

Without being bound by theory, osteoarthritis, by contrast, is thought to occur mostly independently of inflammation and immune cells, although inflammation can also be present. Osteoarthritis is further thought to derive from mechanical damage of the cartilage, leading to a progressive degradation of the cartilage and, eventually, degradation of the bones.

While rheumatoid arthritis and osteoarthritis are different diseases, they are understood to share cellular and molecular characteristics. For example, cartilage extracellular matrix damage is the common pathological feature and typical early manifestation of both osteoarthritis and rheumatoid arthritis. The key pathological feature of arthritis in general is the breakdown of cartilage. This breakdown begins with the degradation of the cartilage extracellular matrix (ECM), and can eventually lead to the loss of chondrocytes, the cells that synthesise and maintain the cartilage. In addition, as the disease progresses, it can also lead to bone erosion and loss. Hence, the method disclosed herein is taken to be used in treatment carried out during the onset of the diseases to prevent cartilage damage, up until late stages of the diseases disclosed herein.

In this application, evidence of activation of the GALA pathway in human samples of arthritis, for example, rheumatoid arthritis and osteoarthritis joint tissues, is shown. Synovial fibroblasts, which are the cause of cartilage degradation, rely on the GALA pathway to activate matrix degradation. This is thought to happen, in particular, through the cell surface exposure of the Calnexin (CNX) complex (that is, the CNX-PDIA3 complex), which mediates disulfide bond reduction. In other words, the inhibition of the disulphide isomerase activity of the CNX-PDIA3 complex is thought to ameliorate cartilage degradation. Genetic inhibition of the GALA pathway in synovial fibroblasts has been shown to block matrix degradation *in vitro* and reduce arthritic symptoms *in vivo.* Targeting Calnexin (CNX) using antibodies, for example, produced similar results, indicating a therapeutic approach. Overall, the results disclosed herein show that the GALA glycosylation pathway is a key determinant of arthritic pathologies.

### Treatment of disorders with GALA inhibitors

Also disclosed herein is the treatment of cartilage disorders, joint disorders, and arthritic conditions, such as, but not limited to, rheumatoid arthritis (RA) and osteoarthritis (OA). Currently, there is no cure for either rheumatoid arthritis (RA) or osteoarthritis (OA) patients. Rheumatoid arthritis patients are subject to disease-modifying anti-rheumatic drugs or immunomodulatory agents (for example, but not limited to, anti-TNF alpha and anti-IL-6) that primarily target the immune system. A major problem is the immunodeficiency induced by these treatments. And it has been shown that these known treatments are unable to completely halt disease progression and prevent disease recurrence. For example, patients with severe end stage symptomatic rheumatoid arthritis (RA) or osteoarthritis (OA), who have previously failed to respond to such anti-rheumatic drugs or immunomodulatory agents, have to undergo total joint replacement, which comes with a high risk of serious adverse events. Thus, therapies based on alternative pathways for patients with disorders associated with cartilage degradation, joint disorders, and arthritis are a highly important unmet medical need.

It was found that arthritis-active synovial fibroblasts (SF) display activation of the glycopathway known as GalNAc-T activation (GALA). GALA activation is present in several human rheumatoid arthritis, in most human osteoarthritis samples, and is induced in a mouse model of rheumatoid arthritis. In addition, inhibiting GalNAc-T activation (GALA) in synovial fibroblasts reduces cartilage degradation *in vitro.* For example, targeting a target of GalNAc-T activation (GALA), Calnexin, has been shown to be equally efficacious. *In vivo,* inhibiting GalNAc-T activation (GALA) in a mouse model of rheumatoid arthritis using a protein inhibitor, for example, the protein inhibitor ER-2Lec, leads to a near complete block of cartilage degradation.

Thus, it is shown that inhibiting GalNAc-T activation (GALA) in synovial fibroblasts has therapeutic application. In one example, there is disclosed a method of treating arthritis in a subject, wherein the method comprises administration of a therapeutic agent which inhibits the GalNAc-T activation (GALA) pathway. In another example, there is described the use of a therapeutic agent which inhibits the GalNAc-T activation (GALA) pathway in the manufacture of a medicament for treating arthritis. Also disclosed herein are one or more therapeutic agents which inhibit the GalNAc-T activation (GALA) pathway for use in treating arthritis.

In one example of therapeutic applications, nucleic acid sequences encoding the protein ER-2Lec are transduced or transfected into synovial fibroblasts using, for example, but not limited to, adeno-associated virus (AAV) or lentiviral transfection systems, or any other genetic delivery methods. In another example, a therapeutic application is to inhibit one or more of the targets selected from, but not limited to, Calnexin (CNX), Protein disulfide-isomerase A4 (PDIA4), Protein disulfide-isomerase A3 (PDIA3), the thioreductase activity of the Calnexin-PDIA3 complex, inhibiting glycosylated MMP14 (for example, O-glycosylated MMP14), or any cell-surface bound GALA-relevant proteins on the surface of synovial fibroblasts. In one example, such an inhibition can be brought about or caused by using blocking antibodies, fragments thereof, or derivatives thereof.

Thus, in one example, the method or use is as described herein, wherein the therapeutic agent inhibits any one or more of the cell surface markers involved in the GalNAc-T activation (GALA) pathway.

In another example, the targets of treatment for disorders associated with cartilage degradation, joint disorders, and arthritis, as disclosed herein, include targeting Calnexin (CNX), PDIA4, PDIA3, and/or a complex of CNX and PDIA3. In another example, the targets are cell-surface bound or presenting Calnexin (CNX), PDIA4, and/or PDIA3.

In addition to targeting the immune system, regulation the synovium itself, by for instance targeting cadherin 11, has also been contemplated.

The synovium is formed by the enlargement of the synovial membrane. Normally restricted at the periphery of the joint area, during arthritis, this tissue invades the joint area and attacks the cartilage. The synovium contains multiple cell types, but the key cells thought to be involved in extracellular matrix (ECM) degradation are the synovial fibroblasts. While synovial fibroblasts are normally relatively few and quiescent, in arthritic conditions, these cells proliferate and result in an extracellular matrix-degradative activity. What induces synovial fibroblasts to switch on their extracellular matrix-degradative activity is not well understood.

Extracellular matrix (ECM) degradation is mediated by cell surface and secreted proteases, in particular matrix metalloproteases (MMP), such as, but not limited to, MMP14, and O-glycosylated MMP14. These proteins become particularly active in cells in solid malignant tumours. Indeed, it has been shown that cancer growth requires a profound remodelling of the extracellular matrix in the tissue of origin, with degradation and synthesis of new extracellular matrix.

It had been shown that (extracellular) matrix degradation in cancer cells is controlled by a glycopathway known as GALA (for GALNT Activation). GALA regulates the activity of GALNTs, the enzymes that initiate O-glycosylation initiation and the addition of complex sugars on various proteins. The regulation of the GALA pathway (and by extension, the degradation of extracellular matrix in the cancer cell) is mediated through intracellular distribution, with GALNTs being relocated from the Golgi to the endoplasmic reticulum (ER). The relocation results in various substrates becoming more readily O-glycosylated.

One of the exemplary targets of GalNAc-T activation (GALA) is matrix metalloproteinase-14 (MMP14), whose O-glycosylation has been shown to be essential for its proteolytic activity. Another target of GALA is the endoplasmic reticulum-located protein Calnexin (CNX), which forms a complex with ERp57, also known as Protein disulfide-isomerase A3 (PDIA3). Following glycosylation, this complex is translocated to the surface of cancer cells, where it reduces disulfide bridges in the extracellular matrix. The reduction of disulfide bridges has been shown to be essential for the effective activity of matrix metalloproteinases (MMPs) and thus for the degradation of the extracellular matrix. Therefore, GalNAc-T activation (GALA) has been shown to coordinate or combine two enzymatic activities, one proteolytic and one reducing disulfide bonds.

Thus, in one example, the therapeutic agent disclosed herein inhibits one or more of the following targets of the GalNAc-T activation (GALA) pathway selected from the group consisting of Calnexin (CNX), Protein disulfide-isomerase A4 (PDIA4), Protein disulfide-isomerase A3 (PDIA3), a Calnexin-PDIA3 (CNX:PDIA3) complex, glycosylated matrix metalloproteinase-14 (MMP14), including O-glycosylated matrix metalloproteinase-14 (MMP14), and combinations thereof. In one example, the therapeutic agent inhibits Calnexin (CNX). In another example, the therapeutic agent inhibits PDIA3. In a further example, the therapeutic agent inhibits a Calnexin-PDIA3 complex. In yet another example, the therapeutic agent inhibits Calnexin and PDIA3. In one example, the therapeutic agent inhibits glycosylated MMP14

In the experimental data described herein, it is shown that arthritis-active synovial fibroblasts display signs of marked activation of GALA. GalNAc-T activation (GALA) hallmarks are present in several human rheumatoid arthritis and in most human osteoarthritis samples. GALA is induced in a mouse model of rheumatoid arthritis, preceding the appearance of symptoms. In addition, inhibiting GALA in synovial fibroblasts using the protein inhibitor ER-2Lec has been shown to reduce cartilage degradation *in vitro. In vivo,* inhibiting GALA in a mouse model of rheumatoid arthritis using ER-2Lec expressed in synovial fibroblasts has been shown to specifically prevent disease progression, thereby also confirming the role of GALA in the disease. Finally, in another example, targeting Calnexin using antibodies has been shown to lead to a near complete block of cartilage degradation, indicating the use of these concepts in therapy.

Osteoarthritis and rheumatoid arthritis patients share some common pathological features and have shown overlapping cellular and molecular characteristics. One pathological feature of arthritis is the breakdown of cartilage extracellular matrix (ECM) that occurs in the onset of the disease, subsequently causing irreversible bone loss. Synovial fibroblasts have been reported to be a synoviocyte subset, which have been shown to cause damage to cartilage extracellular matrix through direct invasion, or though secretion of matrix remodelling enzymes (for example, but not limited to, MMP14, ADAMTS).

It has been shown that the relocation of N-acetylgalactosaminyltransferase (GalNAc-T) enzymes from the Golgi to the endoplasmic reticulum (ER), also known as the GalNAc-T activation (GALA) pathway, is the main regulator of tumour extracellular matrix (ECM) remodelling. GalNAc-Ts catalyse the addition of a single sugar on serine (Ser) and/or threonine (Thr) residues. The resulting glycan, called a Tn antigen, is enriched in the endoplasmic reticulum (ER) of GALA-activated cells, and serves as a marker of GALA activation. To test the GALA activation state in human samples of arthritis patients, the levels of Tn glycan were assessed in commercially obtained synovial tissues of rheumatoid arthritis and osteoarthritis patients. It was shown that rheumatoid arthritis patients represented a trend towards increased median levels of Tn (O-GalNac) glycans compared to healthy subjects (0.34 versus 0.24, p = 0.06, Fig. 21). The median levels of Tn glycans in osteoarthritis patients were significantly higher than both healthy subject (0.59 versus 0.24, p=0.002, Fig. 21) and rheumatoid arthritis patients (0.59 versus 0.34, p=0.01). It is noted that the majority of osteoarthritis patients (19/21, 90.4%) had the median levels of Tn glycan higher than that of healthy subjects (Fig. 21). These results show that arthritis patients displayed high GALA activation in synovial tissues compared to healthy individuals, and that osteoarthritis patients presented hyper-activation of GALA.

An *in vivo* preclinical model of collagen antibody induced arthritis was employed to understand the functional contribution of GALA activation in arthritis. Consistent with the findings in clinical samples as outlined above, increased Tn levels were observed in arthritis induced joints (Fig. 2B). This induction correlated with arthritis severity, reaching its peak on day 7-10 (Fig. 2C and 2D). It was further shown that Tn glycan staining in control joints displayed a localisation to the Golgi complex, while Tn glycan staining in arthritis showed co-localisation of Tn glycan with the endoplasmic reticulum-resident protein Calnexin (CNX). Taken together, these localisations are considered to be indicative of the GALA activation state (Fig. 3, zoomed panels). In addition, synovial fibroblasts in the pannus were identified as the major cell subset displaying GALA activation in arthritis joints (Fig. 4).

To specifically inhibit the GalNAc-T activation (GALA) pathway in synovial fibroblasts cells, a genetically modified mouse line was engineered to express an inhibitor of GALA (two lectin domains of GalNAc-T2) exclusively in the endoplasmic reticulum of synovial fibroblasts by using a Cre/LoxP system (Fig. 8A). It was observed that inhibition of the GALA pathway using such an approach was able to restore the integrity of cartilage extracellular matrix and to reduce the severity of arthritis diseases (Fig. 8B to 8H). Together, these results show that the GALA pathway plays an important role in cartilage extracellular matrix degradation in arthritic diseases.

The GalNAc-T activation (GALA) pathway regulates the glycosylation of several cell surface and secreted proteins. For example, the GALA pathway has been shown to hyper-glycosylate and activate the matrix metalloprotease MMP14, a driver of extracellular matrix degradation both in cancer and in rheumatoid arthritis. Upon activation of the GalNAc-T (GALA) pathway, it was shown that endoplasmic reticulum-resident proteins (for example, but not limited to, Calnexin (CNX), PDIA3, PDIA4) were hyper-O-glycosylated. Furthermore, GALA-activated Calnexin (CNX) proteins were shown to relocate to cell surface and form a complex with the Protein disulfide-isomerase A3 (PDIA3), driving the degradation of tumour extracellular matrix components via reduction of the disulfide bonds of matrix component proteins. Without being bound by theory, MMP14 and PDIA4 are targets of GALA pathway modulation/inhibition in the same way that Calnexin (CNX) and PDIA3 have been shown to be targets of GALA pathway modulation/inhibition. Therefore, glycosylated MMP14 is also a target of GALA pathway modulation/inhibition. PDIA4, being a paralogue of PDIA3, functions similarly to PDIA3, and is also a target for arthritis treatment, similar to PDIA3. In one example, the arthritis is rheumatoid arthritis.

To exploit this concept, a high-throughput screening assay was developed to select the most appropriate GALA-relevant targets for arthritis treatment. In this assay, synovial fibroblast cells (cell line SW982) were cultured on a quenched fluorescent cartilage matrix component (in this example, fluorogenic DQ-Collagen) in the presence of GALA target blockers (for example, but not limited to, antibodies or siRNA). The degradation activity of synovial fibroblast cells results in an increase fluorescence signal, which was quantitatively captured using a high content imaging system (Fig. 21A). Consistent with previous findings in liver tumour, this screen identified three GALA-relevant targets, with Calnexin, PDIA3 and MMP14 being amongst the top hits that function as positive regulators of cartilage degradation in synovial fibroblasts (Fig. 21C).

From the screen as described above, GALA target inhibitors for inhibiting degradation activity of cartilage extracellular matrix components in synovial fibroblasts were identified. Of those inhibitors identified, a monoclonal antibody clone targeting Calnexin (CNX) was selected to conduct the follow-up validation of the therapeutic application.

Thus, in one example, the target of the GALA pathway is Calnexin (CNX) and/or Protein disulfide-isomerase A3 (PDIA3).

In a further example, there is disclosed a method of identifying (a) a molecule which inhibits one or more targets of the GalNAc-T activation (GALA) pathway, the method comprising contacting the one or more of the targets with the molecule and determining whether the molecule binds inhibits the one or more targets; (b) a suppressor of the GalNAc-T activation (GALA) pathway, the method comprising contacting a cell with the suppressor and detecting reduced expression, amount or activity of the one or more of the targets of the GalNAc-T activation (GALA) pathway in, on, or of the cell; (c) a molecule suitable for or suitable for the treatment, prophylaxis or alleviation of arthritis, the method comprising determining if a candidate molecule is an agonist or antagonist of Calnexin (CNX), Protein disulfide-isomerase A3 (PDIA3), Protein disulfide-isomerase A4 (PDIA4), or Calnexin (CNX) and Protein disulfide-isomerase A3 (PDIA3), preferably by exposing a candidate molecule to CNX, PDIA4, PDIA3, or CNX and PDIA3 or a cell expressing CNX, PDIA4, PDIA3, or CNX and PDIA3 in order to determine if the candidate molecule is an agonist or antagonist thereof; or (d) an agonist or antagonist of a CNX, PDIA4, PDIA3, or CNX and PDIA3, the method comprising administering a candidate molecule to an animal and determining whether the animal exhibits increased or decreased expression, amount or activity of CNX, PDIA4, PDIA3, or CNX and PDIA3; wherein the one or more targets of the GalNAc-T activation (GALA) pathway are selected from the group consisting of Calnexin (CNX), Protein disulfide-isomerase A3 (PDIA3), Protein disulfide-isomerase A4 (PDIA4), a CNX:PDIA3 complex, and O-glycosylated Matrix metalloproteinase-14 (MMP14). In another example, the method disclosed herein optionally includes the step of isolating or synthesising the molecule, modulator, agonist or antagonist.

In one example, there is disclosed a method of identifying (a) a molecule which inhibits one or more targets of the GalNAc-T activation (GALA) pathway, the method comprising contacting the one or more of the targets with the molecule and determining whether the molecule binds inhibits the one or more targets; (b) a suppressor of the GalNAc-T activation (GALA) pathway, the method comprising contacting a diseased cell with the suppressor and detecting reduced expression, amount or activity of the one or more of the targets of the GalNAc-T activation (GALA) pathway in, on, or of the cell, wherein the disease is arthritis; (c) a molecule suitable for or for the treatment, prophylaxis or alleviation of arthritis, the method comprising determining if a candidate molecule is an agonist or antagonist of Calnexin (CNX), Protein disulfide-isomerase A3 (PDIA3), Protein disulfide-isomerase A4 (PDIA4), or Calnexin (CNX) and Protein disulfide-isomerase A3 (PDIA3), preferably by exposing a candidate molecule to CNX, PDIA4, PDIA3, or CNX and PDIA3 or a cell expressing CNX, PDIA4, PDIA3, or CNX and PDIA3 in order to determine if the candidate molecule is an agonist or antagonist thereof; or (d)an agonist or antagonist of a CNX, PDIA4, PDIA3, or CNX and PDIA3, the method comprising administering a candidate molecule to an arthritis animal model and determining whether the arthritis animal model exhibits increased or decreased expression, amount or activity of CNX, PDIA4, PDIA3, or CNX and PDIA3; wherein the one or more targets of the GalNAc-T activation (GALA) pathway are selected from the group consisting of Calnexin (CNX), Protein disulfide-isomerase A4 (PDIA4), Protein disulfide-isomerase A3 (PDIA3), a Calnexin-PDIA3 complex, and O-glycosylated Matrix metalloproteinase-14 (MMP14); wherein the arthritis is osteoarthritis, psoriasis arthritis, or rheumatoid arthritis; wherein the method optionally comprising isolating or synthesising the molecule, modulator, agonist or antagonist.

In yet another example, the method disclosed herein is a method of identifying or screening a library for compounds or molecules that are capable of inhibiting one or more targets of the GalNAc-T activation (GALA) pathway. In another example, the compounds or molecules identified using the screening method disclosed herein are to be administered to an animal or a subject.

To model the complex physiological features of cartilage degradation activity of synovial fibroblasts in the context of arthritis, primary synovial fibroblasts were isolated from an osteoarthritis patient with activate disease symptoms (also referred to as OASF cells). Cultured OASF cells displayed high GalNAc-T pathway activation (Fig. 21B), consistent with previous observations in synovial tissue, and exhibited highly active dye quenching (DQ)-collagen matrix degradation activity. It was found the selected antibody clone inhibited cartilage degradation activity in the cultured OASF cells (p=0.001, Fig. 21C).

Taken together, this data indicates that the GalNAc-T (GALA) activation pathway impacts cartilage degradation in arthritis. Therefore, targeting the GALA activation pathway serves as a therapeutic target and approach for arthritis treatment.

### Enhanced O-glycosylation in the synovium in rheumatoid arthritis and osteoarthritis

As the GalNAc-T pathway is involved in extracellular matrix degradation in cancer cells, it is thought that the same pathway is also involved in cartilage degradation during arthritic episodes and arthritis. A marker of GalNAc-T pathway activation is increased levels of Tn (also referred to as Tn antigen), the single sugar O-glycan formed by the addition of a GalNac to a serine (Ser) or threonine (Thr) residue. Tn can be detected by for example, lectins, such as, but not limited to, *Vicia villosa* lectin (VVL) and *Helix pomatia* lectin (HPL).

Tissue microarrays of joint tissue were analysed by immunofluorescence using *Vicia villosa* lectin (VVL) as a counterstain for DNA. A clear signal increase was detected in samples from patients suffering from osteoarthritis, and in several samples of rheumatoid arthritis patients (Fig. 1A). Next, the integrated fluorescence intensity of *Vicia villosa* lectin staining, normalised to the DNA signal intensity as a marker of cell density, was quantified (Fig. 1B). While healthy patient samples showed little variation, most, if not all, samples of diseased patients displayed increased Tn levels, with some rheumatoid arthritis and osteoarthritis samples displaying a five- to seven-fold increase in *Vicia villosa* lectin signal.

To further study this correlation, a mouse model of rheumatoid arthritis based on Collagen Antibody Induced Arthritis (CAIA) was employed. Briefly, mice are injected with an antibody against collagen type II, then injected three days later with lipopolysaccharide (LPS). This treatment leads to arthritic symptoms, such as but not limited to, swollen paws and lameness at day 7, persisting until day 14 before progressively resolving (Fig. 2A, 2C). Histologically, the formation of a pannus invading the joint cavity was observed at day 7. The pannus increased in size, and inflammation level appeared to increase based on the influx of immune cells at day 10, with the pannus persisting up to day 14 (Fig. 2A). Using immunofluorescence labelling, an increase in Tn staining was observed at day 7, specifically in the invading pannus (Fig. 2B). The Tn staining persisted at day 10 and receded at day 14 (Fig. 2B, 2D).

### Arthritic synovium display signs of GALA pathway activation

GalNAc-T pathway activation is a well-described mechanism leading to increased Tn levels. One of the hallmarks of GALA pathway activation is the redistribution of N-acetylgalactosaminyltransferases (GALNTs), in particular GALNT1 and GALNT2, from the Golgi apparatus to the endoplasmic reticulum. To verify that the GalNAc-T (GALA) pathway was indeed activated, co-staining of *Vicia villosa* lectin (VVL) with Calnexin, an endoplasmic reticulum marker, was performed (Fig. 3A). In untreated samples, *Vicia villosa* lectin staining and Calnexin staining were clearly separated, with *Vicia villosa* lectin being concentrated in a dotted pattern consistent with the Golgi complex (Fig. 3A). By contrast, in day 7 Collagen Antibody Induced Arthritis (CAIA) samples, the *Vicia villosa* lectin staining and the Calnexin staining were clearly co-localised. Next, a similar analysis with GALNT2 staining was performed. An increase in GALNT2 staining intensity was observed, indicating an increase in the levels of the GALNT2 enzyme in disease conditions (Fig. 3B). In addition, the distribution pattern of the enzyme followed a similar shift as *Vicia villosa* lectin, redistributing into an endoplasmic reticulum-like pattern and co-localising with Calnexin (Fig 3B).

Thus, in one example, the identification of endoplasmic reticulum-localised GALNT1 and GALNT2 are used as targets for rheumatoid arthritis and osteoarthritis treatment. In another example, the target of the GALA pathway is Calnexin (CNX) and/or Protein disulfide-isomerase A3 (PDIA3).

In one example, there is disclosed a method of detecting the presence or absence of a disorder associated with cartilage degradation in a subject, wherein the method comprises the steps of (i) obtaining a synovial fibroblast sample from a subject; detecting the level of Tn antigen, Tn glycan, CNX cell surface expression, CNX glycosylation, and/or ECM degradation in the sample obtained in step (i); comparing the level of Tn antigen, Tn glycan, CNX cell surface expression, CNX glycosylation, and/or ECM degradation in step (ii) with the level of Tn antigen, Tn glycan, CNX cell surface expression, CNX glycosylation, and/or ECM degradation in a synovial fibroblast sample from a control group; wherein an increase in the level of Tn antigen, Tn glycan, CNX cell surface expression, CNX glycosylation, and/or ECM degradation present in the sample compared to the control group is indicative of the presence of a disorder associated with cartilage degradation; wherein the control group consists of subjects without a disorder associated with cartilage degradation.

In another example, there is disclosed a method of detecting the presence or absence of arthritis, optionally osteoarthritis or rheumatoid arthritis, in a subject, wherein the method comprises the steps of (i) obtaining a synovial fibroblast sample from a subject; (ii) detecting the level of Tn antigen, Tn glycan, CNX cell surface expression, CNX glycosylation, and/or ECM degradation in the sample obtained in step (i); comparing the level of Tn antigen, Tn glycan, CNX cell surface expression, CNX glycosylation, and/or ECM degradation in step (ii) with the level of Tn antigen, Tn glycan, CNX cell surface expression, CNX glycosylation, and/or ECM degradation in a synovial fibroblast sample from a control group; wherein an increase in the level of Tn antigen, Tn glycan, CNX cell surface expression, CNX glycosylation, and/or ECM degradation present in the sample compared to the control group is indicative of the presence of arthritis; wherein the control group consists of arthritis-free subjects.

In one example there is disclosed a method of detecting the presence or absence of a disorder associated with cartilage degradation in a subject, wherein the method comprises the steps of: (i) detecting the level of Tn antigen, Tn glycan, CNX cell surface expression, CNX glycosylation, and/or ECM degradation in a synovial fibroblast sample; (ii) comparing the level of Tn antigen, Tn glycan, CNX cell surface expression, CNX glycosylation, and/or ECM degradation in step (i) with the level of Tn antigen/Tn glycan in a synovial fibroblast sample from a control group; wherein an increase in the level of Tn antigen, Tn glycan, CNX expression, CNX glycosylation, and/or ECM degradation present in the sample compared to the control group is indicative of the suitability for treatment. The control group may consist of subjects without a disorder associated with cartilage degradation.

In another example there is disclosed a method of detecting the presence or absence of arthritis in a subject, optionally osteoarthritis or rheumatoid arthritis, wherein the method comprises the steps of: (i) detecting the level of Tn antigen, Tn glycan, CNX cell surface expression, CNX glycosylation, and/or ECM degradation in a synovial fibroblast sample; (ii) comparing the level of Tn antigen, Tn glycan, CNX cell surface expression, CNX glycosylation, and/or ECM degradation in step (i) with the level of Tn antigen/Tn glycan in a synovial fibroblast sample from a control group; wherein an increase in the level of Tn antigen, Tn glycan, CNX expression, CNX glycosylation, and/or ECM degradation present in the sample compared to the control group is indicative of the suitability for treatment. The control group may consist of subjects without arthritis, osteoarthritis or rheumatoid arthritis.

In one example, there is a method of determining whether a subject is suitable for treatment with an anti-cnx antibody through the following steps: (i) detecting the level of Tn antigen, Tn glycan, CNX cell surface expression, CNX glycosylation, and/or ECM degradation in a synovial fibroblast sample; (ii) comparing the level of Tn antigen, Tn glycan, CNX cell surface expression, CNX glycosylation, and/or ECM degradation in step (i) with the level of Tn antigen/Tn glycan in a synovial fibroblast sample from a control group; wherein an increase in the level of Tn antigen, Tn glycan, CNX expression, CNX glycosylation, and/or ECM degradation present in the sample compared to the control group is indicative of the suitability for treatment. The control group may consist of subjects without cartilage degradation, without a disorder associated with cartilage degradation, without arthritis, without osteoarthritis, or without rheumatoid arthritis.

In another example, there is a method of determining whether a subject is suitable for treatment with an anti-cnx antibody through the following steps: (i) obtaining a synovial fibroblast sample from a subject, (ii) detecting the level of Tn antigen, Tn glycan, CNX cell surface expression, CNX glycosylation, and/or ECM degradation in a synovial fibroblast sample; (iii) comparing the level of Tn antigen, Tn glycan, CNX cell surface expression, CNX glycosylation, and/or ECM degradation in step (ii) with the level of Tn antigen/Tn glycan in a synovial fibroblast sample from a control group; wherein an increase in the level of Tn antigen, Tn glycan, CNX expression, CNX glycosylation, and/or ECM degradation present in the sample compared to the control group is indicative of the suitability for treatment. The control group may consist of subjects without cartilage degradation, without a disorder associated with cartilage degradation, without arthritis, without osteoarthritis, or without rheumatoid arthritis.

In another example, there is disclosed a method of detecting the presence or absence of arthritis in a subject, wherein the method comprises the steps of detecting the level of Tn antigen/Tn glycan in a sample obtained from a subject; comparing the level of Tn antigen/Tn glycan detected in the preceding step with the level of Tn antigen/Tn glycan in a synovial fibroblast sample from a control group; wherein an increase in the level of Tn antigen/Tn glycan present in the sample compared to the control group is indicative of the presence of arthritis; wherein the control group consists of arthritis-free subjects.

### Synovial fibroblasts are the major cell type displaying GALA

The synovium in rheumatoid arthritis disease is a complex tissue comprising immune cells and synovial fibroblasts. To establish which cell types displayed increased GalNAc-T levels, Collagen Antibody Induced Arthritis (CAIA) joint samples were co-stained with *Vicia villosa* lectin (VVL) and CD45, a marker of immune cells, or vimentin, a marker of fibroblasts. The Tn positive cells in the pannus were, in a large majority, vimentin positive (vm⁺) and CD45 negative (CD45⁺) (Fig 4). A set of CD45+ cells was also detected, but these were usually located behind the front of the pannus. In human samples of osteoarthritis and rheumatoid arthritis suffering patients, Fibroblast Activated Protein alpha (FAPα) was used as a marker of synovial lining fibroblasts (Fig. 5A, 5B). *Vicia villosa* lectin (VVL) staining was shown to correlate with FAPα in both conditions. In rheumatoid arthritis samples, a CD45-rich compartment was prominent below the layer of FAPα-positive cells, but these immune cells were not significantly labelled by *Vicia villosa* lectin (Fig. 5A, 5B).

### Stimulation of synovial fibroblasts by cytokines and ECM induces higher GALA levels

To understand how the GalNAc-T pathway is activated *in vivo,* primary human synovial fibroblasts derived from patients were used. Fluorescence-activated cell sorting (FACS) analysis using CD90 and CD45 as markers of synovial fibroblasts and immune cells, respectively, established the >90% purity of the cell preparations used (Fig. 6A). Synovial fibroblasts were then stimulated using TNFα and IL1beta cytokines, which are thought to drive disease progression in rheumatoid arthritis. Individual cytokines had a limited effect on GALA activation, however the combination of both cytokines (labelled CYTO) induced a 2-fold increase in *Vicia villosa* lectin (VVL) cellular levels, in particular in rheumatoid arthritis synovial fibroblasts (RASF)(Fig. 6B, 6C). The pattern of *Vicia villosa* lectin staining was clearly endoplasmic reticulum-like, indicating GALA activation (Fig. 6B). Healthy synovial fibroblasts barely responded or did not respond, suggesting the patients' cells were primed for stimulation (Fig. 6B, 6C).

It was questioned whether cartilage extracellular matrix proteins could contribute to the activation of synovial fibroblasts. Indeed, exposing synovial fibroblasts to cartilage extracellular matrix activated the GalNAc-T pathway by almost 3-fold in both osteoarthritis synovial fibroblasts and rheumatoid arthritis synovial fibroblasts. Again, by contrast, the healthy patient cells were non-responsive or marginally responsive (Fig. 6B, 6C). The combination of CYTO (TNFα and IL1beta cytokines) and extracellular matrix had a slightly additive effect (Fig. 6C). The stimulation did not appear to be specific for cartilage extracellular matrix, as the use of rat tail derived collagen I induced a similar activation (Fig. 6C). That is to say, the combined stimulation with both cytokines and native cartilage extracellular matrix is thought to trigger the maximal GALA responses in synovial fibroblasts, with the latter being the strongest inducer, indicating that targeting the GALA is suitable for early onset rheumatoid arthritis patients who do not yet present active inflammation signs. Furthermore, this indicates that the treatment disclosed herein is also suitable for osteoarthritis patients in whom fibroblasts also display GALA activation, despite low level of cytokine production.

### Inhibition of GALA in synovial fibroblasts reduces cartilage extracellular matrix degradation

As the GalNAc-T pathway activation drives extracellular matrix degradation in cancer cells, it was sought to test whether it is also implicated in cartilage extracellular matrix degradation. This was done using a sandwich assay with fluorescent gelatine as previously described. ER-2Lec is a chimeric protein composed of an endoplasmic reticulum (ER)-targeting sequence and a fusion of two lectins of GALNT2. ER-2Lec inhibits the activity of GALA by interfering with endoplasmic reticulum-specific O-glycosylation (Fig. 7A). A cell line derived from a patient with synovial sarcoma, the SW982, was used to generate a stable transfectant (transfected cell line) with ER-2Lec under the control of a doxycycline-inducible promoter system. Next, the SW982 cells were stimulated with CYTO (TNFα and IL1beta cytokines) to stimulate extracellular matrix degradation. An increase in extracellular matrix degradation (Fig. 7B) was observed. However, when ER-2Lec expression was induced, the extracellular matrix degradation was reduced by more than 2-fold (Fig. 7B, 7C).

### ER-2Lec expression in synovial fibroblasts in vivo reduces arthritis

To test whether the ER-2Lec protein could reduce arthritis *in vivo,* a transgenic mouse with ER-2Lec with a Lox cassette was generated. Next, these mice were crossed with mice expressing Cre under the collagen VI alpha1 (Col6a1) promoter (Fig. 8A). Collagen VI is expressed by joint mesenchymal cells, with fibroblasts being the most abundant cell type. This genetic cross results in ER-2Lec being expressed mostly in synovial fibroblasts (Col6a1Cre ER-2Lec). Indeed, it was observed that in the joints, ER-2Lec was mainly expressed in synovial fibroblasts (Fig. 8B).

Next, the Col6a1Cre ER-2Lec animals with were challenged with Collagen Antibody Induced Arthritis (CAIA). In these animals, while there was still formation of a pannus invading the joint cavity, the joint cells displayed a reduction of Tn levels, indicating GALA inhibition (Fig. 8B).

Rheumatoid arthritis symptoms were monitored following Collagen Antibody Induced Arthritis (CAIA) in animals expressing Cre only or also ER-2Lec. The animals expressing ER-2Lec in synovial fibroblasts (Col6a1Cre ER-2Lec) displayed a reduction of swelling in the paws (Fig. 9A). The change in paw thickness between the first day and the seventh day was measured, and an increase in control animals was observed, with some variation between paws (Fig. 9B). By contrast, the ER-2Lec animals displayed almost no swelling. The internationally defined arthritis score was used in a blinded evaluation and a consistent reduction of symptoms was observed in Col6a1Cre ER-2Lec animals (Fig. 10).

Next, histological analysis was performed on day 7 using Alcian blue (AB) and safranin-O (SO) stains, which are commonly used to reveal the cartilage fraction of joints. Formation of a pannus was observed in both groups of animals after Collagen Antibody Induced Arthritis (CAIA) induction, and no significant difference in pannus size (Fig. 11A, 11B). It is of note, and by contrast, there were clear differences in the appearance of the cartilage. For instance, the Alcian blue region was strongly reduced in CAIA Col6a1Cre animals as compared to untreated animals, whereas it appeared preserved for the most part in the Col6a1Cre ER-2Lec animals. The ratio of Alcian blue positive area per mm² of cartilage was measured, and cartilage preservation was observed in the Col6a1Cre ER-2Lec animals treated for CAIA (Fig. 11C). Similar data was obtained after quantification of safranin-O (SO) stains (Fig. 11D).

Thus, in one example, the expression of the ER-2Lec construct in synovial fibroblasts, resulting in GALA-inhibitory effects, is described.

### GALA activates Calnexin glycosylation and surface exposure in synovial fibroblasts

Recently, Calnexin (CNX) had been described as a glycosylation target and effector of GALA. Upon glycosylation, Calnexin is translocated at the cell surface and, in conjunction with Protein disulfide-isomerase A3 (PDIA3), mediates the cleavage of disulfide bonds in extracellular matrix proteins. This reductive activity is essential for matrix degradation by cancer cells. In SW982 synovial fibroblasts, Calnexin (CNX) was found to be hyper-glycosylated by roughly 6-fold after stimulation with cytokines and extracellular matrix (Fig. 12A, 12B). This glycosylation was shown to be GALA-dependent, as expression of ER-2Lec was able to reduce Calnexin glycosylation (Fig. 12A, 12B).

In addition, it was found using fluorescence-activated cell sorting (FACS) that Calnexin surface expression increased after stimulation of these cells with CYTO (TNFα and IL-1β cytokines) and extracellular matrix (Fig. 13A, 13B). Cell surface expression of Calnexin was shown to tightly correlate with GALA, as expression of ER-2Lec reduced the expression of Calnexin (Fig. 13A, 13B).

Thus, in one example, the Calnexin inhibitor is ER-2Lec. In another example, ER-2Lec is used in gene therapy. In yet another example, an expression vector is used to express ER-2Lec. In other words, ER-2Lec is expressed using an expression vector. In another example, ER-2Lec can be transfected into the cell using chemical transfection, viral transfection, lentiviral transduction or cloning. Viral vectors that can be used in such applications are, but are not limited to retroviruses, lentiviruses, adeno-associated viruses (AAV) and adenoviruses. In on example, the vector is an adeno-associated virus vector.

Next, it was sought to confirm these results in primary cells obtained from patients. In healthy synovial fibroblasts, stimulation with cytokines (in this case, TNFα and IL-1β cytokines) and extracellular matrix induced an increase in the proportion of cell surface Calnexin-positive cells (Fig. 14A). Synovial fibroblast cells from a patient suffering from rheumatoid arthritis (RASF) or osteoarthritis (OASF) displayed a 3-fold increase in cell surface levels of Calnexin (Fig. 14B). By contrast, synovial fibroblasts derived from a healthy donor displayed very limited induction of cell surface Calnexin.

Overall, these results indicate that Calnexin glycosylation and its cell surface exposure is dependent on GALA and indicate that Calnexin is involved in cartilage extracellular matrix degradation.

Thus, described herein is the inhibition of Calnexin (CNX) and/or Protein disulfide-isomerase A3 (PDIA3; also known as ERp57), and/or Protein disulfide-isomerase A4 (PDIA4, also known as ERp72) using an inhibitor of Calnexin (CNX) and/or Protein disulfide-isomerase A3 (PDIA3) and/or Protein disulfide-isomerase A4 (PDIA4). It is further noted that PDIA4 is a paralogue of, with similar function to, PDIA3.

As used herein, the term "inhibitor of Calnexin (CNX) and/or Protein disulfide-isomerase A3 (PDIA3) and/or Protein disulfide-isomerase A4 (PDIA4)" refers to any agent or compound capable of inhibiting the expression and/or a function of CNX, or Protein disulfide-isomerase A3 (PDIA3) or Protein disulfide-isomerase A4 (PDIA4) or a complex comprising Calnexin (CNX) and/or Protein disulfide-isomerase A3 (PDIA3). Agents or compounds capable of inhibiting PDIA4, or Calnexin (CNX) and/or PDIA3 are thus referred to as "Protein disulfide-isomerase A4 (PDIA4) inhibitors" or "Calnexin (CNX)/Protein disulfide-isomerase A3 (PDIA3) inhibitors", or "antagonists of Protein disulfide-isomerase A4 (PDIA4), or Calnexin (CNX) and/or Protein disulfide-isomerase A3 (PDIA3)".

In some examples, a Protein disulfide-isomerase A4 (PDIA4), or Calnexin (CNX) and/or Protein disulfide-isomerase A3 (PDIA3) inhibitor is an inhibitor of Calnexin (CNX). In some examples, a Protein disulfide-isomerase A4 (PDIA4), or Calnexin (CNX) and/or Protein disulfide-isomerase A3 (PDIA3) inhibitor is an inhibitor of Protein disulfide-isomerase A3 (PDIA3). In some examples, a Calnexin (CNX) and/or Protein disulfide-isomerase A3 (PDIA3) inhibitor, or Protein disulfide-isomerase A4 (PDIA4) inhibitor is an inhibitor of a complex comprising Calnexin (CNX) and Protein disulfide-isomerase A3 (PDIA3). In some examples, a Protein disulfide-isomerase A4 (PDIA4), or Calnexin (CNX) and/or Protein disulfide-isomerase A3 (PDIA3) inhibitor is an inhibitor of Protein disulfide-isomerase A4 (PDIA4).

In some examples, an inhibitor of Protein disulfide-isomerase A4 (PDIA4), or Calnexin (CNX) and/or Protein disulfide-isomerase A3 (PDIA3) comprises one or more of the following effects: reducing the level of expression (e.g. gene and/or protein expression) of Calnexin (CNX), Protein disulfide-isomerase A3 (PDIA3), Protein disulfide-isomerase A4 (PDIA4), and/or a complex comprising Calnexin (CNX) and Protein disulfide-isomerase A3 (PDIA3); reducing the level of RNA encoding Calnexin (CNX) and/or PDIA3 and/or PDIA4; reducing/inhibiting transcription of nucleic acid encoding Calnexin (CNX) and/or PDIA3 and/or PDIA4; increasing/promoting degradation of RNA encoding Calnexin (CNX) and/or PDIA3 and/or PDIA4; reducing/inhibiting post-transcriptional processing (e.g. splicing, translation, post-translational processing) of RNA encoding Calnexin (CNX) and/or PDIA3 and/or PDIA4; reducing the level of Calnexin (CNX) protein, PDIA4 protein, PDIA3 protein, and/or a protein complex comprising Calnexin (CNX) and PDIA3; increasing/promoting degradation of Calnexin (CNX) protein, PDIA4 protein, PDIA3 protein, and/or a protein complex comprising Calnexin (CNX) and PDIA3; reducing/inhibiting the interaction between (i) Calnexin (CNX), PDIA4, PDIA3, and/or a complex comprising Calnexin (CNX) and PDIA3, and (ii) an interaction partner for Calnexin (CNX), PDIA4, PDIA3, and/or a complex comprising Calnexin (CNX) and PDIA3 (including, for example, reducing/inhibiting interaction between Calnexin (CNX) and PDIA3); reducing the level of a function of Calnexin (CNX), PDIA4, PDIA3, and/or a complex comprising Calnexin (CNX) and PDIA3; reducing/inhibiting extracellular matrix (ECM) degradation by Calnexin (CNX), PDIA4, PDIA3, and/or a complex comprising Calnexin (CNX) and PDIA3; reducing/inhibiting oxidoreductase activity of Calnexin (CNX), PDIA4, PDIA3, and/or a complex comprising Calnexin (CNX) and PDIA3; and/or reducing/inhibiting disulfide bond reductase activity of Calnexin (CNX), PDIA4, PDIA3, and/or a complex comprising Calnexin (CNX) and PDIA3.

A given agent or compound can be evaluated for the properties recited in the preceding paragraph using suitable assays. The assays include, but are not limited to, *in vitro* assays, optionally cell-based assays or cell-free assays. Where the assays are cell-based assays, they can comprise treating cells to upregulate expression and/or activity of Calnexin (CNX), Protein disulfide-isomerase A4 (PDIA4), Protein disulfide-isomerase A3 (PDIA3), and/or a complex comprising Calnexin (CNX) and PDIA3, and treating cells with the test agent or compound in order to determine whether the agent or compound displays one or more of the recited properties.

Gene expression can be analysed by means well known to the skilled person. The level of RNA encoding a given gene can be determined by techniques such as, but not limited to, RT-qPCR, qPCR and the like. Protein expression can also be determined by other means well known to the skilled person, such as chromatography, and Western blots. The level of a given protein/isoform thereof can be determined using, for example, but not limited to, antibody-based methods including, but not limited to, Western blots, immunohistology, immunostaining, immunohistochemistry, cytochemistry, flow cytometry, ELISA, and the like.

Inhibition of gene or protein expression of a given gene can be to less than 1 times, specifically, less or equal to 0.99 times (≤0.99 times), ≤0.95 times, ≤0.9 times, ≤0.85 times, ≤0.8 times, ≤0.75 times, ≤0.7 times, ≤0.65 times, ≤0.6 times, ≤0.55 times, ≤0.5 times, ≤0.45 times, ≤0.4 times, ≤0.35 times, ≤0.3 times, ≤0.25 times, ≤0.2 times, ≤0.15 times, ≤0.1 times, ≤0.05 times, or ≤0.01 times the level of expression observed in the uninhibited state (for example, in the absence of the Protein disulfide-isomerase A4 (PDIA4), or Calnexin (CNX) and/or Protein disulfide-isomerase A3 (PDIA3) inhibitor). In some examples, an agent or compound capable of reducing/inhibiting gene or protein expression inhibits said expression by greater than 5%. In another example, the gene expression is inhibited by more than or equal to 10% (≥10%), ≥15%, ≥20%, ≥25%, ≥30%, ≥35%, ≥40%, ≥45%, ≥50%, ≥55%, ≥60%, ≥65%, ≥70%, ≥75%, ≥80%, ≥85%, ≥90%, ≥91%, ≥92%, ≥93%, ≥94%, ≥95%, ≥96%, ≥97%, ≥98%, ≥99% or 100% of the expression observed in the uninhibited state. Such an inhibition can also be provided using the term "fold change", whereby it is defined as the ratio between the two quantities; for quantities X and Y, then the fold change of Y with respect to X is Y/X. In other words, a change from 30 to 60 is defined as a fold-change of 2. This is also referred to as a "2-fold increase". Similarly, a change from 30 to 15 is referred to as a "2-fold decrease".

Agents or compounds capable of reducing/inhibiting gene expression of Protein disulfide-isomerase A4 (PDIA4), or Calnexin (CNX) and/or Protein disulfide-isomerase A3 (PDIA3) (that is to say, capable of reducing the level of RNA encoding Protein disulfide-isomerase A4 (PDIA4), or Calnexin (CNX) and/or Protein disulfide-isomerase A3 (PDIA3), reducing/inhibiting transcription of nucleic acid encoding Protein disulfide-isomerase A4 (PDIA4), or Calnexin (CNX) and/or PDIA3, and/or increasing/promoting degradation of RNA encoding Protein disulfide-isomerase A4 (PDIA4), or Calnexin (CNX) and/or PDIA3) can be identified using assays comprising detecting the level of RNA encoding Protein disulfide-isomerase A4 (PDIA4), or Calnexin (CNX) and/or PDIA3, for example by RT-qPCR. Such assays can comprise treating cells/tissue with the agent, and subsequently comparing the level of RNA encoding Protein disulfide-isomerase A4 (PDIA4), or Calnexin (CNX) and/or PDIA3 in cells/tissue to the level of RNA encoding Protein disulfide-isomerase A4 (PDIA4), or Calnexin (CNX) and/or PDIA3 in cells/tissue under appropriate control conditions (for example, but not limited to, untreated/vehicle-treated cells/tissue, cells treated with a mock, or an appropriate negative control).

Agents or compounds capable of reducing/inhibiting protein expression of Protein disulfide-isomerase A4 (PDIA4), or Calnexin (CNX) and/or Protein disulfide-isomerase A3 (PDIA3) as described herein can be identified using assays comprising detecting the level of the CNX protein, the PDIA3 protein, the PDIA4 protein, or the level of a protein complex comprising CNX and PDIA3, for example, using antibody/reporter-based methods (Western blot, ELISA, immunohistochemistry, immunohistology, cytochemistry, and the like). Such assays can comprise treating cells/tissue with the agent, and subsequently comparing the level of the protein/protein complex in such cells/tissue to the level in cells/tissue of an appropriate control condition.

Agents or compound capable of reducing/inhibiting interaction between (i) Calnexin (CNX), PDIA4, PDIA3, and/or a complex comprising Calnexin (CNX) and PDIA3 and (ii) an interaction partner for Calnexin (CNX), PDIA4, PDIA3, and/or a complex comprising Calnexin (CNX) and PDIA3 (e.g. interaction between Calnexin (CNX) and PDIA3) can be identified, for example, using assays comprising detecting the level of interaction, e.g. using antibody/reporter-based methods. The level of interaction between (i) Calnexin (CNX), Protein disulfide-isomerase A4 (PDIA4), Protein disulfide-isomerase A3 (PDIA3) and/or a complex comprising Calnexin (CNX) and PDIA3, and (ii) an interaction partner for Calnexin (CNX), PDIA4, PDIA3 and/or a complex comprising CNX and PDIA3 can be analysed using methods such as, but not limited to, resonance energy transfer techniques (e.g. FRET, BRET), or methods analysing a correlate of the interaction. Assays can comprise treating cells/tissue with the agent, and subsequently comparing the level of interaction between (i) Calnexin (CNX), PDIA4, PDIA3, and/or a complex comprising Calnexin (CNX) and PDIA3, and (ii) an interaction partner for Calnexin (CNX), PDIA4, PDIA3, and/or a complex comprising Calnexin (CNX) and PDIA3 in such cells/tissue to the level of interaction observed in cells/tissue of an appropriate control condition (e.g. untreated/vehicle-treated cells/tissue). The level of interaction between (i) Calnexin (CNX), PDIA4, PDIA3, and/or a complex comprising Calnexin (CNX) and PDIA3 and (ii) an interaction partner for Calnexin (CNX), PDIA4, PDIA3, and/or a complex comprising Calnexin (CNX) and PDIA3 (for example, the interaction between Calnexin (CNX) and PDIA3) can also be analysed using techniques such as, but not limited to, enzyme-linked immunosorbent assays (ELISAs), surface plasmon resonance or biolayer interferometry analysis. Assays can comprise comparing the level of interaction in the presence of the agent to the level of interaction in an appropriate control condition, for example in untreated samples or samples in the absence of the agent.

Inhibition of interaction between (i) Calnexin (CNX), Protein disulfide-isomerase A4 (PDIA4), Protein disulfide-isomerase A3 (PDIA3), and/or a complex comprising Calnexin (CNX) and Protein disulfide-isomerase A3 (PDIA3) and (ii) an interaction partner for Calnexin (CNX), Protein disulfide-isomerase A4 (PDIA4), Protein disulfide-isomerase A3 (PDIA3), and/or a complex comprising Calnexin (CNX) and PDIA3 (for example, the interaction between Calnexin (CNX) and PDIA3) can be to less than 1 times, for example, ≤0.99 times, ≤0.95 times, ≤0.9 times, ≤0.85 times, ≤0.8 times, ≤0.75 times, ≤0.7 times, ≤0.65 times, ≤0.6 times, ≤0.55 times, ≤0.5 times, ≤0.45 times, ≤0.4 times, ≤0.35 times, ≤0.3 times, ≤0.25 times, ≤0.2 times, ≤0.15 times, ≤0.1 times, ≤0.05 times, or ≤0.01 times the level of interaction observed in the uninhibited state. In some examples, an agent or compound capable of reducing/inhibiting the interaction between (i) PDIA4, Calnexin (CNX), PDIA3, and/or a complex comprising Calnexin (CNX) and PDIA3 and (ii) an interaction partner for PDIA4, Calnexin (CNX), PDIA3, and/or a complex comprising Calnexin (CNX) and PDIA3 (e.g. interaction between Calnexin (CNX) and PDIA3) inhibits greater than 5%, *e.g.* one of ≥10%, ≥15%, ≥20%, ≥25%, ≥30%, ≥35%, ≥40%, ≥45%, ≥50%, ≥55%, ≥60%, ≥65%, ≥70%, ≥75%, ≥80%, ≥85%, ≥90%, ≥91%, ≥92%, ≥93%, ≥94%, ≥95%, ≥96%, ≥97%, ≥98%, ≥99% or 100% of the interaction observed in the uninhibited state.

Agents or compounds capable of reducing/inhibiting a function of Protein disulfide-isomerase A4 (PDIA4), Calnexin (CNX), Protein disulfide-isomerase A3 (PDIA3), and/or a complex comprising Calnexin (CNX), PDIA3 can be identified using assays for the relevant function. Such assays can comprise treating cells/tissue expressing PDIA4, CNX, PDIA3 and/or a complex comprising CNX and PDIA3 with an agent, and subsequently comparing the level of the relevant function to the level observed in an appropriate control condition, for example untreated/vehicle-treated cells/tissue samples, a negative control, or cell/tissue samples treated with ineffective compounds or compounds without the required effect.

Agents or compounds capable of reducing/inhibiting a function of Protein disulfide-isomerase A4 (PDIA4), Calnexin (CNX), Protein disulfide-isomerase A3 (PDIA3), and/or a complex comprising Calnexin (CNX) and PDIA3 can be identified using assays comprising detecting the level of a correlate of a function of Calnexin (CNX), Protein disulfide-isomerase A4 (PDIA4), Protein disulfide-isomerase A3 (PDIA3), and/or a complex comprising CNX and PDIA3, for example, gene and/or protein expression, and/or activity, of one or more proteins whose expression is directly/indirectly upregulated or downregulated as a consequence of a function of CNX, PDIA4, PDIA3 and/or a complex comprising CNX and PDIA3. Such assays can comprise treating cells/tissue expressing CNX, PDIA4, PDIA3, and/or a complex comprising CNX and PDIA3 with the agent or compound, and subsequently comparing the level of the correlate of a function of CNX, PDIA4, PDIA3, and/or a complex comprising CNX and PDIA3 in such cells/tissue to the level of the correlate of the relevant function in an appropriate control condition as disclosed herein.

A function of Calnexin (CNX), Protein disulfide-isomerase A4 (PDIA4), Protein disulfide-isomerase A3 (PDIA3), and/or a complex comprising CNX and PDIA3 can be, but is not limited to, extracellular matrix (ECM) degradation, oxidoreductase activity or disulphide bond reductase activity. A correlate of a function of PDIA4, CNX, PDIA3 and/or a complex comprising CNX and PDIA3 can be, but is not limited to, a product of extracellular matrix (ECM) degradation, oxidoreductase activity or disulphide bond reductase activity.

Agents capable of reducing/inhibiting extracellular matrix (ECM) degradation by Calnexin (CNX), Protein disulfide-isomerase A4 (PDIA4), Protein disulfide-isomerase A3 (PDIA3), and/or a complex comprising CNX and PDIA3 can be identified using assays comprising detecting the level of extracellular matrix (ECM), or extracellular matrix (ECM) degradation activity, for example, but not limited to, using antibody/reporter-based methods. Extracellular matrix (ECM) degradation can be measured using methods known in the art available to the skilled person. Assays can comprise treating cells/tissue expressing CNX, PDIA4, PDIA3, and/or a complex comprising CNX and PDIA3 with the agent, and subsequently comparing the level of extracellular matrix (ECM) degradation to the level of extracellular matrix (ECM) degradation observed in an appropriate control condition.

Agents or compounds capable of reducing/inhibiting oxidoreductase activity and/or disulphide bond reductase activity of Calnexin (CNX), Protein disulfide-isomerase A4 (PDIA4), Protein disulfide-isomerase A3 (PDIA3), and/or a complex comprising CNX and PDIA3 can be identified using assays comprising detecting the level of the relevant activity. For example, oxidoreductase activity and/or disulphide bond reductase activity can be measured using, for example, an insulin reduction assay as known in the art. Assays can comprise comparing the level of the relevant activity displayed by CNX, PDIA4, PDIA3, and/or a complex comprising CNX and PDIA3 in the presence of the agent to the level observed in the absence of the agent.

Inhibition of a function of Calnexin (CNX), Protein disulfide-isomerase A4 (PDIA4), Protein disulfide-isomerase A3 (PDIA3), and/or a complex comprising CNX and PDIA3 (for example, but not limited to, extracellular matrix (ECM) degradation, oxidoreductase activity, disulphide bond reductase activity) can be less than 1 times, for example, less than or equal to 0.99 times (≤0.99 times), ≤0.95 times, ≤0.9 times, ≤0.85 times, ≤0.8 times, ≤0.75 times, ≤0.7 times, ≤0.65 times, ≤0.6 times, ≤0.55 times, ≤0.5 times, ≤0.45 times, ≤0.4 times, ≤0.35 times, ≤0.3 times, ≤0.25 times, ≤0.2 times, ≤0.15 times, ≤0.1 times, ≤0.05 times, or ≤0.01 times the level of activity observed in the uninhibited state. In some examples, an agent or compound capable of reducing/inhibiting a function of CNX, PDIA4, PDIA3 and/or a complex comprising CNX and PDIA3 (for example, extracellular matrix (ECM) degradation, oxidoreductase activity, disulphide bond reductase activity) inhibits greater than 5%, or less or equal to 10% (≥10%), ≥15%, ≥20%, ≥25%, ≥30%, ≥35%, ≥40%, ≥45%, ≥50%, ≥55%, ≥60%, ≥65%, ≥70%, ≥75%, ≥80%, ≥85%, ≥90%, ≥91%, ≥92%, ≥93%, ≥94%, ≥95%, ≥96%, ≥97%, ≥98%, ≥99% or 100% of the relevant activity observed in the uninhibited state.

Protein disulfide-isomerase A4 (PDIA4), or CNX and/or Protein disulfide-isomerase A3 (PDIA3) inhibitors according to the present disclosure can be any kind of agent or type of compound possessing the appropriate inhibitory activity.

In some examples, a Protein disulfide-isomerase A4 (PDIA4), or CNX and/or Protein disulfide-isomerase A3 (PDIA3) inhibitor is selected from: a PDIA4-binding molecule, or a CNX-binding molecule, a PDIA3-binding molecule, a CNX:PDIA3 complex-binding molecule, a molecule capable of reducing the level of CNX, a molecule capable of reducing the level of PDIA3, a molecule capable of reducing the level of PDIA3, and a molecule capable of reducing the level of a CNX:PDIA3 complex.

As used herein, the term "CNX -binding molecule" refers to a molecule capable of binding to CNX. In the same line, a "PDIA3-binding molecule" or "PDIA4-binding molecule" refers to a molecule capable of binding to Protein disulfide-isomerase A3 (PDIA3) or Protein disulfide-isomerase A4 (PDIA4). A "CNX:PDIA3 complex-binding molecule" refers to a molecule which is capable of binding to a complex comprising CNX and PDIA3.

Such binding molecules can be identified using any suitable assay for detecting binding of a molecule to the relevant factor, in the context of the present disclosure, CNX, PDIA4, PDIA3, or a complex comprising CNX and PDIA3. Such assays can comprise detecting the formation of a complex between the relevant factor and the molecule.

CNX -binding molecules, Protein disulfide-isomerase A4 (PDIA4)-binding molecules, Protein disulfide-isomerase A3 (PDIA3)-binding molecules and CNX:PDIA3-binding molecules can be analysed in appropriate assays in order to identify antagonists of CNX or PDIA3. For example, molecules which bind specifically to CNX, PDIA4, PDIA3 and/or complexes comprising CNX and PDIA3 can be evaluated for their ability to inhibit extracellular matrix (ECM) degradation, or for their ability to inhibit oxidoreductase and/or disulphide bond reductase activity.

A CNX-binding, Protein disulfide-isomerase A4 (PDIA4)-binding, Protein disulfide-isomerase A3 (PDIA3)-binding or CNX:PDIA3 complex-binding molecule can inhibit the ability of its target (i.e. CNX, PDIA3, CNX:PDIA3complex) to interact with an interaction partner. In some examples, a PDIA3-binding, a CNX-binding, PDIA3-binding or CNX:PDIA3 complex-binding molecule behaves as a competitive inhibitor of interaction between its target and an interaction partner for its target. The binding molecule can occupy, or otherwise reduce access to or block, a region of its target required for binding to an interaction partner for its target. The ability of a CNX-binding, PDIA4-binding, PDIA3-binding or CNX:PDIA3 complex-binding molecule to inhibit interaction between its target and an interaction partner for its target can be evaluated by, for example, but not limited to, analysis of interaction in the presence of, or following incubation of one or both of the interaction partners with, the relevant binding molecule. An example of a suitable assay to determine whether a given binding agent is capable of inhibiting interaction between CNX, PDIA4, PDIA3 or CNX:PDIA3 complex, and an interaction partner is a competition enzyme-linked immunosorbent assay (ELISA).

Calnexin (CNX)-binding molecules, Protein disulfide-isomerase A4 (PDIA4)-binding molecules, Protein disulfide-isomerase A3 (PDIA3)-binding molecules, and CNX:PDIA3 complex-binding molecules include antigen-binding molecules.

Antigen-binding molecules capable of binding to Calnexin (CNX), Protein disulfide-isomerase A4 (PDIA4)-binding molecules, Protein disulfide-isomerase A3 (PDIA3), or a complex comprising CNX and PDIA3 recognise a region of the relevant target protein/protein complex which is accessible to an antigen-binding molecule when the protein/protein complex is expressed at the cell surface. This is the case, for example, when the protein/protein complex is exposed in, or at, the cell membrane of a cell expressing the protein/protein complex, and allows binding of the same. In some examples, an antigen binding molecule binds to a region of CNX, PDIA4, PDIA3, or a complex comprising CNX and PDIA3 which is extracellular when the protein/protein complex is expressed at the cell surface.

As used herein, the term "antigen-binding molecule" refers to a molecule capable of binding to a given target antigen, and includes in its scope antibodies (immunoglobulins) such as, but not limited to, monoclonal antibodies, polyclonal antibodies, monospecific antibodies, multispecific antibodies (for example, but not limited to, bispecific or trispecific antibodies), and fragments and derivatives thereof (for example, but not limited to Fv, scFv, Fab, scFab, F(ab')₂, Fab₂, diabodies, triabodies, scFv-Fc, minibodies, and single domain antibodies (such as, but not limited to, VhH)).

Antigen-binding molecules can specifically bind to the relevant targets disclosed herein, which are, but are not limited to, Calnexin (CNX), Protein disulfide-isomerase A4 (PDIA4), Protein disulfide-isomerase A3 (PDIA3), or a complex comprising CNX and PDIA3. As used herein, the term "specific binding" refers to binding which is selective, and which can be discriminated from non-specific binding to non-target molecules. An antigen-binding molecule that binds specifically to Calnexin (CNX), PDIA4, PDIA3, or a complex comprising CNX and PDIA3. Preferably, the antigen-binding molecules bind to the relevant targets with greater affinity, and/or with greater duration than it binds to other, non-target molecules.

An antigen-binding molecule can be, or can comprise, an antigen-binding peptide/polypeptide, or an antigen-binding peptide/polypeptide complex. An antigen-binding molecule can be a non-covalent or covalent complex of more than one polypeptide (for example, a complex comprising 2, 3, 4, 6, or 8 polypeptides). In one example, such an antigen-binding molecule is an IgG-like antigen-binding molecule comprising two heavy chain polypeptides and two light chain polypeptides.

Antibodies generally comprise six complementarity-determining regions (CDRs); three in the light chain variable region (VL), usually denoted as LC-CDR1, LC-CDR2, LC-CDR3, and three in the heavy chain variable region (VH), usually denoted as HC-CDR1, HC-CDR2 and HC-CDR3. The six CDRs together define the paratope of the antibody, which is the part of the antibody which binds to the target molecule. The VH region and VL region comprise framework regions (FRs) to either side of each CDR, which provide a scaffold for the complementarity-determining regions (CDRs). As per convention, from N-terminus to C-terminus, heavy chain variable regions (VH) comprise the following structure: N term-[HC-FR1]-[HC-CDR1]-[HC-FR2]-[HC-CDR2]-[HC-FR3]-[HC-CDR3]-[HC-FR4]-C term; and VL regions comprise the following structure: N term-[LC-FR1]-[LC-CDR1]-[LC-FR2]-[LC-CDR2]-[LC-FR3]-[LC-CDR3]-[LC-FR4]-C term.

The present disclosure refers to antibody or antibody antagonists of Calnexin (CNX), Protein disulfide-isomerase A4 (PDIA4), Protein disulfide-isomerase A3 (PDIA3), and/or a complex comprising CNX and PDIA3 which are, but are not limited to antibody antagonists of CNX, PDIA4, PDIA3, and/or a complex comprising CNX and PDIA3. In one example, an antigen-binding molecule according to the present disclosure comprises the complementarity-determining regions (CDRs) and/or the heavy chain variable region (VH) and light chain variable region (VL) of an antibody which binds specifically to the target antigen (for example, CNX, PDIA4, PDIA3, or a complex comprising CNX and PDIA3).

Antibodies to a given target antigen can be made, derived or engineered using techniques known in the art. Such techniques include, but are not limited to, screening antibody gene-phage display libraries for molecules capable of binding to the target antigen, and raising antibodies to a given target antigen by animal immunisation. Approaches to the production of monoclonal antibodies (monoclonal or otherwise) suitable for therapeutic use in humans, or for use according to the methods disclosed herein, include, but are not limited to, using hybridoma technology, raising xenogenic antibodies and subsequent humanisation, human antibody gene-phage display, and production in transgenic mice having human antibody genes.

Phage display techniques can also be employed to identify antibodies capable of binding to a given target, target protein, or protein complex, and are well known to the skilled person.

Antibodies to Calnexin (CNX) include, but are not limited to, monoclonal antibody clone AF18 (Invitrogen Cat. No. MA3-027), clone AF8 (Merck Cat. No. MABF2067), clone TO-5 (Merck Cat. No. C7617), clone 3H4A7 (Invitrogen Cat. No. MA5-15389), clone ARC0648 (Invitrogen Cat. No. MA5-35588), clone GT1563 (GeneTex Cat. No. GTX629976), clone CANX/1541 (GeneTex Cat. No. GTX34446), clone IE2.1C12 (Novus Biologicals Cat No. NBP2-36571), clone 1C2.2D11 (Novus Biologicals Cat No. NBP2-36570SS), clone 2A2C6 (Proteintech Cat. No. 66903-1-Ig), clone C5C9 (Cell Signaling Technology, Inc Cat. No. 2679), clone E-10 (Santa Cruz Biotechnology Cat No. sc-46669), polyclonal antibodies ab10286 and ab22595 (Abcam), and anti-Cnx antibodies disclosed in CN 101659702 A (for example, the antibody produced by hybridoma CGMCC No. 3240). In one example, the antibody to Calnexin (CNX) is ab22595 (Abcam), a rabbit polyclonal antibody raised against human CNX for an epitope beginning at 550 amino acids to the C-terminus.

Antibodies to Protein disulfide-isomerase A3 (PDIA3) include, but are not limited to, monoclonal antibody clone MaP.Erp57 (Enzo Life Sciences), clone CL2444 (Sigma-Aldrich Cat. No. AMAB90988), clone OTI3E1 (ThermoFisher Scientific Cat. No. TA504995), clone OTI3D2 (ThermoFisher Scientific Cat. No. TA504990), clone OTI4D7 (ThermoFisher Scientific Cat. No. TA505008) and polyclonal antibodies ab13506 and ab13507 (Abcam). In another example, the anti-PDIA3 antibody is ab13507 (Abcam).

Antibodies to a given target, target protein or protein complex raised in a non-human animal (for example, but not limited to, mouse, rabbit, horse, dog, donkey and so on), can be engineered in order to improve their suitability for therapeutic use in humans, also known as humanisation or a humanised antibody. In other words, humanised antibodies are antibodies whose protein sequences have been modified to increase their similarity to antibody variants produced naturally in humans. For example, one or more amino acids of monoclonal antibodies raised by animal immunisation can be substituted to arrive at an antibody sequence which is more similar to human germline immunoglobulin sequences, thereby reducing the potential for an (anti-xenogenic) antibody immune responses in the human subject treated with the antibody. Modifications in the antibody variable domains can focus on the framework regions in order to preserve the antibody paratope. The requirement for humanisation can be circumvented by, for example, raising antibodies to a given target protein/protein complex in transgenic model species expressing human immunoglobulin genes, such that the antibodies raised in such animals are fully-human.

Antigen-binding molecules also include, but are not limited to, peptide aptamers, thioredoxins, monobodies, anticalin, Kunitz domains, avimers, knottins, fynomers, atrimers, DARPins, affibodies, nanobodies (such as, but not limited to, single-domain antibodies (sdAbs)) affilins, armadillo repeat proteins (ArmRPs) and OBodies. Such antigen-binding peptides can be identified using methods known in the art, for example, by screening of libraries of the relevant peptides.

Antigen-binding peptides also include, but are not limited to, decoy interaction partners for the relevant target antigen. For example, a decoy interaction partner for Calnexin (CNX) can comprise or consist of a CNX-binding fragment of Protein disulfide-isomerase A3 (PDIA3) or a variant thereof, and conversely, a decoy interaction partner for PDIA3 can comprise or consist of a PDIA3-binding fragment of CNX or a variant thereof. The decoy interaction partner can be truncated, or otherwise modified relative to the peptide on which it is based, such that decoy interaction partner associates with its target to form a non-functional complex and/or a complex having reduced level of a functional property possessed by the complex formed by the target and the interaction partner upon which the decoy interaction partner is based. Thus, in one example, decoy interaction partners behave as competitive inhibitors of interaction between the target and their interaction partner upon which they are based.

Calnexin (CNX)-binding molecules, Protein disulfide-isomerase A4 (PDIA4)-binding molecules, Protein disulfide-isomerase A3 (PDIA3)-binding molecules and CNX:PDIA3 complex-binding molecules include, but are not limited to, small molecules, which can be identified by, for example, screening small molecule libraries. As used herein, the term "small molecule" refers to a low molecular weight. Such a low molecular weight is usually less than 1000 daltons (< 1000 daltons (Da)). Typically, a small molecule is between 300 to 700 Da, and is an organic compound.

Examples of small molecule inhibitors of include, but are not limited to, tenecteplase (for example, for Calnexin (CNX)) and p-hydroxymercuribenzoic acid sodium salt (for example, for PDIA3).

Examples of Calnexin (CNX)-binding molecules, Protein disulfide-isomerase A3 (PDIA3)-binding molecules and CNX:PDIA3 complex-binding molecules include aptamers. Nucleic acid aptamers can comprise DNA and/or RNA, and can be single stranded or double stranded. In one example, they can comprise chemically modified nucleic acids, in which the sugar and/or phosphate and/or base is chemically modified. Such modifications can improve the stability of the aptamer or make the aptamer more resistant to degradation. In one example, such modifications include, but are not limited to, modification at the 2' position of ribose. Nucleic acid aptamers can be chemically synthesised, for example, on a solid support. Solid phase synthesis can comprise phosphoramidite chemistry. In on example, a solid supported nucleotide is detritylated, then coupled with a suitably activated nucleoside phosphoramidite to form a phosphite triester linkage. Capping can then occur, followed by oxidation of the phosphite triester with an oxidant, typically iodine. The cycle can then be repeated to assemble the aptamer.

Molecules which are capable of reducing the level of Calnexin (CNX), Protein disulfide-isomerase A4 (PDIA4), Protein disulfide-isomerase A3 (PDIA3), and CNX:PDIA3 complex include, but are not limited to, molecules capable of reducing gene and/or protein expression of PDIA4, CNX and/or PDIA3. In some embodiments, a molecule capable of reducing the level of CNX, PDIA4, PDIA3 or CNX:PDIA3 complex reduces or prevents the expression of a polypeptide encoded by the genes *PDIA4, CANX,* or *PDIA3,* respectively.

Inhibition of gene or protein expression of a given target gene (for example, but not limited to the gene *CANX, PDIA4,* or *PDIA3*) can comprise, but is not limited to, inhibiting transcription of the gene, inhibiting post-transcriptional processing (for example, splicing) of RNA transcribed from the gene, reducing the stability of RNA transcribed from the gene, promoting degradation of RNA transcribed from the gene, inhibiting translation of RNA transcribed from the gene into protein, inhibiting post-translational processing of a polypeptide encoded by the gene, reducing the stability of a polypeptide encoded by the gene, or promoting degradation of a polypeptide encoded by the gene.

Inhibition of gene or protein expression can be achieved, for example, by altering/disrupting the nucleotide sequence of the gene, or by altering/disrupting nucleotide sequence required for expression of the gene (for example, a regulatory sequence governing expression of the gene). In some examples, inhibiting gene or protein expression comprises altering a nucleotide sequence. Such an alteration or modification can be done, for example, by substitution, deletion or insertion of one or more nucleotides. In particular examples, the present disclosure describes inhibiting gene or protein expression by deletion of all or part of the nucleotide sequence of the relevant gene.

In some examples, altering/disrupting the nucleotide sequence, can comprise altering or removing a regulatory sequence (such as, but not limited to a promoter or an enhancer) for transcription of the gene, introducing a premature stop codon in the sequence transcribed from the gene, altering the nucleotide sequence to encode a truncated and/or non-functional gene product, or altering the nucleotide sequence to encode a gene product which is misfolded and/or degraded. Nucleotides sequences can also be disrupted by, for example, but not limited to, homologous recombination, or by target nucleic acid modification using site-specific nucleases (SSNs).

In some examples, altering/disrupting the nucleotide sequence in order to inhibit or prevent gene or protein expression from a gene can be referred to as 'knocking out' a gene.

Modification by homologous recombination can involve the exchange of nucleic acid sequence through crossover events guided by homologous sequences.

Gene editing is also a method that can be used to edit, alter, or disrupt expression or function of a nucleotide sequence. Enzymes capable of creating site-specific double strand breaks (DSBs) can be engineered to introduce DSBs to target nucleic acid sequences of interest. Double strand breaks can be repaired by either error-prone non-homologous end-joining (NHEJ), in which the two ends of the break are rejoined, often with insertion or deletion of nucleotides. Alternatively, double strand breaks can be repaired by highly homology-directed repair (HDR), in which a DNA template with ends homologous to the break site is supplied and introduced at the site of the DSB. Site-specific nucleases (SSNs) are capable of being engineered to generate target nucleic acid sequence-specific double strand breaks include, but are not limited to, zinc-finger nucleases (ZFNs), transcription activator-like effector nucleases (TALENs) and clustered regularly interspaced palindromic repeats/CRISPR-associated-9 (CRISPR/Cas9) systems.

Zinc-finger nuclease (ZFN) systems comprise a programmable Zinc Finger DNA-binding domain and a DNA-cleaving domain (for example, a *Fok*I endonuclease domain). The DNA-binding domain can be identified by screening a Zinc Finger array capable of binding to the target nucleic acid sequence. In another example, transcription activator-like effector nucleases (TALEN) are restriction enzymes that can be engineered to cut specific sequences of DNA. TALENs comprise a programmable DNA-binding TALE domain and a DNA-cleaving domain (for example, a *Fok*I endonuclease domain). TALEs comprise repeat domains consisting of repeats of between 33 to 39 amino acids, which are identical except for two residues at positions 12 and 13 of each repeat, also termed repeat variable di-residues (RVDs). As known in the art, each RVD determines binding of the repeat to a nucleotide in the target DNA sequence according to the following relationship: "HD" binds to C, "NI" binds to A, "NG" binds to T and "NN" or "NK" binds to G. Further examples of gene editing systems include, but are not limited to, CRISPR/Cas9 and related systems, for example, CRISPR/Cpf1, CRISPR/C2c1, CRISPR/C2c2 and CRISPR/C2c3. These systems comprise an endonuclease (for example, Cas9, Cpf1, and others) and the single-guide RNA (sgRNA) molecule. The sgRNA can be engineered to target endonuclease activity to nucleic acid sequences of interest.

An example of a CRISPR/Cas9 system for targeted knockout of expression of CNX is the system encoded by plasmids Calnexin CRISPR/Cas9 KO Plasmid (h) (Santa Cruz Biotechnology Cat. No. sc-400154) and Calnexin HDR Plasmid (h) (Santa Cruz Biotechnology Cat. No. sc-400154-HDR).

Disruption of a nucleotide sequence using site-specific nucleases (SSNs) can be achieved in an animal, for example, by administering nucleic acid encoding the components of the relevant site-specific nuclease system to the animal. For example, the animal can be administered with one or more vectors comprising nucleic acid encoding the constituents of the site-specific nuclease system for targeting the relevant gene.

Inhibition of gene or protein expression can also be achieved, for example, by treatment with an agent or compound capable of reducing gene or protein expression. For example, inhibition of gene or protein expression can be achieved using an inhibitory nucleic acid, such as, but not limited to, an antisense nucleic acid. Antisense nucleic acids can bind to target nucleic acid by complementary base pairing. Where the target nucleic acid is an RNA (for example, RNA transcribed from the relevant gene), the binding of the antisense nucleic acid to the target RNA can promote degradation of and/or inhibit translation of the RNA. An example of an inhibitory nucleic acid is an antisense oligonucleotide (ASO).

An inhibitory nucleic acid can inhibit gene or protein expression by RNA interference (RNAi). RNAi involves inhibition of gene expression and translation by targeted neutralisation of mRNA molecules. In some examples, the inhibitory nucleic acid is small interfering RNA (siRNA), a short hairpin RNA (shRNA), or a micro RNA (miRNA).

Small interfering RNAs (siRNAs) are derived by processing of long double stranded RNAs and, when found in nature, are typically of exogenous origin. Micro-interfering RNAs (miRNAs) are endogenously encoded small non-coding RNAs. Both siRNA and miRNA can inhibit the translation of mRNAs bearing partially complimentary target sequences without RNA cleavage and degrade mRNAs bearing fully complementary sequences. siRNAs are typically double-stranded and, in order to optimise the effectiveness of RNA mediated inhibition of the function of a target gene, it is preferred that the length of the siRNA molecule is chosen to ensure correct recognition of the siRNA by the RNA-induced silencing complex (RISC), that mediates the recognition by the siRNA of the mRNA target. DNA sequences encoding miRNAs include, but are not limited to, the miRNA sequence and an approximate reverse complement. When this DNA sequence is transcribed into a single-stranded RNA molecule, the miRNA sequence and its reverse-complement base pair to form a partially double stranded RNA segment.

Examples of siRNAs targeting the gene *CANX* include, but are not limited to, siRNA generated based on, or using, the target sequence of 5'-CAAGAGUGGUCCUAGGAGAUU-3' (SEQ ID NO: 2), as an exemplary target for Calnexin.

Examples of siRNAs targeting *PDIA3* include, but are not limited to, siRNA generated based on, or using, any one of the target sequence of GGAAUAGUCCCAUUAGCAA (SEQ ID NO: 3), GGGCAAGGACUUACUUAUU (SEQ ID NO: 4), AGACCCAAAUAUCGUCAUA (SEQ ID NO: 5), AGGAGUUCUCGCGUGAUG (SEQ ID NO: 6), GAACGAGUAUGAUGAUAAU (SEQ ID NO: 7), GGACAAGACUGUGGCAUAU (SEQ ID NO: 8), GGCAAGGACUUACUUAUUG(SEQ ID NO: 9), UGAUAAAGAUGCCUCUAUA (SEQ ID NO: 10), and combinations thereof, as an exemplary target for PDIA3. SEQ ID Nos 3 to 6 refer to (L-003674-00-0005, ON-TARGETplus Human PDIA3 (2923) siRNA • SMARTpool, 5 nmol), while SEQ ID Nos 7 to 10 refer to M-003674-01-0005, siGENOME Human PDIA3 (2923) siRNA • SMARTpool, 5 nmol.

Short hairpin RNAs (shRNAs) are considered to be more stable than synthetic siRNAs. A short hairpin RNA (shRNA) consists of short inverted repeats separated by a small loop sequence. One inverted repeat is complimentary to the gene target. In cells, the shRNA is processed by the endoribonuclease DICER into siRNA, which degrades the target gene mRNA and suppresses its expression. Short hairpin RNAs (shRNAs) can be produced within a cell, for example, by transcription from a vector.

### Anti-Calnexin antibodies prevent arthritic symptoms in Collagen Antibody Induced Arthritis mice

First, the presence of disulfide bonds in cartilage extracellular matrix was tested for using a previously described method. Briefly, cartilage extracellular matrix was reduced with tris(2-carboxyethyl)phosphine (TCEP), then treated with N-ethylmaleimide (NEM), and then treated with the anti-OX133 antibody. As with liver extracellular matrix, an abundance of signal was observed, indicating cartilage extracellular matrix is heavily cross-linked with disulfide bonds (Fig. 15).

Next, the effect of anti-Calnexin antibodies on extracellular matrix degradation was tested *in vitro.* Osteoarthritis synovial fibroblast (OASF) cells were seeded on cartilage extracellular matrix covering fluorescent gelatine, and their extracellular matrix degradation activity was measured as described above. The addition of a polyclonal anti-Calnexin antibody was shown to block this degradative activity (Fig. 16).

Collagen Antibody Induced Arthritis (CAIA) animals were treated with the anti-Calnexin (anti-CNX) antibody, injecting 25 micrograms (or the equivalent of 1.25mg/kg, based on an average mouse model weight of 20 grams per animal) every two days from day 3 till day 7 after initiation of CAIA (Fig. 17A). Paw thickness was monitored at regular intervals and the animals were analysed at day 10 for arthritic score. The paws swelled very little in anti- Calnexin treated animals as compared to control animals treated with an isotype antibody (Fig. 17B, 17C). While some redness and swelling in the fingers still occurred, raising the arthritic score, the mean score of the animals treated with the anti-Calnexin (anti-CNX) antibody was half of CAIA control animals (Fig. 18).

Thus, in one example, the therapeutic agent disclosed herein is an antibody. In another example, the antibody is an anti-Calnexin antibody. In yet another example, the antibody is an anti-PDIA3 antibody.

At the histological level, the reduction in safranin-O (SO) positive cartilage was pronounced in control animals at day 10, to the point of almost disappearing in some areas (Fig. 19A, 19B). In contrast, anti-Calnexin antibody treated animals had an overall preserved cartilage structure (Fig. 19A, 19B). Histology analysis revealed that the synovium in anti-Calnexin antibody treated mice swelled and invaded the joint as in CAIA animals, suggesting the initial part of the activation cascade did occur (Fig. 19A, 19B). However, the pannus did not attack the cartilage and invade it as deeply as in the control animals.

Finally, to verify that the antibody had indeed reached the synovium, it was detected using an anti-rabbit IgG antibody. A signal in cells of the synovium of animals treated with the anti-Calnexin antibody was observed, and no signal was seen in animals treated with a control rabbit IgG, suggesting the anti-CNX antibody was internalised specifically in synovial fibroblasts (Fig. 20).

Overall, these results indicate that inhibiting Calnexin results in an inhibition of cartilage extracellular matrix degradation and can form the basis of an arthritis therapeutics. Thus, in one example, the therapeutic agent disclosed herein is an antibody. In another example, the antibody is an anti-Calnexin antibody.

As shown herein, the activation of the GALA regulatory pathway in synovial fibroblasts of both osteoarthritis and rheumatoid arthritis joints is described. While these diseases are different, they share the key feature of cartilage extracellular matrix degradation. In both cases, the main drivers of cartilage degradation are the fibroblastic cells derived from the synovial membrane.

It remains unclear how synovial fibroblasts switch from a healthy state to an aggressive, matrix-degradative state. In the former, Calnexin form the intimal lining of the synovial membrane and contribute to the composition of the synovial fluid by secreting various factors. In rheumatoid arthritis, Calnexin proliferates and engages in collective migration to invade the joint cavity, degrade the cartilage extracellular matrix, and eventually invade the cartilage itself. In osteoarthritis, proliferation is not so apparent, but synovial fibroblasts are still engaged in matrix degradation. The results disclosed herein indicate that activation of GALA is a control switch in both diseases. Indeed, it is shown that GALA is activated specifically in synovial fibroblasts, and much less prominently in the immune cells found in the inflamed synovial membrane. Consistent with a key role of synovial fibroblasts in the degradation of cartilage, it had been previously reported that GALA controls extracellular matrix degradative ability in cancer cells.

How GALA is activated in these cells is unclear. The described results indicate that cytokines, such as, but not limited to, IL-1β and TNF*α*, which are known to drive the disease, are stimulating GALA in synovial fibroblasts *in vitro.* IL-1β has been reported to activate the tyrosine kinase Src. Src acts at the Golgi as a key regulator of GALA. Contact with extracellular matrix also activated the GALA pathway in synovial fibroblasts, although the mechanism is unclear at present. It possibly involves integrins and signalling through the FAK and, again, Src kinase. Osteoarthritis synovial fibroblasts (OASF) and rheumatoid arthritis synovial fibroblasts (RASF) display comparable global methylation profiles, which are distinct from synovial fibroblasts from healthy subjects. Differences were identified in genes involved in platelet-derived growth factor (PDGF) and epidermal growth factor (EGF) signalling. Both growth factors have been shown previously to activate GALA, suggesting that rheumatoid arthritis synovial fibroblasts (RASF) and osteoarthritis synovial fibroblasts (OASF) can be primed to activate the GALA pathway more readily by epigenetic imprinting.

A feature of N-acetylgalactosaminyltransferases (GALNTs), the glycosylation enzymes controlled by GALA, is to harbour a lectin domain in addition to the catalytic domain. It has been shown that this lectin domain is essential for GALA effects. This is further derived from the data obtained using the ER-2Lec construct as a GALA-specific inhibitor, which resulted in preventing GALNT full activity in the endoplasmic reticulum specifically. Expressing the ER-2Lec inhibitor using the Cre-Lox system under a collagen VI promoter allowed targeting to synovial fibroblasts. The reduction of symptoms in the transgenic animals expressing ER-2Lec provides a clear demonstration of the importance of pathway for the disease.

Thus, in one example, the therapeutic agent disclosed herein is a fusion protein. In another example, the fusion protein is ER-2Lec. In yet another example, the fusion protein is ER-2Lec with the sequence of SEQ ID NO: 1.

GALA controls N-acetylgalactosaminyltransferases (GALNTs), glycosylation enzymes that act on thousands of proteins. One of the important targets of GALA is MMP14, whose glycosylation is critical for proteolytic activity. In rheumatoid arthritis and osteoarthritis, MMP14 and other matrix metalloproteinases (MMPs) have been shown to be upregulated in synovial fibroblasts and play critical roles for the degradation of collagen.

In addition, it had been recently shown that GALA activates at the liver cancer cell surface a complex composed of Calnexin and Protein disulfide-isomerase A3 (PDIA3). This complex mediates the cleavage of disulfide bonds in the extracellular matrix, an activity that is necessary for proteases to degrade the liver extracellular matrix. Here, it was shown that Calnexin (CNX) is also required for cartilage extracellular matrix degradation by synovial fibroblasts. Indeed, both the synovial fibroblast cell line and cells derived from a patient suffering from osteoarthritis had reduced degradative ability when incubated with anti-Calnexin antibodies.

It is of note that Calnexin is normally an intracellular, endoplasmic reticulum-located protein. Its trafficking at the cell surface appears to be controlled by GALA-induced glycosylation. Anti-Calnexin antibodies are therefore expected to bind mostly, or only, to cells with high GALA. However, high GALA levels are not the case in healthy tissue. This is thought to have helped concentrate the anti-CNX antibody in the joint tissue. In other words, the high concentration of anti-CNX antibody on the cell surface is caused by the high levels of GALA, which in turn resulted in the increased trafficking of CNX to the cell surface. This means that more CNX was detectable using the anti-CNX antibodies. A full characterisation of biodistribution is needed to confirm this point. It is also of note that during these experiments with anti-Calnexin antibodies, no adverse effects were shown or detected, as shown, for example in Fig. 22 showing unchanged body weight of animals receiving the CNX antibodies versus isotype control antibodies.

Overall, the results disclosed herein indicate that the GALA pathway is activated in synovial fibroblasts in both rheumatoid arthritis and osteoarthritis and is essential for the pathology of rheumatoid arthritis in mice.

As used herein, the term "arthritis" refers to any disorder that affects joints, usually including symptoms for example, but not limited to, joint pain and stiffness. Other symptoms can include, but are not limited to, redness, warmth, swelling, and decreased range of motion of the affected joints. In some types of arthritis, organs other than the joints of the subject can also be affected. In the context of the present disclosure, the methods disclosed herein are to be applied to various forms of arthritis. This is indicated, for example, by the fact that GALA is activated in rheumatoid arthritis, psoriasis arthritis and osteoarthritis. Without being bound by theory, it is thought that this common GALA activation indicates that said GALA activation is a relevant and targetable mechanism for treating any form of arthritis in which GALA is activated.

In the context of the present disclosure, the term "rheumatoid arthritis" or "RA" refers to a chronic systemic autoimmune disease that primarily involves the joints. Rheumatoid arthritis causes damage mediated by cytokines, chemokines, and metalloproteases. Characteristically, peripheral joints (for example, wrists, metacarpophalangeal joints) are symmetrically inflamed, leading to progressive destruction of articular structures, usually accompanied by systemic symptoms. Diagnosis is based on specific clinical, laboratory, and imaging features. Treatment involves drugs, physical measures, and sometimes surgery. Treatments, such as, but not limited to, disease-modifying, anti-rheumatic drugs (DMARDs), nonsteroidal anti-inflammatory drugs (NSAIDs), corticosteroids, immunomodulatory, cytotoxic, and immunosuppressive drugs (for example, azathioprine or cyclosporine) help to control symptoms and to slow disease progression.

In the context of the present disclosure, the term "osteoarthritis" or "OA" refers to a chronic disease of the joints (arthropathy) characterized by disruption and potential loss of joint cartilage along with other joint changes, including bone hypertrophy (osteophyte formation). Symptoms include gradually developing pain aggravated or triggered by activity, stiffness lasting at least 30 minutes or more on awakening and after inactivity, and occasional joint swelling. Diagnosis is confirmed by x-rays, and treatment includes physical measures, rehabilitation, patient education, and drugs. Osteoarthritis can be classified as being primary (idiopathic) or secondary, that is, as a result from conditions that change the microenvironment of the cartilage. Such conditions include, but are not limited to, significant trauma, congenital joint abnormalities, metabolic defects (for example, hemochromatosis, Wilson disease), infections (causing post-infectious arthritis), endocrine and neuropathic diseases, and disorders that alter the normal structure and function of hyaline cartilage (for example, rheumatoid arthritis, gout, chondrocalcinosis).

As used herein, the term "arthritis flare", "arthritic flare up", or "arthritic episode" is defined as an episode of increased disease activity or worsening symptoms. A flare is typically defined by the sudden intensity in joint pain accompanied by other characteristic symptoms such as, but not limited to fever, fatigue, malaise, stiffness, or joint swelling. A flare can involve a single joint or multiple joints. For example, a person with osteoarthritis will either have single joint involvement or recurrent flares with the same, multiple joints. By contrast, those with autoimmune arthritis, like rheumatoid arthritis or psoriatic arthritis, can often experience multiple joint flares concurrently. In one example, the methods disclosed herein are used to alleviate the symptom of arthritic flare ups.

In one example, the arthritis is selected from, but not limited to, the group of rheumatoid arthritis, psoriasis arthritis, juvenile idiopathic arthritis (JIA), osteoarthritis, and arthritic flares/flare up. In another example, the arthritis is rheumatoid arthritis. In yet another example, the arthritis is osteoarthritis. In yet another example, the arthritis is psoriasis arthritis. In a further example, the symptom to be treated is an arthritic flare/flare up.

In another example, the anti-rheumatic drug is, but not limited to, celecoxib, ibuprofen, nabumetone, naproxen sodium, naproxen, piroxicam, azathioprine cyclosporine, methotrexate, hydroxychloroquine, sulfasalazine, leflunomide, rituximab, abatacept, anakinra, tumor necrosis factor (TNF)-alpha antagonists (for example, but not limited to, adalimumab, etanercept, golimumab, certolizumab pegol, infliximab, and their biosimilars), sarilumab, tocilizumab, baricitinib, upadacitinib, filgotinib, and peficitinib, and combinations thereof.

As used herein, the term "treatment" refers to any and all uses which remedy a disease state or symptoms, prevent the establishment of disease, or otherwise prevent, hinder, retard, or reverse the progression of disease or other undesirable symptoms in any way. The methods disclosed herein can also be used to alleviate any symptoms of the diseases or conditions disclosed herein.

Also disclosed herein are kits or diagnostic kits for detecting arthritis in an individual, or susceptibility to arthritis in an individual. The diagnostic kit can comprise means for detecting expression, amount or activity of CNX, Protein disulfide-isomerase A4 (PDIA4), or Protein disulfide-isomerase A3 (PDIA3), or a complex of CNX and PDIA3 in an individual, by any means as described herein. The diagnostic kit can therefore comprise any one or more of the following: a CNX, PDIA4, or PDIA3 polynucleotide or a fragment thereof; a complementary nucleotide sequence to CNX, PDIA4, or PDIA3 nucleic acid or a fragment thereof; a CNX, PDIA4, or PDIA3 polypeptide or a fragment thereof, or an antibody to a CNX, PDIA4, or PDIA3. The diagnostic kit can comprise instructions for use, or other indicia. The diagnostic kit can further comprise means for treatment or prophylaxis of arthritis, such as any of the compositions described herein, or any means known in the art for treating arthritis.

In one example, the diagnostic kit comprises a CNX, PDIA4, PDIA3, and/or CNX:PDIA3 inhibitor as described herein. In another example, the diagnostic kit comprises a CNX, PDIA4, PDIA3, and/or CNX:PDIA3 inhibitor obtained by screening. The term "CNX:PDIA3" denotes a complex comprising CNX and PDIA3. The diagnostic kit can comprise a therapeutic drug such as, but not limited to, celecoxib, ibuprofen, nabumetone, naproxen sodium, naproxen, piroxicam, and combinations thereof, or other therapeutic agents as disclosed herein. The therapeutic agent can be, for example, an anti-CNX, anti-DPA14, or anti-PDIA3 antibody.

In yet another example, there is disclosed a kit for performing the method of detecting the presence of absence of arthritis in a subject according to the present disclosure.

Materials necessary for screening compounds or agents to identify agonists, antagonists, ligands, receptors, substrates, enzymes of Calnexin (CNX), Protein disulfide-isomerase A4 (PDIA4), or Protein disulfide-isomerase A3 (PDIA3), for example to identify polypeptides or compounds which decrease or enhance the production of CNX, PDIA4, or PDIA3, can be packaged into a screening kit. In one example, the screening kit comprises any one or more or all of the following: a CNX, PDIA4, or PDIA3 polypeptide; a recombinant cell expressing a CNX, PDIA4, or PDIA3 polypeptide; or an antibody to CNX, PDIA4, or PDIA3 polypeptide. The screening kit can further comprise a library. The screening kit can comprise any one or more of the components needed for screening, as described herein. The screening kit can optionally comprise instructions for use. Such screening kits can also be provided which are capable of detecting CNX, PDIA4, or PDIA3 expression at the nucleic acid level. Such kits can comprise a primer for amplification of CNX, PDAI4, or PDIA3, or a pair of primers for amplification. The primer or primers can be chosen from any suitable sequence, for example a portion of the CNX, PDIA4, or PDIA3 sequence. Methods of identifying primer sequences are well known in the art, and the skilled person will be able to design such primers with ease. The kits can comprise a nucleic acid probe for CNX, PDIA4, or PDIA3 expression, as described in this document. The kits can also optionally comprise instructions for use.

Also disclosed herein are polyclonal antibodies, for use in the methods and treatments as described herein. If polyclonal antibodies are desired, a selected mammal (for example, but not limited to, mouse, rabbit, goat, and horse) is immunised with an immunogenic composition comprising a Calnexin (CNX), Protein disulfide-isomerase A4 (PDIA4), or Protein disulfide-isomerase A3 (PDIA3) polypeptide or peptide. Depending on the host species, various adjuvants can be used to increase immunological response. Such adjuvants include, but are not limited to, Freund's, mineral gels such as aluminium hydroxide, and surface-active substances such as, but not limited to, lysolecithin, pluronic polyols, polyanions, peptides, oil emulsions, keyhole limpet hemocyanin, and dinitrophenol. BCG (*Bacilli Calmette-Guerin*) and *Corynebacterium parvum* are human adjuvants which can be employed, if purified the substance amino acid sequence is to be administered to immunologically compromised individuals for the purpose of stimulating systemic defence.

Serum from the immunised animal is collected and treated according to known procedures for obtaining antibodies. If serum containing polyclonal antibodies to an epitope obtainable from a Calnexin (CNX), Protein disulfide-isomerase A4 (PDIA4), or Protein disulfide-isomerase A3 (PDIA3) polypeptide contains antibodies to other antigens, the polyclonal antibodies can be purified by immunoaffinity chromatography. Techniques for producing and processing polyclonal antisera are known in the art. In order to make such antibodies, CNX, PDIA4, or PDIA3 amino acid sequences or fragments thereof can be haptenised to another amino acid sequence for use as immunogens in animals or humans.

Also included in the scope of the present disclosure are monoclonal antibodies directed against epitopes obtainable from a Calnexin (CNX), Protein disulfide-isomerase A4 (PDIA4), or Protein disulfide-isomerase A3 (PDIA3) polypeptide or peptide. These monoclonal antibodies can be readily produced by one skilled in the art. The general methodology for making monoclonal antibodies by hybridomas or hybridoma cell lines is well known in the art. Immortal, antibody-producing cell lines can be created by cell fusion, and also by other techniques such, but not limited to, as direct transformation of B lymphocytes with oncogenic DNA, or transfection with Epstein-Barr virus. Panels of monoclonal antibodies produced against orbit epitopes can be screened for various properties, for example and not limited to, isotype and epitope affinity.

Monoclonal antibodies can be prepared using any technique which provides for the production of antibody molecules by continuous cell lines in culture as known in the art.

Recombinant DNA technology can be used to improve the antibodies as described here. Thus, chimeric antibodies can be constructed in order to decrease the immunogenicity thereof in diagnostic or therapeutic applications. Such techniques comprise, for example, splicing of mouse antibody genes to human antibody genes to obtain a molecule with appropriate antigen specificity and biological activity. Moreover, immunogenicity can be minimised by humanising the antibodies by, for example, complementarity-determining regions (CDR) grafting and, optionally, framework modification, both methods known in the art.

Antibodies, both monoclonal and polyclonal, which are directed against epitopes obtainable from a Calnexin (CNX), Protein disulfide-isomerase A4 (PDIA4), or Protein disulfide-isomerase A3 (PDIA3) polypeptide or peptide are disclosed herein and are considered to be particularly useful in the methods disclosed herein, including diagnosis. Monoclonal antibodies, in particular, can be used to raise anti-idiotype antibodies. Anti-idiotype antibodies are immunoglobulins which carry an "internal image" of the substance and/or agent against which protection is desired. Techniques for raising anti-idiotype antibodies are known in the art. These anti-idiotype antibodies can also be useful in methods of treatment and therapy as disclosed herein. In one example, the antibodies can be bound to a solid support and/or packaged into kits in a suitable container along with suitable reagents, controls, instructions and the like.

Antibodies can also be produced by inducing *in vivo* production in the lymphocyte population or by screening recombinant immunoglobulin libraries or panels of highly specific binding reagents as disclosed in the art.

Antibody fragments containing specific binding sites for the target polypeptide or peptide can also be generated. For example, such fragments include, but are not limited to, the F(ab')₂ fragments which can be produced by pepsin digestion of the antibody molecule and the Fab fragments which can be generated by reducing the disulfide bridges of the F(ab')₂ fragments. Alternatively, Fab expression libraries can be constructed to allow rapid and easy identification of monoclonal Fab fragments with the desired specificity.

Techniques known in the art for the production of single chain antibodies can also be adapted to produce single chain antibodies to Calnexin (CNX), Protein disulfide-isomerase A4 (PDIA4), or Protein disulfide-isomerase A3 (PDIA3) polypeptides. Also, transgenic mice, or other organisms, including other mammals, can be used to express humanized antibodies.

The antibodies described herein can also be employed to isolate or to identify clones expressing the polypeptide or to purify the polypeptides by affinity chromatography.

Recombinant DNA technology can be used to produce the antibodies according to established procedure, in bacterial or mammalian cell culture. The selected cell culture system can secrete the antibody product.

An exemplary process for the production of an antibody comprises culturing a host, for example, but not limited to, *E. coli* or a mammalian cell, which has been transformed with a hybrid vector comprising an expression cassette comprising a promoter operably linked to a first DNA sequence encoding a signal peptide linked in the proper reading frame to a second DNA sequence encoding said antibody protein, and isolating said protein.

*In vitro* production provides relatively pure antibody preparations and allows scale-up to give large amounts of the desired antibodies. Techniques for bacterial cell, yeast or mammalian cell cultivation are known in the art and include homogeneous suspension culture, for example, in an airlift reactor or in a continuous stirrer reactor, or immobilised or entrapped cell culture, for example, in hollow fibres, microcapsules, on agarose microbeads or ceramic cartridges.

Hybridoma cells secreting the monoclonal antibodies are also provided. Hybridoma cells can be genetically stable, secrete monoclonal antibodies of the desired specificity and can be activated from deep-frozen cultures by thawing and recloning.

Also included herein is an exemplary process for the preparation of a hybridoma cell line secreting monoclonal antibodies directed to the Calnexin (CNX), Protein disulfide-isomerase A4 (PDIA4), or Protein disulfide-isomerase A3 (PDIA3) polypeptide, characterised in that a suitable mammal, for example a Balb/c mouse, is immunised with a one or more Calnexin (CNX), Protein disulfide-isomerase A4 (PDIA4), or Protein disulfide-isomerase A3 (PDIA3) polypeptides, or antigenic fragments thereof; antibody-producing cells of the immunised mammal are fused with cells of a suitable myeloma cell line, the hybrid cells obtained in the fusion are cloned, and cell clones secreting the desired antibodies are selected. For example, spleen cells of Balb/c mice immunised with Calnexin (CNX), Protein disulfide-isomerase A4 (PDIA4), or Protein disulfide-isomerase A3 (PDIA3) are fused with cells of the myeloma cell line PAI or the myeloma cell line Sp2/0-Ag14, the obtained hybrid cells are screened for secretion of the desired antibodies, and positive hybridoma cells are cloned.

Thus, described herein is a process for the preparation of a hybridoma cell line, characterised in that Balb/c mice are immunised by injecting subcutaneously and/or intraperitoneally between 10 and 10⁷ and 10⁸ cells expressing Calnexin (CNX), Protein disulfide-isomerase A4 (PDIA4), or Protein disulfide-isomerase A3 (PDIA3) and a suitable adjuvant several times, for example, four to six times, over several months, for example, between two and four months, and spleen cells from the immunised mice are taken two to four days after the last injection and fused with cells of the myeloma cell line PAI in the presence of a fusion promoter, such as polyethylene glycol. The myeloma cells can be fused with a three- to twenty-fold excess of spleen cells from the immunised mice in a solution containing about 30% to about 50% polyethylene glycol of a molecular weight around 4000. After fusion, the cells are expanded in suitable culture media as described herein, and supplemented with a selection medium, for example HAT medium, at regular intervals in order to prevent normal myeloma cells from overgrowing the desired hybridoma cells.

Recombinant DNA comprising an insert coding for a heavy chain variable domain and/or for a light chain variable domain of antibodies directed to Calnexin (CNX), Protein disulfide-isomerase A4 (PDIA4), or Protein disulfide-isomerase A3 (PDIA3) as described herein is also disclosed. By definition, such DNA comprises coding single stranded DNA or double stranded DNA consisting of said coding DNAs and of complementary DNAs thereto, or these complementary (single stranded) DNA themselves.

Furthermore, DNA encoding a heavy chain variable domain and/or for a light chain variable domain of antibodies directed to Calnexin (CNX), Protein disulfide-isomerase A4 (PDIA4), or Protein disulfide-isomerase A3 (PDIA3) can be enzymatically or chemically synthesised DNA having the authentic DNA sequence coding for a heavy chain variable domain and/or for the light chain variable domain, or a mutant thereof. A mutant of the authentic DNA is a DNA encoding a heavy chain variable domain and/or a light chain variable domain of the above-mentioned antibodies, in which one or more amino acids are deleted or exchanged with one or more other amino acids. The modification(s) can be, but is not limited to, regions be outside the complementarity-determining regions (CDRs) of the heavy chain variable domain and/or of the light chain variable domain of the antibody. Such mutant DNA can also be a silent mutant, wherein one or more nucleotides are replaced by other nucleotides, resulting in new codons coding for the same amino acid(s). Such a mutant sequence can also be a degenerated sequence. Degenerated sequences are degenerated, within the meaning of the genetic code in that an unlimited number of nucleotides are replaced by other nucleotides, without resulting in a change of the amino acid sequence originally encoded. Such degenerated sequences can be useful due to their different restriction sites and/or frequency of particular codons, which are preferred by the specific host, particularly *E. coli*, to obtain an optimal expression of the heavy chain murine variable domain and/or a light chain murine variable domain.

The term "mutant" also includes a DNA mutant obtained by *in vitro* mutagenesis of the authentic DNA according to methods known in the art.

For the assembly of, for example, complete tetrameric immunoglobulin molecules and the expression of chimeric antibodies, the recombinant DNA inserts coding for heavy and light chain variable domains are fused with the corresponding DNAs coding for heavy and light chain constant domains, then transferred into appropriate host cells, for example after incorporation into hybrid vectors.

Also disclosed herein are recombinant DNA comprising an insert coding for a heavy chain murine variable domain of an antibody directed to Calnexin (CNX), Protein disulfide-isomerase A4 (PDIA4) or Protein disulfide-isomerase A3 (PDIA3) fused to a human constant domain g, for example γ1, γ*2,* γ3 or γ4, such as γ1 or γ4. Also, recombinant DNA comprising an insert coding for a light chain murine variable domain of an antibody directed to Calnexin (CNX), Protein disulfide-isomerase A4 (PDIA4), or Protein disulfide-isomerase A3 (PDIA3) fused to a human constant domain κ or λ, such as κ are also disclosed.

In another example, there is disclosed recombinant DNA coding for a recombinant polypeptide, wherein the heavy chain variable domain and the light chain variable domain are linked by way of a spacer group, optionally comprising a signal sequence facilitating the processing of the antibody in the host cell and/or a DNA coding for a peptide facilitating the purification of the antibody and/or a cleavage site and/or a peptide spacer and/or an effector molecule.

Anti-Calnexin (CNX), anti-Protein disulfide-isomerase A4 (PDIA4) antibodies, and anti-Protein disulfide-isomerase A3 (PDIA3) antibodies can be used in method of detecting a CNX, PDIA4, or Protein disulfide-isomerase A3 (PDIA3) polypeptide present in biological samples. In one example, such a method comprises: (a) providing an anti-CNX, anti- PDIA4 antibody, or anti- PDIA3 antibody; (b) incubating a biological sample with the antibody under conditions which allow formation of an antibody-antigen complex; and (c) determining whether an antibody-antigen complex comprising said antibody is formed.

As used herein, the term "biological sample" or "sample" includes, but is not limited to, any quantity of a substance from a living thing or formerly living thing. Such living things include, but are not limited to, humans, mice, monkeys, rats, rabbits, and other animals. Such substances include, but are not limited to, blood, serum, plasma, urine, cells, organs, tissues, bone, bone marrow, lymph, lymph nodes, synovial tissue, synovial cells, tissue (particularly inflamed tissue), cartilage, and skin.

The antibodies disclosed herein, for example, against the CNX, PDIA4, or PDIA3 protein can be delivered into a cell by means of techniques known in the art, for example by the use of liposomes, polymers, (for example, but not limited to, polyethylene glycol (PEG), N-(2-hydroxypropyl) methacrylamide (HPMA) copolymers, polyamidoamine (PAMAM) dendrimers, HEMA, linear polyamidoamine polymers), and combinations thereof. The immunoglobulins and/or antibodies can also be delivered into cells as, for example, protein fusions or conjugates with a protein capable of crossing the plasma membrane and/or the nuclear membrane. For example, the immunoglobulin and/or target can be fused or conjugated to a domain or sequence from such a protein responsible for the translocational activity. Translocation domains and sequences can include domains and sequences from the HIV-1-trans-activating protein (Tat), *Drosophila* Antennapedia homeodomain protein and the herpes simplex-1 virus VP22 protein.

While it is possible for the Calnexin (CNX), Protein disulfide-isomerase A4 (PDIA4), or Protein disulfide-isomerase A3 (PDIA3) inhibitor to be administered alone, for example, as a CNX, PDIA4, or Protein disulfide-isomerase A3 (PDIA3) nucleic acid, polypeptide, fragment, homologue, variant or derivative thereof, modulator, agonist or antagonist, a structurally related compound, or an acidic salt of either, the active ingredient can be formulated as a pharmaceutical formulation.

Thus, also disclosed herein are pharmaceutical compositions comprising Calnexin (CNX), Protein disulfide-isomerase A4 (PDIA4), or Protein disulfide-isomerase A3 (PDIA3) inhibitor. In another example, pharmaceutical compositions comprising Calnexin (CNX) or Protein disulfide-isomerase A3 (PDIA3) inhibitor. Such pharmaceutical compositions can be used to deliver the Calnexin (CNX), Protein disulfide-isomerase A4 (PDIA4), or Protein disulfide-isomerase A3 (PDIA3) inhibitor, such as in the form of a composition as described, to an individual for the treatment or alleviation of symptoms and or disease described herein. In one example, the pharmaceutical composition is a composition of matter comprising at least a Calnexin (CNX), Protein disulfide-isomerase A4 (PDIA4), or Protein disulfide-isomerase A3 (PDIA3) inhibitor as an active ingredient.

The pharmaceutical formulations comprise an effective amount of the Calnexin (CNX) or Protein disulfide-isomerase A3 (PDIA3) inhibitor together with one or more pharmaceutically acceptable carriers. An "effective amount", as used herein, refers to the amount sufficient to alleviate at least one symptom of a disease as described. This so-called effective amount will vary depending upon the particular disease or syndrome to be treated or alleviated, as well as other factors including the age and weight of the patient, how advanced the disease state is, the general health of the patient, the severity of the symptoms, and whether the Calnexin (CNX) or Protein disulfide-isomerase A3 (PDIA3) inhibitor is being administered alone, or in combination with other therapies.

Suitable pharmaceutically acceptable carriers are well known in the art and vary with the desired form and mode of administration of the pharmaceutical formulation. For example, such carriers can include, but are not limited to, diluents or excipients such as fillers, binders, wetting agents, disintegrators, surface-active agents, lubricants and the like. Typically, the carrier is a solid, a liquid or a vaporizable carrier, or a combination thereof. Each carrier should be "acceptable" in that it is compatible with the other ingredients in the formulation and not injurious to the patient. Therefore, a carrier should be biologically acceptable without eliciting an adverse reaction (for example, an immune response or an allergic reaction) when administered to the host.

The active ingredient(s) of a pharmaceutical composition is contemplated to exhibit therapeutic activity, for example, in the alleviation of, for example, but not limited to, arthritis, rheumatoid arthritis, osteoarthritis, arthritic flare ups and other related diseases or symptoms. Dosage regimes can be adjusted to provide the optimum therapeutic response. For example, several divided doses can be administered daily, or the dose can be proportionally reduced as indicated by the exigencies of the therapeutic situation.

The active compound can be administered in a convenient manner such as by oral, intravenous (where water soluble), intramuscular, subcutaneous, intranasal, intradermal or suppository routes or by implanting (for example, using slow-release molecules). Depending on the route of administration chosen, the active ingredient may be required to be coated in a material to protect said ingredients from the action of enzymes, acids and other natural conditions which can inactivate said ingredient.

The inhibitors disclosed herein can be administered alone, or in combination with other therapeutic agents. Other therapeutic agents suitable for use herein are any compatible drugs that are effective for the intended purpose, or drugs that are complementary to the agent formulation. The formulation utilised in a combination therapy can be administered simultaneously, or sequentially with other treatment, such that a combined effect is achieved. Thus, in one example, the therapeutic agent is, but is not limited to, the group consisting of an antibody, a drug, a gene, a fusion protein, and an expression vector. In another example, the therapeutic agent is to be administered together, separately or sequentially. In a further example, the (first) therapeutic agent is to be administered with a further therapeutic agent, wherein the further therapeutic agent is an anti-rheumatic drug. In another example, such an anti-rheumatic drug is, but not limited to, celecoxib, ibuprofen, nabumetone, naproxen sodium, naproxen, piroxicam, azathioprine cyclosporine, methotrexate, hydroxychloroquine, sulfasalazine, leflunomide, rituximab, abatacept, anakinra, tumor necrosis factor (TNF)-alpha antagonists (for example, but not limited to, adalimumab, etanercept, golimumab, certolizumab pegol, infliximab, and their biosimilars), sarilumab, tocilizumab, baricitinib, upadacitinib, filgotinib, and peficitinib, and combinations thereof.

In some embodiments, the inhibitor of Calnexin (CNX), Protein disulfide-isomerase A4 (PDIA4), or Protein disulfide-isomerase A3 (PDIA3), or the CNX:PDIA3 complex, activity, expression or amount is provided as an oral composition and administered accordingly. The dosage of the inhibitor of Calnexin (CNX) or Protein disulfide-isomerase A3 (PDIA3) activity, expression or amount can be between about 1 mg/day to about 10 mg/day.

The pharmaceutical composition can be administered in an oral formulation in the form of tablets, capsules or solutions. An effective amount of the oral formulation is administered to patients one to three times daily until the symptoms of the disease alleviated.

The effective amount of agent depends on the age, weight and condition of a patient. In general, the daily oral dose of agent is less than 1200 mg, and more than 100 mg. The daily oral dose can be about 300mg to about 600 mg. Oral formulations are conveniently presented in a unit dosage form and can be prepared by any method known in the art of pharmacy. The composition can be formulated together with a suitable pharmaceutically acceptable carrier into any desired dosage form. Typical unit dosage forms include, but are not limited to, tablets, pills, powders, solutions, suspensions, emulsions, granules, capsules, suppositories. In general, the formulations are prepared by uniformly and intimately bringing into association the agent composition with liquid carriers or finely divided solid carriers or both, and as necessary, shaping the product. The active ingredient can be incorporated into a variety of basic materials in the form of a liquid, powder, tablets or capsules to give an effective amount of active ingredient to treat the disease.

The composition can be suitably orally administered, for example, with an inert diluent or with an assimilable edible carrier, or the composition can be enclosed in hard or softshell gelatine capsules, or compressed into tablets, or incorporated directly with the food of the diet. For oral therapeutic administration, the active compound

In some embodiments, the Calnexin (CNX), Protein disulfide-isomerase A4 (PDIA4), or Protein disulfide-isomerase A3 (PDIA3) inhibitor, or the inhibitor of the CNX:PDIA3 complex, is provided as an injectable or intravenous composition and administered accordingly. The dosage of the Calnexin (CNX) or Protein disulfide-isomerase A3 (PDIA3) inhibitor can be between about 5 mg/kg/2 weeks to about 10 mg/kg/2 weeks. The Calnexin (CNX) or Protein disulfide-isomerase A3 (PDIA3) inhibitor can be provided in a dosage of between 10 mg/day to 300 mg/day, such as, for example, at least 30 mg/day, less than 200 mg/day or between 30mg/day to 200 mg/day.

The pharmaceutical forms suitable for injectable use include sterile aqueous solutions (where water soluble) or dispersions and sterile powders for the extemporaneous preparation of sterile injectable solutions or dispersion. In all cases, the form must be sterile and must be fluid to the extent that the viscosity allows use of a syringe. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by using a coating such as, but not limited to, lecithin, by maintaining the required particle size in the case of dispersion, and by using surfactants.

The composition disclosed herein can be administered in an adjuvant, co-administered with enzyme inhibitors, or in liposomes. The term "adjuvant" is used in its broadest sense and includes, but is not limited to, any immune stimulating compound such as, for example, interferons. Adjuvants contemplated herein include, but are not limited to, resorcinols, non-ionic surfactants such as polyoxyethylene oleyl ether and n-hexadecyl polyethylene ether. Enzyme inhibitors include, but are not limited to, pancreatic trypsin. Liposomes include, but are not limited to, water-in-oil-in-water CGF emulsions as well as conventional liposomes.

The term "control group", "negative control" or "control" when used in the context of sample analysis refers to the use of samples obtained from diseases-free or healthy subjects, whereby these samples are then treated in the same manner as other sample, with the difference that the control samples are treated with, for example, buffers that do not contain the active compound or molecule in question. The comparison of the concentration of one or more targets (for example when comparing absolute concentrations or relative expression levels of a target), or the determination of the presence or absence of one or more targets as disclosed herein (for example, one or more proteins, oligomers or oligonucleotides) is determined based on the comparison of the levels determined in a sample obtained from a diseased subject and a sample obtained from a disease-free (or healthy) subject. In other words, a comparison of a target is based on the comparison of the level of one or more targets determined in the diseased subject and the level of the same one or more targets determined in a control group or control individual. In the present disclosure, the control sample is obtained from an individual that is disease-free. That is to say that the individual, from which the control sample is obtained, is free of the disease for which the test is undertaken. Usually, the term disease-free implies that the subject is healthy.

As used herein, the term "differential expression" refers to the measurement of a target in comparison to a control or another sample, and thereby determining the difference in, for example concentration, presence or intensity of said target. The result of such a comparison can be given in the absolute, that is a target is present in the samples and not in the control, or in the relative, that is the expression or concentration of target is increased or decreased compared to the control. The terms "increased" and "decreased" in this case can be used interchangeably with the terms "upregulated" and "downregulated".

As used herein, the term "pharmaceutically acceptable carrier and/or diluent" includes within its scope any and all solvents, dispersion media, coatings, antibacterial and antifungal agents, isotonic and absorption delaying agents and combinations thereof. The use of such media and agents for pharmaceutical active substances is well known in the art. Except insofar as any conventional media or agent is incompatible with the active ingredient, use thereof in the therapeutic compositions is contemplated. Supplementary active ingredients can also be incorporated into the compositions.

The invention illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the inventions embodied therein herein disclosed may be resorted to by those skilled in the art, and that such modifications and variations are considered to be within the scope of this invention.

### Functional properties

The agents, treatments, or other therapies described herein, such as antibodies and antigen binding fragments, can be defined by their functional properties. For example, a GALA inhibitor, Calnexin (CNX) inhibitor, Protein disulfide-isomerase A4 (PDIA4) inhibitor, or Protein disulfide-isomerase A3 (PDIA3) inhibitor, or an inhibitor of the CNX:PDIA3 complex can be defined by functional properties. Additionally, an anti-Calnexin (anti-CNX) antibody, an anti-Protein disulfide-isomerase A4 (anti-PDIA4) antibody, or an anti-Protein disulfide-isomerase A3 (anti-PDIA3) antibody, or an anti-CNX:PDIA3 complex antibody can be defined by functional properties.

In some cases, the agents, such as inhibitors, antibodies and antigen binding fragments, are capable of:
- reducing cartilage degradation
- reducing ECM degradation
- reducing an ECM degradation activity
- reducing an ECM degradation activity of CNX:PDIA3 (also known as Cnx/ERp57)
- reducing an ECM degradation activity of CNX
- reducing an ECM degradation activity of fibroblasts
- reducing an ECM degradation activity of synovial fibroblasts
- reducing an oxireductase activity
- reducing an oxireductase activity of CNX:PDIA3 (also known as Cnx/ERp57)
- reducing an oxireductase activity of CNX
- reducing disulphide bond reductase activity
- reducing a disulphide bond reductase activity of CNX:PDIA3 (also known as Cnx/ERp57)
- reducing a disulphide bond reductase activity of CNX
- reducing O-glycosylation
- reducing O-glycosylation of CNX
- reducing glycosylation of CNX
- reducing GALA mediated O-glycosylation of CNX
- reducing CNX activity
- reducing PDIA3 activity
- reducing PDIA4 activity
- reducing the number or proportion of cells expressing CNX
- reducing the number or proportion of cells expressing PDIA3
- reducing the number or proportion of cells expressing PDIA4
- reducing the number or proportion of cells expressing CNX:PDIA3

The functional properties highlighted above can all be effectively assayed though methods known in the art, for example through microscopy, qPCR, qrtPCR, western blotting, biochemical assays, enzymatic assays, and other techniques known by the skilled person.

Relative levels of cartilage degradation and cartilage degradation activity can be determined through many methods known in the art. For example, through well known methods such as microscopic analysis, conventional radiographs (X-rays), magnetic resonance imaging (MRI), computed tomography (CT), and ultrasound imaging. Additionally, the skilled person would be aware of biochemical and biomarker assays which can be used to determine the level of cartilage degradation. Serum biomarkers and urine biomarkers can be used to assay for the level of cartilage degradation. Biomarkers include Cpropeptide of type II procollagen (CPII), cartilage oligomeric matrix protein (COMP), collagenase generated carboxy-terminal neoepitope of type II collagen (sC2C), cartilage intermediate layer protein 2 (CILP-2), C-telopeptide of type II collagen (CTX-II) and collagenase-generated peptide(s) of type II collagen (C2C-HUSA).

The skilled person could determine whether cartilage degradation or cartilage degradation activity has been reduced/inhibited by comparison to a relevant control.

Relative levels of ECM degradation and ECM degradation activity can be determined through several methods. The skilled person is aware of a number of ways in which ECM degradation and ECM degradation activity can be measured, for example those shown in the examples of this specification. The skilled person could readily determine whether a Cnx/ERp57 inhibitor is capable of inhibiting ECM degradation activity, an ECM degradation activity of Cnx/ERp57, and/or an ECM degradation activity of Cnx.

The following steps are one way in which the skilled person can assay for ECM degradation activity.

A commercial solution of gelatin (2%) can be labeled with 5-Carboxy-X-Rhodamine, Succinimidyl Ester. The labeled gelatin can then be transferred on sterile coverslips to create a thin layer, before being stabilised by glutaraldehyde fixation. Finally, a solution of rat tail collagen can be used to coat the coverslips, creating a thin layer of collagen on top of the gelatin.

Then, the coverslips can be transferred in culture vessels and cells with degradative activity (for instance human hepatocellular carcinoma Huh7) are seeded and incubated for 48h to allow for degradation to occur.

After fixation, the coverslips can be stained with Hoescht to permit the counting of cells. The coverslips can then be imaged on a confocal microscope. The images can then be analysed using ImageJ. A threshold can be defined manually to reveal the surface of degraded gelatin and the total area per field was measured. In parallel, the number of nuclei can be calculated and the final result can be normalised to the cells in each field.

The skilled person could determine whether ECM degradation activity has been reduced/inhibited by comparison to a relevant control.

Relative levels of oxireductase activity can be determined by a number of methods. The skilled person is aware of a number of ways in which oxireductase activity can be measured, for example those shown in the examples of this specification. The skilled person could readily determine whether a Cnx/ERp57 inhibitor is capable of inhibiting oxireductase activity, an oxireductase activity of Cnx/ERp57, and/or an oxireductase activity of Cnx.

The use of an insulin reduction assay, e.g. as described in Hirano et al., Eur J Biochem. (1995) 234(1):336-42, is one way in which the skilled person could test for oxireductase activity.

The skilled person could determine whether oxireductase activity has been reduced/inhibited by comparison to a relevant control.

Relative levels of disulphide bond reductase activity can be determined by a number of methods. The skilled person is aware of a number of ways in which disulphide bond reductase activity can be measured, for example those shown in the examples of this specification. The skilled person could readily determine whether a Cnx/ERp57 inhibitor is capable of inhibiting disulphide bond reductase activity, a disulphide bond reductase activity of Cnx/ERp57, and/or a disulphide bond reductase activity of Cnx.

The following steps are one way in which the skilled person can assay for disulphide bond reductase activity: (1) Plate out ECM (1mg/ml collagen with 25 ug/ml fibronectin (1 h polymerisation at 37'C) or 0.5 mg/ml Matrigel (2.5 h polymerisation at RT)), (2) Seed cells at appropriate density, (3) Allow cells to adhere (~45 min), (4) Add antibodies at 10 ug/ml with 10-25 mM GM6001, (5) Incubate for 72h, (6) Add hoechst to visualise nuclei, (7) Decellularise using warmed 20 mM Ammonium hydroxide , 0.5 % (v/v) triton X-100 for 1-2 min, (8) Wash with 1% BSA in PBS, (9) Add 2.5 mM TCEP for controls for 30 min at RT, (10) Wash with 1% BSA in PBS, (11) Add 5 mM NEM in 1% BSA for 30 min at RT, (12) Wash 1% BSA in PBS, (13) Fix with 4% PFA, (14) Wash with PBS, (15) Primary antibody mouse anti-NEM OX133 (1:200) and IgG controls overnight at 4'C, (16) Wash with PBS, (17) Secondary antibody anti-mouse 647 (1:400) and secondary only controls for 45 min at RT, (18) Wash with PBS, and (19) Visualise the same wells again to determine the cysteine reduction signal and normalise to number of cells.

The skilled person could determine whether disulphide bond reductase activity has been reduced/inhibited by comparison to a relevant control.

The presence and relative level of glycosylation can be determined by methods well known in the art, such as methods used in the examples and commercial assay kits. Methods to detect and analyze glycosylation and glycoproteins include: glycan staining or labelling, glycoprotein purification or enrichment, and analysis by mass spectrometry. The skilled person would be aware of such methods.

The presence and relative level of O-glycosylation can be determined through methods known in the art, such as those used in the examples of this specification. A hallmark of O-glycosylation is increased cellular levels of Tn (T nouvelle), the O-glycan formed by the addition of a GalNac to a Ser or Thr residue. Tn can be detected by Tn-binding proteins such as Vicia Villosa Lectin (VVL) and Helix Pomatia Lectin (HPL) (Gill, et al., Proc. Natl. Acad. Sci. U. S. A. 110, E3152-61 2013).

Relative levels of CNX, PDIA3 can be determined through methods known in the art. For example, the relative levels of gene expression (e.g. calnexin expression) can be determined through a number of different methods known by the skilled person. The level of RNA encoding a given gene can be determined e.g. by techniques such as RNAseq, RT-PCR, and RT-qPCR amongst other well known methods. Additionally, the relative level of protein expression (e.g. calnexin expression) can be determined by means well known to the skilled person. The level of a given protein/isoform thereof can be determined e.g. by antibody-based methods including western blot, immunohisto/cytochemistry, flow cytometry, ELISA, etc. Furthermore, CNX, PDIA3, and/or PDIA4 activity can be determined by isolating enzymes and performing enzymatic assays.

The number or proportion of cells expressing CNX, PDIA3, PDIA4, and/or CNX:PDIA3 can be determined by several methods, including single cell transcriptomic analysis, microscopy with mmunohistochemistry, and flow cytometry.

As highlighted above, the therapeutic agent (e.g. GALA inhibitor, Calnexin (CNX) inhibitor, Protein disulfide-isomerase A4 (PDIA4) inhibitor, or Protein disulfide-isomerase A3 (PDIA3) inhibitor, inhibitor of the CNX:PDIA3 complex, or anti-calnexin antibody) may lead to a reduction of a certain characteristic or activity (e.g. ECM degradation, oxireductase activity, disulphide bond reductase activity, glycosylation, protein activity, or number or proportion of a specific cell-type). In some cases, this reduction may be greater than 5%, e.g. one of ≥10%, ≥15%, ≥20%, ≥25%, ≥30%, ≥35%, ≥40%, ≥45%, ≥50%, ≥55%, ≥60%, ≥65%, ≥70%, ≥75%, ≥80%, ≥85%, ≥90%, ≥91%, ≥92%, ≥93%, ≥94%, ≥95%, ≥96%, ≥97%, ≥98%, ≥99% or 100% of the level observed in a relevant control.

The relevant control may be the uninhibited state i.e. the level of the characteristic before treatment.

In some embodiments, the therapeutic agent (e.g. GALA inhibitor, Calnexin (CNX) inhibitor, Protein disulfide-isomerase A4 (PDIA4) inhibitor, or Protein disulfide-isomerase A3 (PDIA3) inhibitor, inhibitor of the CNX:PDIA3 complex, or anti-calnexin antibody) leads to a reduction in the level of ECM degradation. In some cases, this reduction in ECM degradation may be greater than 5%, e.g. one of ≥10%, ≥15%, ≥20%, ≥25%, ≥30%, ≥35%, ≥40%, ≥45%, ≥50%, ≥55%,≥60%, ≥65%, ≥70%, ≥75%, ≥80%, ≥85%, ≥90%, ≥91%, ≥92%, ≥93%, ≥94%, ≥95%, ≥96%, ≥97%, ≥98%,≥99% or 100% of the level observed in a relevant control (e.g. in the uninhibited state).

In some embodiments, the therapeutic agent (e.g. GALA inhibitor, Calnexin (CNX) inhibitor, Protein disulfide-isomerase A4 (PDIA4) inhibitor, or Protein disulfide-isomerase A3 (PDIA3) inhibitor, inhibitor of the CNX:PDIA3 complex, or anti-calnexin antibody) leads to a reduction in the level of an ECM degradation activity (e.g. an ECM degradation activity of CNX, ECM degradation activity of CNX/ERP57, or ECM degradation activity of synovial fibroblasts). In some cases, this reduction in ECM degradation activity may be greater than 5%, e.g. one of ≥10%, ≥15%, ≥20%, ≥25%, ≥30%, ≥35%, ≥40%, ≥45%, ≥50%, ≥55%, ≥60%, ≥65%, ≥70%, ≥75%, ≥80%, ≥85%, ≥90%, ≥91%, ≥92%, ≥93%, ≥94%, ≥95%, ≥96%, ≥97%, ≥98%, ≥99% or 100% of the level observed in a relevant control (e.g. in the uninhibited state).

In some embodiments, the therapeutic agent (e.g. GALA inhibitor, Calnexin (CNX) inhibitor, Protein disulfide-isomerase A4 (PDIA4) inhibitor, or Protein disulfide-isomerase A3 (PDIA3) inhibitor, inhibitor of the CNX:PDIA3 complex, or anti-calnexin antibody) leads to a reduction in the level of an oxireductase activity (e.g. an ECM degradation activity of CNX, ECM degradation activity of CNX/ERP57, or ECM degradation activity of synovial fibroblasts). In some cases, this reduction in oxireductase activity may be greater than 5%, e.g. one of ≥10%, ≥15%, ≥20%, ≥25%, ≥30%, ≥35%, ≥40%, ≥45%, ≥50%, ≥55%, ≥60%, ≥65%, ≥70%, ≥75%, ≥80%, ≥85%, ≥90%, ≥91%, ≥92%, ≥93%, ≥94%, ≥95%, ≥96%, ≥97%, ≥98%, ≥99% or 100% of the level observed in a relevant control (e.g. in the uninhibited state).

In some embodiments, the therapeutic agent (e.g. GALA inhibitor, Calnexin (CNX) inhibitor, Protein disulfide-isomerase A4 (PDIA4) inhibitor, or Protein disulfide-isomerase A3 (PDIA3) inhibitor, inhibitor of the CNX:PDIA3 complex, or anti-calnexin antibody) leads to a reduction in the level of a disulfide bond reductase activity (e.g. an ECM degradation activity of CNX, ECM degradation activity of CNX/ERP57, or ECM degradation activity of synovial fibroblasts). In some cases, this reduction in disulfide bond reductase activity may be greater than 5%, e.g. one of ≥10%, ≥15%, ≥20%, ≥25%, ≥30%, ≥35%, ≥40%, ≥45%, ≥50%, ≥55%, ≥60%, ≥65%, ≥70%, ≥75%, ≥80%, ≥85%, ≥90%, ≥91%, ≥92%, ≥93%, ≥94%, ≥95%, ≥96%, ≥97%, ≥98%, ≥99% or 100% of the level observed in a relevant control (e.g. in the uninhibited state).

### General terms

As used in this application, the singular form "a," "an," and "the" include plural references unless the context clearly dictates otherwise. For example, the term "a genetic marker" includes a plurality of genetic markers, including mixtures and combinations thereof.

As used herein, the term "about", in the context of concentrations of components of the formulations, typically means +/- 5% of the stated value, more typically +/- 4% of the stated value, more typically +/- 3% of the stated value, more typically, +/- 2% of the stated value, even more typically +/- 1% of the stated value, and even more typically +/- 0.5% of the stated value.

Throughout this disclosure, certain embodiments may be disclosed in a range format. It should be understood that the description in range format is merely for convenience and brevity and should not be construed as an inflexible limitation on the scope of the disclosed ranges. Accordingly, the description of a range should be considered to have specifically disclosed all the possible sub-ranges as well as individual numerical values within that range. For example, description of a range such as from 1 to 6 should be considered to have specifically disclosed sub-ranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Certain embodiments may also be described broadly and generically herein. Each of the narrower species and sub-generic groupings falling within the generic disclosure also form part of the disclosure. This includes the generic description of the embodiments with a proviso or negative limitation removing any subject matter from the genus, regardless of whether or not the excised material is specifically recited herein.

The invention has been described broadly and generically herein. Each of the narrower species and sub-generic groupings falling within the generic disclosure also form part of the invention. This includes the generic description of the invention with a proviso or negative limitation removing any subject matter from the genus, regardless of whether or not the excised material is specifically recited herein.

Other embodiments are within the following claims and non- limiting examples. In addition, where features or aspects of the invention are described in terms of Markush groups, those skilled in the art will recognize that the invention is also thereby described in terms of any individual member or subgroup of members of the Markush group.

### EXPERIMENTAL SECTION

### EXAMPLE 1

### Patient samples, cell lines and mouse strains

Patient samples: synovial tissue specimens were obtained from patients with rheumatoid arthritis (RA) or osteoarthritis (OA), who were undergoing joint replacement surgery at the Tan Tock Seng Hospital (Singapore). The procedures were approved by the Ethics Committee of The National Healthcare Group domain specific review board under protocol no. 2018/00980. All patients were given written consent and met the diagnostic criteria for rheumatoid arthritis or osteoarthritis.

Cell line: The SW982 cells (ATCC HTB-93) are synovial fibroblast cells derived from synovium of a patient with synovial sarcoma. SW982 cells were maintained in Leibovitz's L-15 Medium (Gibco; ThermoFisher Scientific) supplemented with 10% (v/v) foetal calf serum (FCS) and 1% (w/v) penicillin/streptomycin (Gibco; ThermoFisher Scientific) at 37°C in a free gas exchange with atmospheric air. The SW982 cells were engineered to stably express a doxycycline-inducible gene encoding the ER-2Lec using the Sleeping Beauty transposon system.

Mice: Col6a1Cre mice expressing collagen type VI promoter driven on the C57BL/6J background were provided by G. Bressan (University of Milano, Milano, Italy). It is noted that comparable murine models known in the art can be used as a background to generate the mice required according to the present disclosure. ER-2Lec mice on the same background expressing an endoplasmic reticulum (ER)-localized double-lectin domain (ER-2Lec) under the control of a loxP-flanked STOP cassette were custom generated according to specifics provided by the inventors by Ozgene. Col6a1Cre ER-2Lec mice were generated by crossing Col6a1Cre mice with ER-2Lec mice, resulting in mice expressing ER-2Lec in the mesenchymal cell lineages. All animals were bred and maintained under specific pathogen-free conditions in micro-isolator cages with access to food and water at Biological Resource Centre (ASTAR, Singapore). Experiments were performed using age- and sex-matched animals and complied with guidelines approved by the Animal Ethics Committees at Biological Research Centre (ASTAR, Singapore) under the protocol IACUC no. 201548.

### Isolation of primary synovial fibroblasts (SF) from human tissues

Synovial tissues from osteoarthritis (OA) and rheumatoid arthritis (RA) patients were obtained at the time of synovectomy or synovial biopsy. Following excision, the tissues were immediately minced and digested in collagenase IV (1 mg/ml, Gibco) in Dulbecco's modified Eagle medium (DMEM) for 1.5 hours at 37°C with gentle agitation. The mixture was passed through a 70 µm mesh cell strainer and cell pellet was obtained after centrifugation at 250 g for 10 minutes. Human synovial fibroblasts from osteoarthritis and rheumatoid arthritis patients (OASF and RASF, respectively) were used between passages 3 and 9 (Rosengren et al., 2007, Methods in Molecular Medicine, Vol. 135: Arthritis Research, Volume 1). The purity of culture was confirmed by staining with fibroblast cell identity marker CD90 prior to performing experiments. Normal human synovial fibroblasts were derived from synovial tissues from a healthy human donor (HCSF) and obtained from the Cell Applications, Inc. HCSF were cultured in complete in DMEM supplemented with 10% (v/v) foetal calf serum (FCS) and 1% (w/v) penicillin/streptomycin (Gibco; ThermoFisher Scientific) at 37°C in a humidified atmosphere containing 5% CO₂.

### Reagents

Antibodies: Anti-CNX (ab10286, ab22595), Anti-Vimentin (ab92547), anti-beta actin (ab8226) antibodies were purchased from Abcam. Agarose-bound *Vicia Villosa* Lectin (VVL, AL-1233) was purchased from Vector Laboratories. PE.C7 conjugated anti-CD45 and PE conjugated anti-CD90 were purchased from Biolegend. Anti-FAPα was purchased from R&D systems. Anti-NEM OX133 antibody was purchased from Absolute Antibody. Anti-rabbit IgG-HRP antibody and anti-mouse IgG-horse radish peroxidase (HRP) antibody were purchased from GE Healthcare Life Sciences.

Plasmids: The plasmid expressing doxycycline inducible ER-2Lec was generated as previously described (Gill et al. 2013, PNAS, 2013, E3152-E3161). The resulting vectors, together with the pPGK-SB13 expressing Sleeping Beauty 208 transposase, were used to transfect SW982 cells.

### Immunofluorescence staining

Formalin-fixed, paraffin-embedded tissue sections were deparaffinized in xylene substitute buffer (Sub-X, Leica Biosystems) and rehydrated. For mouse joint tissue and tissue microarray (provitro AG, Berlin, Germany), antigen retrieval was performed by immersion in Epitope Retrieval Solution pH 6 (Leica Biosystems) and incubated at 60°C in an oven for 18 hours. For human tissue specimens, antigen retrieval was performed using Epitope Retrieval Solution pH 6 (Leica Biosystems) in a pressure chamber (2100 Retriever, Akribis Scientific Limited, WA16 0JG, GB). Sections were washed twice with phosphate buffered saline (PBS) and immersed in a blocking buffer (5% horse serum, 1% Triton X100). After 1 hour, sections were incubated overnight at 4°C with a primary antibody mix containing primary antibodies including rabbit anti-CD45, rabbit anti-Vimentin, rabbit anti-Calnexin, rat anti-FAPα, or biotin conjugated *Vicia Villosa* lectin (VVL). Samples were washed three times with blocking buffer and incubated for 2 hours with the corresponding secondary antibodies including anti-rat Alexa Fluor647, anti-rabbit Alexa Fluor 594-conjugated or Alexa Fluor488 streptavidin (ThermoFisher Scientific, 1:400). After washing, cell nuclei were stained with Hoechst 33342 (ThermoFisher Scientific; 1:1000) for 5 minutes before mounting. All images were captured using the same settings on the LSM-700 (Zeiss) confocal microscope. Raw integrated density of VVL and nuclear signals were measured by FIJI image calculator.

### Collagen antibody induced arthritis

On day 0, mice were injected with an antibody cocktail mix containing five different monoclonal antibody clones against collagen type II proteins (Chondrex Inc.) at a dose of 2 mg/mouse (200 µl) via intraperitoneal (IP) injections. On day 3, animals were injected with LPS at 50 µg/mouse via IP injections (100 µl). From day 3 on, arthritis severity was assessed daily by measuring paw thickness using a digital calliper. Assessment was also done by a blinded researcher using a qualitative clinical scoring system provided by Chondrex, Inc.

### Histology analysis and Cartilage extracellular matrix staining

After removing skin, both front and hind paws were formalin-fixed and embedded in paraffin. Tissues were de-paraffinized and rehydrated as described above in the above immunofluorescent staining protocol. Tissue sections were stained with Haematoxylin and Eosin (HE), Safranin-O (SO), and Alcian Blue (AB) stains. Slides were scanned at 20x using a Leica SCN400 slide scanner (Leica Microsystems, Germany). Images were exported to Slidepath Digital Image Hub (Leica Microsystems, Germany) for viewing. Selected regions were analysed using Measure Stained Area Assay of Slidepath Tissue Image Analysis 2.0 software (Leica Microsystems, Germany). A quantitative analysis of cartilage extracellular matrix (ECM) staining areas was performed using FIJI image calculator.

### Flow cytometry

Skin from the feet was removed and the joints were cut 3 mm above the heel. To avoid contamination with the bone marrow, bone marrow cavity in the tibia was thoroughly flushed with Hank's balanced salt solution (HBSS). The joints were cut into small pieces and incubated in digestion buffer (1 mg/ml collagenase IV and 1 mg/ml of DNase I in HBSS) for 60 minutes at 37°C. Cells released during the digestion were filtered through 70 µm cell strainers; erythrocytes were lysed using a red blood lysis buffer (BD Biosciences). The cells were stained with live/dead Aqua (Invitrogen) viability dyes, incubated with Fc Block at 1:50 (BD Biosciences), and stained with fluorochrome-conjugated antibodies including PE-conjugated anti-CD90 (Biolegend), PECy7-conjugated anti-CD45 (Biolegend) and FITC-conjugated *Vicia Villosa* lectin (VVL; Life Technologies) for 30 minutes. For intracellular staining, cells were fixed by using BD Cytofix/Cytoperm solution (BD Biosciences). Fixed cells were permeabilized in 1x BD Perm/Wash buffer (BD Biosciences) prior staining with FITC conjugated *Vicia Villosa* lectin (VVL). Samples were acquired on FACS BD LSRII and analysed using Kaluza softwares.

### Western blot and VVL-Coimmunoprecipitatio (VVL-CoIP)

Cells were seeded at 2×10⁵ cell/ml in 10 cm dishes pre-coated with 2 mg/ml cartilage extracellular matrix (ECM; Xylyx Bio.) and left to rest overnight. After stimulation with 100 µg/ml TNFα (PeproTech) and 100 µg/ml IL-1β (PeproTech) for 24 hours, cells were harvested and lysed in a low-stringency RIPA lysis buffer (50 mM Tris, 200 mM NaCl, 0.5% NP-40, Complete and PhoStop inhibitor [Roche Applied Science]) and lysed for 30 minutes at 4°C. Lysates were then clarified by centrifugation at 13000 g for 10 minutes at 4°C. Clarified tissue lysates were incubated with agarose-bound *Vicia Villosa* lectin (VVL) beads (Vector Laboratories) overnight at 4°C. Beads were washed three times with RIPA lysis buffer, and the precipitated proteins were eluted in 2x LDS sample buffer containing 50 mM DTT. Lysates were boiled at 95°C for 5 minutes and separated by SDS-PAGE electrophoresis using 4-12% Bis-Tris 80 NuPage gels (Invitrogen) at 180 V for 70 minutes. Samples were then transferred on nitrocellulose membranes using iBlot transfer system (Invitrogen) and blocked using 3% bovine serum albumin (BSA) dissolved in TBST (tris-buffered saline (TBS) and Polysorbate 20 (also known as Tween 20)- 50 mM Tris, 150 mM NaCl and 0.1% Tween-20) for 1 hour at room temperature. The nitrocellulose membranes were then incubated with primary antibodies (1/1000 diluted in 3% BSA-TBST) overnight at 4°C. The next day, membranes were washed three times with TBST and incubated with secondary antibodies conjugated with horseradish peroxidase (HRP) for 2 hours at room temperature. Membranes were washed three more times with TBST before electrochemiluminescence (ECL) exposure.

### Matrix degradation assay

Red gelatine coverslips were prepared as previously described (Ros et al., Nat Cell Biol, 2020, vol 22, November 2020, 1371-1381). The coverslips were coated with 0.2 mg/ml cartilage extracellular matrix (ECM; Xylyx Bio) for 3 hours at 37°C. Synovial fibroblasts cells (including SW982 cells, osteoarthritis synovial fibroblasts (OASF), rheumatoid arthritis synovial fibroblasts (RASF) and healthy human donor synovial fibroblasts (HCSF)) were seeded at 5×10⁴ cells/ml/well in 24-well plates overnight. Cells were then stimulated with 100 µg/ml TNFα and 100 µg/ml IL-1β. After 24 hours, the cells were fixed with 4% paraformaldehyde (PFA) and stained for nuclear using Hoechst 33342 (Life Technologies). Stained coverslips were mounted onto glass microscope slides and 10 to 30 images were acquired for each condition. The normalized area of matrix degradation relative to the number of cells was measured by using ImageJ software as previously described. (Martin et al., J Vis Expr, 2012, vol 66, e4119) Briefly, the area of degradation using the fluorescent gelatine images after thresholding and the same threshold were applied for all images. The cell counter tool was used to count the number of nuclei and the area of gelatine degradation per total number of cells was calculated. Experiments were done in three biological replicates.

### Statistical analyses

GraphPad Prism (version 8.4.3, GraphPad Software, CA, USA) was used for statistical analyses and graphical preparation. Data analysis was performed by one-way (Kruskal Wallis test), two-way ANOVA (Tukey's multiple comparisons test) or Mann-Whitney test as indicated. Differences were considered statistically significant for p-values < 0.05.

### EXAMPLE 2

### Results

### Enhanced O-glycosylation in Rheumatoid Arthritis and Osteoarthritis synovium

A hallmark of GALA is increased cellular levels of Tn (T nouvelle), the O-glycan formed by the addition of a GalNac to a Ser or Thr residue. Tn can be detected by Tn-binding proteins such as Vicia Villosa Lectin (VVL) and Helix Pomatia Lectin (HPL) (Gill, et al., Proc. Natl. Acad. Sci. U. S. A. 110, E3152-61 2013).

We analysed microarrays of joint tissues by immunofluorescence using VVL and counterstained for DNA. We detected a distinct increase in Tn samples in 18/21 samples from OA patients, 2/6 samples from patients with Psoriasis arthritis and 9/18 samples of RA patients (Fig. 1A and fig. 23). We quantified the integrated fluorescence intensity of VVL staining normalised to the DNA signal intensity as a marker of cell density (Fig. 1B). While healthy patient samples showed little variation, most samples of RA and OA samples displayed enhanced Tn levels, with in some areas with up to seven-fold increase of VVL signal.

To further characterise GALA in arthritis, we adopted a mouse model of RA based on Collagen Antibody Induced Arthritis (CAIA) (32). Briefly, animals were injected with an antibody against collagen type II, then 3 days later with lipopolysaccharide (LPS) and developed symptoms starting at day 5. Initial immuno-histochemistry analysis revealed a marked increase in the levels of Tn in and around the joint in animals with arthritis (Fig. 23).

In the CAIA model, the symptoms took about 7 days to reach a peak and lasted for 10 days before slowly decreasing. We sampled animals on day 0, 7, 10 and 14 and used immunofluorescence staining to quantify Tn levels. Histologically, a pannus invading the joint cavity was apparent on day 7 and increased in size with the influx of immune cells on day 10. By day 14, the amount of immune cells had drastically diminished but synovial tissue remained in the joint cavity (Fig. 2A and B). High levels of Tn were observed in cells invading the joint cavity at day 7 and persisted till day 10 (Fig. 2C and D). By day 14, cellular Tn levels had subsided in a large fraction of the animals. We observed some Tn staining in fibrous material that was devoid of cells, and this staining did not decrease significantly at day 14.

### High Tn levels in the arthritic synovium are consistent with GALA activation

High cellular Tn levels can be induced by the GALA pathway, which is characterised by abundant Tn signal in the ER. Because the ER is distributed throughout the cell body, GALA activation typically shows up as an increased diffuse signal (Bard et al., Trends Cell Biol. 26, 379-388, 2016). We co-stained CAIA synovium samples for Tn and Calnexin, an ER marker (Fig. 3A). In untreated samples, Tn and Calnexin staining were clearly separated, with Tn concentrated in a perinuclear pattern consistent with the Golgi. By contrast, in day 7 CAIA samples, Tn staining was elevated, filling the cellular space and colocalizing with the ER marker Calnexin, strongly suggestive of GALA induction. To corroborate GALA induction in the CAIA context, we stained with an GALNT2, a ubiquitously expressed GALNT transferase previously shown to be relocalized to ER in cancer with GALA activation. A similar change in localisation from perinuclear in untreated sample to ER pattern in CAIA sample with colocalization with CNX was observed (Fig. 3B). Our results suggest the relocation of GALNT2 from the Golgi to ER as the basis of Tn levels increase in CAIA context. These results are in line with the previously reported description of GALA phenotype in breast and liver cancer (Gill, et al., Proc. Natl. Acad. Sci. U. S. A. 110, E3152-61, 2013; Nguyen, et al., Cancer Cell. 32, 639-653.e6, 2017).

### Synovial fibroblasts are the major cell type displaying GALA

The synovium in RA disease is a complex tissue comprising immune cells and synovial fibroblasts (Choy et al., Rheumatology . 51 Suppl 5, v3-11, 2012). To establish which cell type displayed increased GALA, we co-stained 7d mouse CAIA joint samples with VVL, CD45 a marker of immune cells and vimentin a marker of fibroblasts. We observed vimentin positive cells at the forefront of the invading panus (Fig. 4). CD45 positive cells were typically clustered and located behind the invading front of the pannus. Remarkably, Tn positive cells in the pannus largely co-stained with vimentin positive region and not CD45 positive region. This result suggests GALA activation in synovial fibroblast (Fig. 4). We validated this result in human samples of RA and OA, using the Fibroblast Activated Protein alpha (FAP*α*) as a marker of synovial lining fibroblasts (Bauer et al. Arthritis Res. Ther. 8, R171, 2006). The FAP*α* positive cells formed a layer located at the edge of the pannus, with a larger layer of CD45 positive cells behind in RA sample (Fig. 5A, 5B and fig. 24A). In OA samples, the number of CD45 cells was reduced compared to RA samples but FAP*α* cells displayed similar VVL staining (Fig. 24). Strikingly, VVL staining co-localised exclusively with FAP*α* in both RA and OA conditions. Thus, lining synovial fibroblasts are the major cells displaying GALA activation in OA and RA synovium.

### Stimulation of SF by cytokines and ECM induces higher GALA levels

To understand how GALA is activated in vivo, we used primary human SFs derived from patients. FACS analysis using CD90 and CD45 as markers of SFs and immune cells respectively established the >90% purity of the cell preparations we used (Fig. 24B). We then performed high content imaging analysis of Tn level in these SF patient derived cells with HPL staining. Seeding cells on plastic wells showed increased levels of Tn in OA and even more in RA cells as compared to healthy SF (Fig. 6). Then, we stimulated SFs with TNFα and IL1**β** cytokines, which are thought to drive disease progression in RA (Kagari and Shimozato. J. Immunol. 169, 1459-1466, 2002). Individual cytokines had a relatively limited effect on GALA activation, however the combination of both cytokines (labelled CYTO) induced a 2 fold increase in Tn cellular levels. Interestingly, the effect was marked in RASF and more limited in OASF and almost nonexistent in healthy SFs (HCSF).

Next, we wondered whether cartilage ECM proteins might contribute to the activation of SF. Exposing SF to cartilage ECM activated GALA by up to three fold in both OASF and RASF. By contrast, HCSF cells were almost non-responsive. The combination of CYTO and ECM had an additive effect on Tn levels (Fig. 6). The stimulation was not specific for cartilage ECM as the use of rat tail derived collagen I induced a similar activation (Fig. 6).

Under unstimulated conditions, Tn staining was exclusively detected in the Golgi of HCSF, whereas OASF and RASF displayed additional ER-like Tn staining (Fig. 6). Upon stimulation with a combination of CYTO and cartilage ECM, the ER-localized Tn staining was enhanced in both OASF and RASF but not HCSF (Fig. 6).

To summarize, this analysis revealed that OA and RA patients' SFs have elevated GALA compared to healthy SFs in cell culture. RA SFs activate further GALA in response to cytokines, while both RA and OA SFs activate GALA upon exposure to ECM. By contrast, healthy SFs had very limited GALA responses, suggesting the pathway is primed for activation in patients' cells.

### Inhibition of GALA in SF reduces ECM degradation and arthritis in vivo

As GALA drives ECM degradation in cancer cells, we sought to test whether it is also implicated in cartilage ECM degradation by SFs. We found that the human synovial sarcoma SW982 cells are able to degrade collagen (in a sandwich assay with fluorescent gelatin previously described) (Ros et al., Nat. Cell Biol. 22, 1371-1381, 2020). We described previously the ER-2Lec chimeric protein, composed of an ER-targeting sequence and a fusion of two lectins of GALNT2 (Gill, et al., Proc. Natl. Acad. Sci. U. S. A. 110, E3152-61 2013). ER-2Lec inhibits specifically the activity of GALA by interfering with ER O-glycosylation.

We generated a stable SW982 transfectant with ER-2Lec under a Doxycycline inducible promoter system. Similarly to RA SFs, SW982 cells stimulated with the CYTO mix were more active at degrading the ECM (Fig. 7B and 7C). However, when ER-2Lec expression was induced, the degradation was reduced significantly, by more than 2 fold (Fig. 7B and 7C).

To test whether ER-2Lec could reduce arthritis in vivo, we generated a transgenic mouse line with ER-2Lec with a Lox cassette. We crossed them with mice expressing Cre under the collagen VI alpha1 (Col6a1) promoter (Fig. 3C). Collagen VI is expressed by joint mesenchymal cells, and in particular SFs (Danks et al., Annals of the Rheumatic Diseases. 75, 2016, pp. 1187-1195; Armaka, et al.,. J. Exp. Med. 205, 331-337, 2008). Thus this genetic cross is expected to result in ER-2Lec being expressed mostly in SFs and reduce Tn levels in arthritic mice. We induced CAIA in these mice and monitored ER-2Lec-GFP expression and Tn levels in the pannus after 7 days. Tn was markedly reduced, consistent with GALA inhibition (Fig. 8).

We monitored RA symptoms in Col6a1Cre ER-2Lec animals and in Cre expressing controls. The ER-2Lec expressing animals displayed a marked reduction of swelling in the paws (Fig. 9A and B). We measured the change in paw thickness over time and observed significant reduced thickness in ER-2Lec animals as compared to controls. We also used the internationally defined arthritis score in a blinded evaluation and observed a consistent reduction of symptoms in Col6a1Cre ER-2Lec animals (Fig. 10).

We also performed histological analysis on day 7 using H&E and the alcian blue (AB) and safranin-O (SO) stains, which are commonly used to reveal the cartilage fraction of joints. H&E staining of Col6a1Cre ER-2Lec joints showed a reduction of pannus size, consistent with the reduction of swelling (Fig. 11). Interestingly, the infiltration of immune cells seemed much reduced in the ER-2Lec expressing animals (Fig. 11 and fig. 25A). The AB positive region was also significantly preserved in CAIA Col6a1Cre ER-2Lec animals (Fig. 11). Significant change was also obtained after quantification of SO stains (Fig. 25). Taken together, our results demonstrate that inhibiting GALA with the ER-2Lec protein in SF can limit arthritis disease progression.

### GALA activates CNX glycosylation and surface exposure in SF

We recently described Calnexin (CNX) as a glycosylation target and effector of GALA (Ros et al., Nat. Cell Biol. 22, 1371-1381, 2020). Upon glycosylation, CNX is translocated at the cell surface and, in conjunction with PDIA3, mediates the cleavage of disulfide bonds in ECM proteins. This reductive activity is essential for matrix degradation by cancer cells (Ros et al., Nat. Cell Biol. 22, 1371-1381, 2020). In SW982 SF, we found that CNX is hyper-glycosylated by ~6 fold after stimulation with cytokines and ECM (Fig. 12A and B). This glycosylation was GALA dependent as expression of ER-2Lec was able to significantly reduce CNX glycosylation.

In addition, we found, using FACS, that CNX surface expression increased significantly by about 10 % after stimulation of SW982 SF cells with CYTO and ECM (Fig. 13A and B). Strikingly, the increase in cell surface CNX signal was fully repressed by ER-2Lec expression (Fig. 13A and B).

We sought to confirm this result in primary cells obtained from patients. In SFs from healthy controls (HCSF), the proportion of cell surface CNX positive cells was only ~7% and stimulation with cytokines and ECM increased it slightly (Fig. 14A and B). By contrast, SF cells from the patient suffering from RA (RASF) or OA (OASF) displayed significantly increased levels and were more sensitive to stimulation, with a three fold increase in the percentage of cells with cell surface CNX (Fig. 14A and B). Overall, these results indicate that CNX glycosylation and its cell surface exposure is enhanced in arthritic SFs and is dependent on GALA.

### Anti-CNX antibodies prevent arthritic symptoms in CAIA mice

We have previously shown that antibodies against Calnexin can block ECM degradation by preventing the necessary reduction of disulfide bonds (Ros et al., Nat. Cell Biol. 22, 1371-1381, 2020). We hypothesized that blocking Calnexin would similarly prevent cartilage ECM degradation. We tested first for the presence of disulfide bonds in cartilage ECM using a previously described method (Ros et al., Nat. Cell Biol. 22, 1371-1381, 2020). Briefly, cartilage ECM was reduced with TCEP, then exposed to N-Ethylmaleimide (NEM) and then treated with the anti-OX133 antibody. As described previously with liver ECM, we observed an abundant OX133 signal colocalizing with collagen 3/collagen 1 and fibronectin/collagen 1 fibers, suggesting cartilage ECM is heavily cross-linked with disulfide bonds (Fig. 15).

We next tested the effect of anti-CNX antibodies on ECM degradation. OASF cells seeded on cartilage ECM covering fluorescent gelatin were allowed to degrade ECM overnight. The addition of a polyclonal anti-CNX antibody blocked this degradative activity (Fig. 16 and B).

Encouraged by these results, we aimed to treat animals with the anti-CNX antibody. We first monitored the weight of animals receiving three injections with the antibody over 10 days; we did not detect any weight loss (Fig. 22). Next, we treated CAIA animals with the anti-CNX antibody, injecting 25 micrograms every two days from day 3 till day 7 after initiation of CAIA (Fig. 17A). We monitored paw thickness at regular intervals and measured arthritic score at day 10. Strikingly, the paws exhibited reduced swelling in anti-CNX treated animals as compared to control animals treated with an isotype antibody (Fig. 17B). While some redness and swelling in the fingers still occurred, raising the arthritic score, the mean score of treated animals was half of CAIA control animals (Fig. 17C). This is reminiscent of the results obtained with ER-2Lec expression.

At the histological level, the reduction in SO positive cartilage was very pronounced in control animals at day 10 (Fig. 18). In addition, the synovium had adhered to the underlying bone, possibly indicating the onset of bone remodelling. By contrast, anti-CNX treated animals had a well-preserved joint cavity with abundant cartilage remaining (Fig 18).

Our working hypothesis is that the Cnx antibody bound to lining synovial fibroblasts and inhibited their degradative activity. To test whether the antibody had indeed interacted with these cells, we stained the joints of treated animals with an anti-rabbit IgG. A signal in cells of the synovium of animals treated with anti-CNX antibody was clearly detectable, where no signal appeared in animals treated with a control rabbit IgG (Fig. 19C).

Overall, these results indicate that inhibiting CNX results in a potent inhibition of cartilage ECM degradation and could form the basis of an arthritis therapeutics.

### EXAMPLE 3

### Discussion

In this study, we show that arthritic synovial fibroblasts have markedly up-regulated GalNac O-glycosylation compared to healthy counterparts. This increase is due to the activation of the GALA pathway, with relocation of GALNTs from the Golgi to the ER.

In cancer cells, activation of EGF-R and in particular the Src kinase drive GALA (Gill et al., J. Cell Biol. 189, 843-858, 2010; Chia et al., PLoS One. 14, e0214118, 2019). Other signaling molecules, such as the ERK8 kinase, constitutively and dynamically inhibit the pathway (Chia et al., Elife. 3, e01828, 2014). In vitro, RA and OA patient derived fibroblasts have moderately elevated GALA levels compared to healthy human SFs. However, RA fibroblasts activate GALA in response to an IL-1beta and TNF-alpha cytokine mixture. Interestingly, RA SFs response is more marked than normal or OA SFs, suggesting RA SFs have been primed to respond to these cytokines. IL-1β has been reported to activate the tyrosine kinase Src (Mon et al., Oncol. Lett. 13, 955-960, 2017), suggesting a possible link between the cytokine and GALA.

Exposure to ECM strongly activates GALA in both OA and RA fibroblasts, more readily than in the healthy control cells. It was proposed previously that adhesion of SFs to elements of cartilage ECM is involved in arthritis development (Pap et al., Arthritis Res. 2, 361-367, 2000). Injection of fibronectin in joints leads to the degradation of cartilage proteoglycans (Homandberg, et al., J. Rheumatol. 20, 1378-1382, 1993). Integrins, the fibronectin receptors, are also activators of the Src kinase (Shattil, Trends Cell Biol. 15, 399-403, 2005; Huveneers, and Danen, J. Cell Sci. 122, 1059-1069, 2009). Thus, a signaling cascade could link external ECM signals to integrins, Src and then GALA, activating ECM degradation (Gill et al., J. Cell Biol. 189, 843-858, 2010). This hypothetical cascade would feed a pathological positive feedback loop. It remains unclear why GALA response to ECM in healthy SFs is much more limited. SFs from arthritic joints have been proposed to be epigenetically primed for degradation (Nygaard et al., Nat. Rev. Rheumatol. 16, 316-333 (2020). Indeed, OASF and RASF display comparable global methylation profiles, which are distinct from SF from healthy subjects (Nakano et al., Ann. Rheum. Dis. 72, 110-117, 2013). Some differences were identified in genes involved in PDGF and EGF signalling, also regulators of GALA (Chia et al., PLoS One. 14, e0214118, 2019). Thus, an epigenetic priming could include higher propensity to activate GALA. In addition, GALA glycosylation is probably synergistic with other regulatory mechanisms. For instance, we found increased levels of CNX in the synovial tissues of arthritic mice, consistent with gene expression data reported in previous studies (Broeren et al., PLoS One. 11, e0167076, 2016; Nzeusseu Toukap, et al., Arthritis Rheum. 56, 1579-1588, 2007). GALNT1, 3 and 5 were found upregulated in these studies and we also observed increased expression of GALNT1 and 2.

Whether activated by immune signals or by ECM proteins, GALA glycosylation may not be activated continuously during arthritis. Indeed, in the CAIA mouse model, the levels of GALA significantly decreased at day 10, preceded by the full recovery of the animals (at day 14 or later). In patient samples, GALA is detectable in samples of OA, RA and psoriasis arthritis, but a significant fraction of samples display low GALA levels. This suggests that GALA is only fully activated during the active ECM degradative phase of the disease, a phase that would correspond to flares in patients. By contrast, in phases of remission, there is less ECM degradation and correspondingly low GALA levels.

Among the targets of GALA glycosylation is MMP14, a cell surface protease that degrades collagen fibers and activates other MMPs (Nguyen, et al., Cancer Cell. 32, 639-653.e6, 2017, Gialeli, et al., FEBS J. 278, 16-27, 2011). MMP14 is one of the MMPs involved in arthritis, and activates MMP-2 and 13 (Rose and Kooyman, Dis. Markers. 2016, 4895050, 2016). MMP14 O-glycosylation is essential for its protease activity and occurs in a low complexity region of the protein in the form of a cluster: six or more amino-acids are modified with GalNAc or more complex O-glycans (Nguyen et al., Cancer Cell. 32, 639-653.e6 (2017).

Calnexin also displays a clustered glycosylation pattern, located in the N-terminal region (Ros et al., Nat. Cell Biol. 22, 1371-1381, 2020). Clustered glycosylation is a frequent feature of GalNac glycosylation, exemplified in mucin proteins. The ER-2Lec chimeric protein inhibits this clustered glycosylation (Gill et al., Proc. Natl. Acad. Sci. U. S. A. 110, E3152-61, 2013). As in cancer cells, ER-2Lec reduced the levels of Tn signal in SFs. Thus, it is likely to inhibit at least partially MMP14 and Cnx glycosylation inhibiting ECM degradation by SFs in vitro and in vivo. As GALNTs act on thousands of proteins and preliminary, unpublished data indicate that GALA affects many proteins, additional glycoproteins may be involved in the pathological activity of SFs and affected by ER-2Lec (Steentoft, et al., Nat. Methods. 8, 977-982, 2011).

ER-2Lec activated by Cre under a Collagen type VI promoter results in CAIA-treated mice being protected from the loss of cartilage. ER-2Lec expression was mostly restricted to SFs, with no expression detected in immune cells. Interestingly, ER-2Lec expression reduced swelling and inflammation of the joint. Effective protection of the cartilage ECM might reduce SFs activation, preventing release of cytokines and thus inflammation. The fact that anti-Cnx antibody treatment also reduces inflammation supports this explanation.

The role of Calnexin in the degradation of ECM was only recently established. In complex with PDIA3, Calnexin participates in the reduction of disulfide bonds in liver ECM proteins (Ros et al., Nat. Cell Biol. 22, 1371-1381, 2020). Disulfide bonds, like other cross-linking bonds, prevent the action of proteases (Philp et al., Am. J. Respir. Cell Mol. Biol. 58, 594-603, 2018). The cartilage ECM contains an abundance of disulfide bonds. Anti-Calnexin antibodies blocked matrix degradation by SFs in vitro and provided a significant protection of cartilage ECM in animals.

Other strategies to inhibit synoviocytes have been developed, such as targeting the adhesion molecule Cadherin 11 (Lee, et al., Science. 315, 1006-1010, 2007; Kiener, et al., Arthritis Rheum. 60, 1305-1310, 2009). More recently, targeting the tyrosine phosphatase PTPRS at the cell surface of SFs has also been shown to protect the cartilage in RA mice (Svensson, et al., Sci Adv. 6, eaba4353, 2020). In addition, the targeting of MMPs has been researched for several decades and specific MMP inhibitors such as Trocade have demonstrated protective effects against RA and OA in animal models (Lewis et al. Br. J. Pharmacol. 121, 540-546 (1997; Brewster, et al., Arthritis Rheum. 41, 1639-1644, 1998). Poor tolerability of these compounds led to clinical trial failures (Close. Ann. Rheum. Dis. 60 Suppl 3, iii62-7 (2001). To date, while some progress has been made, inhibiting MMP therapeutically remains relatively challenging (Fields. Cells. 8. 2019, doi:10.3390/cells8090984). Targeting the Calnexin-ERp57 complex with antibodies may represent a more attractive approach as less toxicity is expected.

Overall, our data opens perspectives for biomarker discovery and a new therapeutic approach targeting Cnx with antibodies. More generally, our results suggest that activation of O-glycosylation through GALA is a critical control switch for ECM degradation in synovial fibroblasts as in cancer cells, indicating the broad pathological relevance of the pathway.

### EXAMPLE 4

### Materials and methods - Isolation and characterisation of 2G9 monoclonal anti-Calnexin antibody

### Identification and cloning of 2G9 by phage display:

To select for an antibody against Calnexin, we screened a library of human Fab domains cloned in a phage display library. We isolated clones with affinity for Calnexin. We selected the clone 2G9 for further characterization.

Anti-Cnx antibodies were isolated from HX02 human Fab phage display library (Humanyx Pte Ltd) via in vitro selection. We followed the procedures of biopanning, phage amplification, Fab expression and purification described by de Haard et al. (J Biol Chem 1999; 274:18218 - 30; http://dx.doi.org/10.1074/jbc.274.26.18218; PMID: 10373423). Briefly, biopanning was performed using biotinylated human calnexin. In the first two rounds of biopanning, calnexin was immobilized on M280 streptavidin-coated magnetic beads (Life Technologies); in the third round, biotinylated calnexin was immobilized on the neutravidin-coated microplate in order to avoid isolation of streptavidin magnetic beads binders. About a thousand cfu phage in 1 mL casein-PBS blocking buffer was used in the first round, and a thousand cfu phage were used in the second and third rounds. After three rounds of biopanning, the Fab of selected clones was expressed in E. coli TG1 cells (Stratagene) to screen for calnexin binders by ELISA.

Clone 2G9 was identified by DNA fingerprinting, confirmed by DNA sequencing, and converted into IgG1 and IgG4 formats for further analysis. Briefly, the Fab was amplified by PCR and cloned in frame with the human Fc region of the respective IgG. The plasmid was then amplified and 2G9 was expressed by transient transfection in 293T cells.

### ELISA assay:

100ng of recombinant CNX-His protein diluted in 50mM sodium carbonate, pH9.6 buffer was coated on 96-well maxisorp plates (Nunc) overnight in 4°C . Negative control wells were similarly coated with 0.5ug/ml bovine serum albumin (BSA) on maxisorp plates (Nunc) overnight in 4°C. The coated wells were subsequently washed with 0.05% PBS-Tween (PBST) and PBS 3 times each. The wells were blocked with 5% fetal bovine serum (FBS) for 1hour at 37°C. After blocking, primary antibody were added to wells and incubated for 2hours at 37°C. The wells were washed with PBST and PBS 3 times each. Wells were added with secondary antibody conjugated with horseradish peroxidase (HRP) in 5%FBS-PBST and incubated for 1hour at 37°C. The wells were washed with PBST and PBS 3 times each. To detect HRP, wells were added with TMB substrate and incubated for 5 minutes before the addition of 1M hydrochloric acid to stop the reaction. Measure OD450 on Tecan microplate reader.

### ECM degradation assay:

Gelatin (G1393, Sigma) was first coupled to 5-carboxy-X-rhodamine succinimidyl ester (C-6125, ThermoFisher Scientific). Sterile coverslips were coated with gelatin for 20 min, then fixed with 0.5% glutaraldehyde (15960, Electron Microscopy Sciences) for 40 min. After washing, a layer of 0.5 mg ml⁻¹ type I collagen (354236 rat tail, Corning) was coated onto these gelatin coverslips and incubated for 4 h at 37 °C before cell seeding. Cells were incubated overnight before fixation and staining. A total of 10-30 images were acquired for each condition, and experiments were done in three biological replicates. The area of degradation was quantified using ImageJ and normalized to the number of nuclei in each image.

### EXAMPLE 5

### Results - Isolation and characterisation of 2G9 monoclonal anti-Calnexin antibody

### 2G9 has high binding affinity and specificity for Calnexin:

ELISA assays were utilised to test 2G9 binding affinity. Purified His-tagged Calnexin was adsorbed and coated on plastic wells; control wells were coated with BSA. Then, we incubated 2G9, a control human IgG or the commercial monoclonal antibody ab10286 on the plates at 10 micrograms/ml. The assay revealed high specificity for Cnx for both antibodies, with little binding to BSA coated wells (Figure 26). The negative control hIgG did not bind significantly to Calnexin.

To quantitatively compare 2G9 and ab10286, we repeated the ELISA assay using serial dilutions of the antibodies (Figure 27). 2G9 displayed saturated binding at 0.1 micrograms/ml, with a calculated EC50 of ~3. 10e-6 micrograms/ml. This concentration is lower than the commercial monoclonal antibody ab10286, suggesting a higher affinity and/or avidity.

### 2G9 prevents ECM degradation by HUH7 liver cancer cells

The ability of 2G9 in IgG1 format to prevent ECM degradation was assessed through the use of a degradation assay, with rat tail ECM overlaid on a thin layer of fluorescently labelled gelatin. With active ECM degradation, cells degrade the ECM, then the gelatin, creating dark spots in the gelatin layer that can be measured (Figure 28A). HUH7 cells were seeded on top and incubated with 2G9 or control antibody for 48h. While the control IgG had no effect on matrix degradation or even seemed to stimulate it, 2G9 was able to reduce by 90% ECM degradation compared to untreated controls (Figure 28B).

### 2G9 in IgG4 format is able to block ECM degradation:

The 2G9 antibody was converted into IgG4 format. We re-tested the ECM protective activity using the gelatin overlay assay. As was found for the IgG1 format, we also found that 2G9 was able to protect ECM degradation (Figure 29). This effect was most apparent at 20 ug/ml.

**Table of sequences**

| **ER-2Lec sequence (SEQ ID NO: 1)** | | | | | |
|---|---|---|---|---|---|
| AATGTAGTCTTATGCAATACTCTTGTAGTCTTGCAACATGGTAACGATGAGTTAGCAACAT GCCTTACAAGGAGAGAAAAAGCACCGTGCATGCCGATTGGTGGAAGTAAGGTGGTACGA TCGTGCCTTATTAGGAAGGCAACAGACGGGTCTGACATGGATTGGACGAACCACTGAATT GCCGCATTGCAGAGATATTGTATTTAAGTGCCTAGCTCGATACATAAACGGGTCTCTCTGG TTAGACCAGATCTGAGCCTGGGAGCTCTCTGGCTAACTAGGGAACCCACTGCTTAAGCCT CAATAAAGCTTGCCTTGAGTGCTTCAAGTAGTGTGTGCCCGTCTGTTGTGTGACTCTGGTA ACTAGAGATCCCTCAGACCCTTTTAGTCAGTGTGGAAAATCTCTAGCAGTGGCGCCCGAA CAGGGACTTGAAAGCGAAAGGGAAACCAGAGGAGCTCTCTCGACGCAGGACTCGGCTTG CTGAAGCGCGCACGGCAAGAGGCGAGGGGCGGCGACTGGTGAGTACGCCAAAAATTTTG ACTAGCGGAGGCTAGAAGGAGAGAGATGGGTGCGAGAGCGTCAGTATTAAGCGGGGGAG AATTAGATCGCGATGGGAAAAAATTCGGTTAAGGCCAGGGGGAAAGAAAAAATATAAAT TAAAACATATAGTATGGGCAAGCAGGGAGCTAGAACGATTCGCAGTTAATCCTGGCCTGT TAGAAACATCAGAAGGCTGTAGACAAATACTGGGACAGCTACAACCATCCCTTCAGACAG GATCAGAAGAACTTAGATCATTATATAATACAGTAGCAACCCTCTATTGTGTGCATCAAA GGATAGAGATAAAAGACACCAAGGAAGCTTTAGACAAGATAGAGGAAGAGCAAAACAA AAGTAAGACCACCGCACAGCAAGCGGCCGCTGATCTTCAGACCTGGAGGAGGAGATATG AGGGACAATTGGAGAAGTGAATTATATAAATATAAAGTAGTAAAAATTGAACCATTAGG AGTAGCACCCACCAAGGCAAAGAGAAGAGTGGTGCAGAGAGAAAAAAGAGCAGTGGGA ATAGGAGCTTTGTTCCTTGGGTTCTTGGGAGCAGCAGGAAGCACTATGGGCGCAGCGTCA ATGACGCTGACGGTACAGGCCAGACAATTATTGTCTGGTATAGTGCAGCAGCAGAACAAT TTGCTGAGGGCTATTGAGGCGCAACAGCATCTGTTGCAACTCACAGTCTGGGGCATCAAG CAGCTCCAGGCAAGAATCCTGGCTGTGGAAAGATACCTAAAGGATCAACAGCTCCTGGGG ATTTGGGGTTGCTCTGGAAAACTCATTTGCACCACTGCTGTGCCTTGGAATGCTAGTTGGA GTAATAAATCTCTGGAACAGATTTGGAATCACACGACCTGGATGGAGTGGGACAGAGAA ATTAACAATTACACAAGCTTAATACACTCCTTAATTGAAGAATCGCAAAACCAGCAAGAA AAGAATGAACAAGAATTATTGGAATTAGATAAATGGGCAAGTTTGTGGAATTGGTTTAAC ATAACAAATTGGCTGTGGTATATAAAATTATTCATAATGATAGTAGGAGGCTTGGTAGGT TTAAGAATAGTTTTTGCTGTACTTTCTATAGTGAATAGAGTTAGGCAGGGATATTCACCAT TATCGTTTCAGACCCACCTCCCAACCCCGAGGGGACCCGACAGGCCCGAAGGAATAGAAG AAGAAGGTGGAGAGAGAGACAGAGACAGATCCATTCGATTAGTGAACGGATCTCGACGG TATCGGTTAACTTTTAAAAGAAAAGGGGGGATTGGGGGGTACAGTGCAGGGGAAAGAAT AGTAGACATAATAGCAACAGACATACAAACTAAAGAATTACAAAAACAAATTACAAAAA TTCAAAATTTTATCGATAAGCTTGGGAGTTCCGCGTTACATAACTTACGGTAAATGGCCCG CCTGGCTGACCGCCCAACGACCCCCGCCCATTGACGTCAATAATGACGTATGTTCCCATAG TAACGCCAATAGGGACTTTCCATTGACGTCAATGGGTGGAGTATTTACGGTAAACTGCCC ACTTGGCAGTACATCAAGTGTATCATATGCCAAGTACGCCCCCTATTGACGTCAATGACG GTAAATGGCCCGCCTGGCATTATGCCCAGTACATGACCTTATGGGACTTTCCTACTTGGCA GTACATCTACGTATTAGTCATCGCTATTACCATGGTGATGCGGTTTTGGCAGTACATCAAT GGGCGTGGATAGCGGTTTGACTCACGGGGATTTCCAAGTCTCCACCCCATTGACGTCAAT GGGAGTTTGTTTTGGCACCAAAATCAACGGGACTTTCCAAAATGTCGTAACAACTCCGCC CCATTGACGCAAATGGGCGGTAGGCGTGTACGGTGGGAGGTCTATATAAGCAGAGCTCGT TTAGTGAACCGTCAGATCGCCTGGAGACGCCATCCACGCTGTTTTGACCTCCATAGAAGA CACCGGGACCGATCCAGCCTCCGGACTCTAGAGGATCCCTACCGGTGATATCCTCGAGAC Catggctacaggctcccggacgtccctgctcctggcttttggcctgctctgcctgccctggcttcaagagggcagtgccttcccaaccattcccttatc cGTGAGCAAGGGCGAGGAGCTGTTCACCGGGGTGGTGCCCATCCTGGTCGAGCTGGACGG CGACGTAAACGGCCACAAGTTCAGCGTGTCCGGCGAGGGCGAGGGCGATGCCACCTACG GCAAGCTGACCCTGAAGTTCATCTGCACCACCGGCAAGCTGCCCGTGCCCTGGCCCACCC TCGTGACCACCTTCACCTACGGCGTGCAGTGCTTCGCCCGCTACCCCGACCACATGAAGCA GCACGACTTCTTCAAGTCCGCCATGCCCGAAGGCTACGTCCAGGAGCGCACCATCTTCTTC AAGGACGACGGCAACTACAAGACCCGCGCCGAGGTGAAGTTCGAGGGCGACACCCTGGT GAACCGCATCGAGCTGAAGGGCATCGACTTCAAGGAGGACGGCAACATCCTGGGGCACA | | | | | |
| AGCTGGAGTACAACTACAACAGCCACAAGGTCTATATCACCGCCGACAAGCAGAAGAAC GGCATCAAGGTGAACTTCAAGACCCGCCACAACATCGAGGACGGCAGCGTGCAGCTCGCC GACCACTACCAGCAGAACACCCCCATCGGCGACGGCCCCGTGCTGCTGCCCGACAACCAC TACCTGAGCACCCAGTCCGCCCTGAGCAAAGACCCCAACGAGAAGCGCGATCACATGGTC CTGCTGGAGTTCGTGACCGCCGCCGGGATCACTCTCGGCATGGACGAGCTGTACAAGGGC TCGAGCCCATCAACAAGTTTGTACAAAAAAGCAGGCTccGATAACGGGAAGCCTATCCCTA ACCCTCTCCTCGGTCTCGATTCTACGgagttaagggttccagaccatcaggatatagcttttggggccttgcagcagggaact aactgcctcgacactttgggacactttgctgatggtgtggttggagtttatgaatgtcacaatgctgggggaaaccaggaatggccttgacgaagga gaagtcggtgaagcacatggatttgtgccttactgtggtggaccgggcaccgggctctcttataaagctgcagggctgccgagaaaatgacagcag acagaaatgggaacagatcgagggcaactccaagctgaggcacgtgggcagcaacctgtgcctggacagtcgcacggccaagagcgggggcct aagcgtggaggtgtgtggcccggccctttcgcagcagtggaagttcacgctcaacctgcagcagGCACAGGGAGCCCAAGGGA GATCCGATAACGGGAAGCCTATCCCTAACCCTCTCCTCGGTCTCGATTCTACGgagttaagggttc cagaccatcaggatatagcttttggggccttgcagcagggaactaactgcctcgacactttgggacactttgctgatggtgtggttggagtttatgaatg tcacaatgctgggggaaaccaggaatggccttgacgaaggagaagtcggtgaagcacatggatttgtgccttactgtggtggaccgggcaccgg gctctcttataaagctgcagggctgccgagaaaatgacagcagacagaaatgggaacagatcgagggcaactccaagctgaggcacgtgggcag caacctgtgcctggacagtcgcacggccaagagcgggggcctaagcgtggaggtgtgtggcccggccctttcgcagcagtggaagttcacgctca acctgcagcagAAAGACGAGCTGtagGACCCAGCTTTCTTGTACAAAGTGGTTGATATCCAGCAC AGTGGCGGCCGCTCGAGTCTAGAGGGCCCGCGGTTCGAAGGTAAGCCTATCCCTAACCCT CTCCTCGGTCTCGATTCTACGCGTACCGGTTAGTAATGATCGACAATCAACCTCTGGATTA CAAAATTTGTGAAAGATTGACTGGTATTCTTAACTATGTTGCTCCTTTTACGCTATGTGGA TACGCTGCTTTAATGCCTTTGTATCATGCTATTGCTTCCCGTATGGCTTTCATTTTCTCCTCC TTGTATAAATCCTGGTTGCTGTCTCTTTATGAGGAGTTGTGGCCCGTTGTCAGGCAACGTG GCGTGGTGTGCACTGTGTTTGCTGACGCAACCCCCACTGGTTGGGGCATTGCCACCACCTG TCAGCTCCTTTCCGGGACTTTCGCTTTCCCCCTCCCTATTGCCACGGCGGAACTCATCGCCG CCTGCCTTGCCCGCTGCTGGACAGGGGCTCGGCTGTTGGGCACTGACAATTCCGTGGTGTT GTCGGGGAAGCTGACGTCCTTTCCATGGCTGCTCGCCTGTGTTGCCACCTGGATTCTGCGC GGGACGTCCTTCTGCTACGTCCCTTCGGCCCTCAATCCAGCGGACCTTCCTTCCCGCGGCC TGCTGCCGGCTCTGCGGCCTCTTCCGCGTCTTCGCCTTCGCCCTCAGACGAGTCGGATCTC CCTTTGGGCCGCCTCCCCGCCTGGCGATGGTACCGGTGTGGAAAGTCCCCAGGCTCCCCA GCAGGCAGAAGTATGCAAAGCATGCATCTCAATTAGTCAGCAACCAGGTGTGGAAAGTCC CCAGGCTCCCCAGCAGGCAGAAGTATGCAAAGCATGCATCTCAATTAGTCAGCAACCATA GTCCCGCCCCTAACTCCGCCCATCCCGCCCCTAACTCCGCCCAGTTCCGCCCATTCTCCGC CCCATGGCTGACTAATTTTTTTTATTTATGCAGAGGCCGAGGCCGCCTCTGCCTCTGAGCT ATTCCAGAAGTAGTGAGGAGGCTTTTTTGGAGGCCTAGGCTTTTGCAAAAAGCTCCCGGG AGCTTGTATATCCATTTTCGGATCTGATCAGCACGTGTTGACAATTAATCATCGGCATAGT ATATCGGCATAGTATAATACGACAAGGTGAGGAACTAAACCATGGCCAAGCCTTTGTCTC AAGAAGAATCCACCCTCATTGAAAGAGCAACGGCTACAATCAACAGCATCCCCATCTCTG AAGACTACAGCGTCGCCAGCGCAGCTCTCTCTAGCGACGGCCGCATCTTCACTGGTGTCA ATGTATATCATTTTACTGGGGGACCTTGTGCAGAACTCGTGGTGCTGGGCACTGCTGCTGC TGCGGCAGCTGGCAACCTGACTTGTATCGTCGCGATCGGAAATGAGAACAGGGGCATCTT GAGCCCCTGCGGACGGTGCCGACAGGTGCTTCTCGATCTGCATCCTGGGATCAAAGCCAT AGTGAAGGACAGTGATGGACAGCCGACGGCAGTTGGGATTCGTGAATTGCTGCCCTCTGG TTATGTGTGGGAGGGCTAAGCACAATTCGAGCTCGGTACCTTTAAGACCAATGACTTACA AGGCAGCTGTAGATCTTAGCCACTTTTTAAAAGAAAAGGGGGGACTGGAAGGGCTAATTC ACTCCCAACGAAGACAAGATCTGCTTTTTGCTTGTACTGGGTCTCTCTGGTTAGACCAGAT CTGAGCCTGGGAGCTCTCTGGCTAACTAGGGAACCCACTGCTTAAGCCTCAATAAAGCTT GCCTTGAGTGCTTCAAGTAGTGTGTGCCCGTCTGTTGTGTGACTCTGGTAACTAGAGATCC CTCAGACCCTTTTAGTCAGTGTGGAAAATCTCTAGCAGTAGTAGTTCATGTCATCTTATTA TTCAGTATTTATAACTTGCAAAGAAATGAATATCAGAGAGTGAGAGGAACTTGTTTATTG CAGCTTATAATGGTTACAAATAAAGCAATAGCATCACAAATTTCACAAATAAAGCATTTT TTTCACTGCATTCTAGTTGTGGTTTGTCCAAACTCATCAATGTATCTTATCATGTCTGGCTC TAGCTATCCCGCCCCTAACTCCGCCCATCCCGCCCCTAACTCCGCCCAGTTCCGCCCATTC TCCGCCCCATGGCTGACTAATTTTTTTTATTTATGCAGAGGCCGAGGCCGCCTCGGCCTCT GAGCTATTCCAGAAGTAGTGAGGAGGCTTTTTTGGAGGCCTAGGGACGTACCCAATTCGC CCTATAGTGAGTCGTATTACGCGCGCTCACTGGCCGTCGTTTTACAACGTCGTGACTGGGA | | | | | |
| AAACCCTGGCGTTACCCAACTTAATCGCCTTGCAGCACATCCCCCTTTCGCCAGCTGGCGT AATAGCGAAGAGGCCCGCACCGATCGCCCTTCCCAACAGTTGCGCAGCCTGAATGGCGAA TGGGACGCGCCCTGTAGCGGCGCATTAAGCGCGGCGGGTGTGGTGGTTACGCGCAGCGTG ACCGCTACACTTGCCAGCGCCCTAGCGCCCGCTCCTTTCGCTTTCTTCCCTTCCTTTCTCGC CACGTTCGCCGGCTTTCCCCGTCAAGCTCTAAATCGGGGGCTCCCTTTAGGGTTCCGATTT AGTGCTTTACGGCACCTCGACCCCAAAAAACTTGATTAGGGTGATGGTTCACGTAGTGGG CCATCGCCCTGATAGACGGTTTTTCGCCCTTTGACGTTGGAGTCCACGTTCTTTAATAGTG GACTCTTGTTCCAAACTGGAACAACACTCAACCCTATCTCGGTCTATTCTTTTGATTTATAA GGGATTTTGCCGATTTCGGCCTATTGGTTAAAAAATGAGCTGATTTAACAAAAATTTAACG CGAATTTTAACAAAATATTAACGCTTACAATTTAGGTGGCACTTTTCGGGGAAATGTGCGC GGAACCCCTATTTGTTTATTTTTCTAAATACATTCAAATATGTATCCGCTCATGAGACAAT AACCCTGATAAATGCTTCAATAATATTGAAAAAGGAAGAGTATGAGTATTCAACATTTCC GTGTCGCCCTTATTCCCTTTTTTGCGGCATTTTGCCTTCCTGTTTTTGCTCACCCAGAAACG CTGGTGAAAGTAAAAGATGCTGAAGATCAGTTGGGTGCACGAGTGGGTTACATCGAACTG GATCTCAACAGCGGTAAGATCCTTGAGAGTTTTCGCCCCGAAGAACGTTTTCCAATGATG AGCACTTTTAAAGTTCTGCTATGTGGCGCGGTATTATCCCGTATTGACGCCGGGCAAGAGC AACTCGGTCGCCGCATACACTATTCTCAGAATGACTTGGTTGAGTACTCACCAGTCACAGA AAAGCATCTTACGGATGGCATGACAGTAAGAGAATTATGCAGTGCTGCCATAACCATGAG TGATAACACTGCGGCCAACTTACTTCTGACAACGATCGGAGGACCGAAGGAGCTAACCGC TTTTTTGCACAACATGGGGGATCATGTAACTCGCCTTGATCGTTGGGAACCGGAGCTGAAT GAAGCCATACCAAACGACGAGCGTGACACCACGATGCCTGTAGCAATGGCAACAACGTT GCGCAAACTATTAACTGGCGAACTACTTACTCTAGCTTCCCGGCAACAATTAATAGACTG GATGGAGGCGGATAAAGTTGCAGGACCACTTCTGCGCTCGGCCCTTCCGGCTGGCTGGTT TATTGCTGATAAATCTGGAGCCGGTGAGCGTGGGTCTCGCGGTATCATTGCAGCACTGGG GCCAGATGGTAAGCCCTCCCGTATCGTAGTTATCTACACGACGGGGAGTCAGGCAACTAT GGATGAACGAAATAGACAGATCGCTGAGATAGGTGCCTCACTGATTAAGCATTGGTAACT GTCAGACCAAGTTTACTCATATATACTTTAGATTGATTTAAAACTTCATTTTTAATTTAAAA GGATCTAGGTGAAGATCCTTTTTGATAATCTCATGACCAAAATCCCTTAACGTGAGTTTTC GTTCCACTGAGCGTCAGACCCCGTAGAAAAGATCAAAGGATCTTCTTGAGATCCTTTTTTT CTGCGCGTAATCTGCTGCTTGCAAACAAAAAAACCACCGCTACCAGCGGTGGTTTGTTTGC CGGATCAAGAGCTACCAACTCTTTTTCCGAAGGTAACTGGCTTCAGCAGAGCGCAGATAC CAAATACTGTTCTTCTAGTGTAGCCGTAGTTAGGCCACCACTTCAAGAACTCTGTAGCACC GCCTACATACCTCGCTCTGCTAATCCTGTTACCAGTGGCTGCTGCCAGTGGCGATAAGTCG TGTCTTACCGGGTTGGACTCAAGACGATAGTTACCGGATAAGGCGCAGCGGTCGGGCTGA ACGGGGGGTTCGTGCACACAGCCCAGCTTGGAGCGAACGACCTACACCGAACTGAGATAC CTACAGCGTGAGCTATGAGAAAGCGCCACGCTTCCCGAAGGGAGAAAGGCGGACAGGTA TCCGGTAAGCGGCAGGGTCGGAACAGGAGAGCGCACGAGGGAGCTTCCAGGGGGAAACG CCTGGTATCTTTATAGTCCTGTCGGGTTTCGCCACCTCTGACTTGAGCGTCGATTTTTGTGA TGCTCGTCAGGGGGGCGGAGCCTATGGAAAAACGCCAGCAACGCGGCCTTTTTACGGTTC CTGGCCTTTTGCTGGCCTTTTGCTCACATGTTCTTTCCTGCGTTATCCCCTGATTCTGTGGA TAACCGTATTACCGCCTTTGAGTGAGCTGATACCGCTCGCCGCAGCCGAACGACCGAGCG CAGCGAGTCAGTGAGCGAGGAAGCGGAAGAGCGCCCAATACGCAAACCGCCTCTCCCCG CGCGTTGGCCGATTCATTAATGCAGCTGGCACGACAGGTTTCCCGACTGGAAAGCGGGCA GTGAGCGCAACGCAATTAATGTGAGTTAGCTCACTCATTAGGCACCCCAGGCTTTACACTT TATGCTTCCGGCTCGTATGTTGTGTGGAATTGTGAGCGGATAACAATTTCACACAGGAAAC AGCTATGACCATGATTACGCCAAGCGCGCAATTAACCCTCACTAAAGGGAACAAAAGCTG GAGCTGCAAGCTT | | | | | |
| | | | | | |

| **Annotation of the ER-2Lec sequence (SEQ ID NO: 1)** | | | | | |
|---|---|---|---|---|---|
| Name | Range | Number of bases | | | |
| vector | 1..9522 | 9522 | misc_feature | | |
| source | 1..9522 | 9522 | source | | |
| RSV/5' LTR hybrid promoter | 1..410 | 410 | misc_recomb | | |
| promoter | 1..229 | 229 | promoter | | |
| HIV-1 5' LTR | 230..410 | 181 | LTR | | |
| 5' splice donor | 520..520 | 1 | misc_feature | | |
| HIV-1 psi packaging signal | 521..565 | 45 | misc_feature | | |
| HIV-1 Rev response element (RRE) | 1075..1308 | 234 | misc_feature | | |
| 3' splice acceptor | 1656..1656 | 1 | misc_feature | | |
| 3' splice acceptor | 1684..1684 | 1 | misc_feature | | |
| cPPT | 1801..1923 | 123 | misc_feature | | |
| promoter | 1935..2117 | 183 | promoter | | |
| insert | 2118..3411 | 1294 | misc_feature | | |
| promoter | 2118..2452 | 335 | promoter | | |
| KOZAK | 2573..2579 | 7 | RBS | | |
| signal peptide | 2576..2674 | 99 | sig_peptide | | |
| Emerald GFP | 2675..3388 | 714 | N_region | | |
| attB1 site | 3404..3428 | 25 | misc_recomb | | |
| clone | 3412..4392 | 981 | misc_feature | | |
| clone | 3412..3905 | 494 | misc_feature | | |
| insert | 3429..3905 | 477 | misc_feature | | |
| attB 1 | 3429..3430 | 2 | misc_feature | | |
| V5 epitope | 3437..3478 | 42 | misc_feature | | |
| lectin domain ppGalNAcT2 | 3479..3886 | 408 | misc_feature | | |
| clone | 3906..4392 | 487 | misc_feature | | |
| insert | 3906..4382 | 477 | misc_feature | | |
| V5 epitope | 3917..3958 | 42 | misc_feature | | |
| lectin domain ppGalNAcT2 | 3959..4381 | 423 | misc_feature | | |
| attB2 | 4382..4382 | 1 | misc_feature | | |
| attB2 site | 4383..4407 | 25 | misc_recomb | | |
| vector | 4393..3411 | 8541 | misc_feature | | |
| V5 epitope | 4460..4501 | 42 | C_region | | |
| enhancer | 4520..5117 | 598 | enhancer | | |
| promoter | 5128..5436 | 309 | promoter | | |
| promoter | 5491..5557 | 67 | promoter | | |
| Blasticidin resistance gene | 5558..5956 | 399 | gene | | |
| delta-U3/3' LTR | 6042..6276 | 235 | misc_recomb | | |
| Delta U3 | 6042..6095 | 54 | misc_feature | | |
| 3' LTR | 6096..6276 | 181 | LTR | | |
| SV40 polyadenylation signal | 6348..6479 | 132 | polyA_signal | | |
| promoter | | 7338..7436 | 99 | | promoter |
| Amplicillin (bla) resistance gene | | 7437..8297 | 861 | | gene |
| pUC origin | | 8442..9115 | 674 | | rep_origin |
| | | | | | |

| SEQ ID NO: | Comment | | | Sequence | |
|---|---|---|---|---|---|
| 2 | siRNA target sequence for Calnexin (CNX) | | | CAAGAGUGGUCCUAGGAGAUU | |
| 3 | siRNA target sequence for PDIA3 | | | GGAAUAGUCCCAUUAGCAA | |
| 4 | siRNA target sequence for PDIA3 | | | GGGCAAGGACUUACUUAUU | |
| 5 | siRNA target sequence for PDIA3 | | | AGACCCAAAUAUCGUCAUA | |
| 6 | siRNA target sequence for PDIA3 | | | AGGAGUUCUCGCGUGAUG | |
| 7 | siRNA target sequence for PDIA3 | | | GAACGAGUAUGAUGAUAAU | |
| 8 | siRNA target sequence for PDIA3 | | | GGACAAGACUGUGGCAUAU | |
| 9 | siRNA target sequence for PDIA3 | | | GGCAAGGACUUACUUAUUG | |
| 10 | siRNA target sequence for PDIA3 | | | UGAUAAAGAUGCCUCUAUA | |

### References

1. D. J. Gill, J. Chia, J. Senewiratne, F. Bard, Regulation of O-glycosylation through Golgi-to-ER relocation of initiation enzymes. J. Cell Biol. 189, 843-858 (2010).
2. D. J. Gill, K. M. Tham, J. Chia, S. C. Wang, C. Steentoft, H. Clausen, E. A. Bard-Chapeau, F. A. Bard, Initiation of GalNAc-type O-glycosylation in the endoplasmic reticulum promotes cancer cell invasiveness. Proc. Natl. Acad. Sci. U. S. A. 110, E3152-61 (2013).
3. F. Bard, J. Chia, Cracking the Glycome Encoder: Signaling, Trafficking, and Glycosylation. Trends Cell Biol. 26, 379-388 (2016).
4. A. T. Nguyen, J. Chia, M. Ros, K. M. Hui, F. Saltel, F. Bard, Organelle Specific O-Glycosylation Drives MMP14 Activation, Tumor Growth, and Metastasis. Cancer Cell. 32, 639-653.e6 (2017).
5. M. Ros, A. T. Nguyen, J. Chia, S. Le Tran, X. Le Guezennec, R. McDowall, S. Vakhrushev, H. Clausen, M. J. Humphries, F. Saltel, F. A. Bard, ER-resident oxidoreductases are glycosylated and trafficked to the cell surface to promote matrix degradation by tumour cells. Nat. Cell Biol. 22, 1371-1381 (2020).
6. J. Chia, F. Tay, F. Bard, The GalNAc-T Activation (GALA) Pathway: Drivers and markers. PLoS One. 14, e0214118 (2019).
7. J. Chia, K. M. Tham, D. J. Gill, E. A. Bard-Chapeau, F. A. Bard, ERK8 is a negative regulator of O-GalNAc glycosylation and cell migration. Elife. 3, e01828 (2014).

The following numbered clauses contain further statements of various aspects of the present invention:-
1. The use of an anti-calnexin (anti-CNX) antibody for the manufacture of a medicament for the treatment or prevention of cartilage degradation in a subject.
2. An anti-calnexin (anti-CNX) antibody for use in the treatment or prevention of cartilage degradation in a subject.
3. A method of treating or preventing cartilage degradation, wherein the method involves the administration of an anti-calnexin (anti-CNX) antibody to a subject.
4. The use of clause 1, the antibody for use of clause 2, or the method of treatment of clause 3, wherein the subject has osteoarthritis, rheumatoid arthritis, psoriasis arthritis, juvenile idiopathic arthritis (JIA), arthritic flares, bursitis, gout, chondrocalcinosis, fibromyalgia, costochondritis, cartilage damage, or polychondritis.
5. The use of clause 1, antibody for use of clause 2, or method of clause 3, wherein the subject has arthritis.
6. The use, antibody for use, or method of clause 5, wherein the arthritis is osteoarthritis or rheumatoid arthritis.
7. The use, antibody for use, or method of any previous clause, wherein the cartilage degradation is in one or more joints.
8. The use, antibody for use, or method of any previous clause, wherein the cartilage degradation is characterised by increased O-glycosylation and/or GALNT Activation (GALA) in joint tissues, cartilage tissue, and/or synovial fibroblasts.
9. The use, antibody for use, or method of any previous clause, wherein the cartilage degradation is characterised by increased O-glycosylation of calnexin in joint tissues, cartilage tissue, and/or synovial fibroblasts.
10. The use, antibody for use, or method of any previous clause, wherein the cartilage degradation is characterised by increased cell surface expression of calnexin in joint tissues, cartilage tissue, and/or synovial fibroblasts.
11. The use, antibody for use, or method of any previous clause, wherein the cartilage degradation is characterised by extracellular matrix (ECM) degradation.
12. The use, antibody for use, or method of any previous clause, wherein the cartilage degradation is mediated by the activity of synovial fibroblasts.
13. The use, antibody for use, or method of any previous clause, wherein the antibody against Cnx is capable of reducing:
   (a) ECM degradation; or
   (b) an oxireductase activity; or
   (c) a disulphide bond reductase activity.
14. The use, antibody for use, or method of any previous clause, wherein the antibody is antagonistic.
15. The use, antibody for use, or method of any previous clause, wherein the antibody is monoclonal.
16. The use, antibody for use, or method of any previous clause, wherein the therapeutic agent is to be administered together, separately, or sequentially with a further therapeutic agent, wherein the further therapeutic agent is an anti-rheumatic drug.
17. The use, antibody for use, or method of any previous clause, wherein the method comprises intravenous, subcutaneous or intraperitoneal administration of the anti-cnx antibody.
18. The use, antibody for use, or method of any previous clause, wherein the treatment or prevention is in a human subject.
19. The use, antibody for use, or method of any previous clause, wherein a subject is determined to be suitable for treatment with the anti-cnx antibody through the following steps:
   i. detecting the level of Tn antigen, Tn glycan, CNX cell surface expression, CNX glycosylation, and/or ECM degradation in a synovial fibroblast sample;
   ii. comparing the level of Tn antigen, Tn glycan, CNX cell surface expression, CNX glycosylation, and/or ECM degradation in step (i) with the level of Tn antigen/Tn glycan in a synovial fibroblast sample from a control group;
      wherein an increase in the level of Tn antigen, Tn glycan, CNX expression, CNX glycosylation, and/or ECM degradation present in the sample compared to the control group is indicative of the suitability for treatment.
20. A method of detecting the presence or absence of a disorder associated with cartilage degradation in a subject, wherein the method comprises the steps of:
   i. detecting the level of Tn antigen, Tn glycan, CNX cell surface expression, CNX glycosylation, and/or ECM degradation in a synovial fibroblast sample;
   ii. comparing the level of Tn antigen, Tn glycan, CNX cell surface expression, CNX glycosylation, and/or ECM degradation in step (i) with the level of Tn antigen/Tn glycan in a synovial fibroblast sample from a control group;
   wherein an increase in the level of Tn antigen, Tn glycan, CNX expression, CNX glycosylation, and/or ECM degradation present in the sample compared to the control group is indicative of the suitability for treatment.
21. The method of clause 20, wherein the method is for detecting the presence or absence of arthritis in a subject.
22. The method of clause 20 or clause 21, wherein the method is for detecting the presence or absence of osteoarthritis or rheumatoid arthritis in a subject.

## Claims

1. An antagonistic anti-calnexin (anti-CNX) antibody for use in the treatment or prevention of a disorder associated with cartilage degradation.

2. The anti-CNX antibody for use according to claim 1, wherein the disorder associated with cartilage degradation is arthritis.

3. The anti-CNX antibody for use according to claim 2, wherein the arthritis is selected from osteoarthritis and rheumatoid arthritis.

4. The anti-CNX antibody for use according to any one of claims 1 to 3, wherein the cartilage degradation is in one or more joints.

5. The anti-CNX antibody for use according to any one of claims 1 to 4, wherein the disorder associated with cartilage degradation is **characterised by** increased O-glycosylation.

6. The anti-CNX antibody for use according to any one of claims 1 to 5, wherein the disorder associated with cartilage degradation is **characterised by** increased GALNT Activation (GALA).

7. The anti-CNX antibody for use according to any one of claims 1 to 6, wherein the disorder associated with cartilage degradation is **characterised by** increased O-glycosylation and/or GALNT Activation (GALA) in a joint tissue, cartilage tissue, and/or synovial fibroblast.

8. The anti-CNX antibody for use according to any one of claims 1 to 7, wherein the disorder associated with cartilage degradation is **characterised by** increased O-glycosylation of calnexin in a joint tissue, cartilage tissue, and/or synovial fibroblast.

9. The anti-CNX antibody for use according to any one of claims 1 to 8, wherein the disorder associated with cartilage degradation is **characterised by** increased cell surface expression of calnexin in a joint tissue, cartilage tissue, and/or synovial fibroblast.

10. The anti-CNX antibody for use according to any one of claims 1 to 9, wherein the cartilage degradation is **characterised by** extracellular matrix (ECM) degradation.

11. The anti-CNX antibody for use according to any one of claims 1 to 10, wherein the cartilage degradation is mediated by the activity of synovial fibroblasts.

12. The anti-CNX antibody for use according to any one of claims 1 to 11, wherein the antibody is monoclonal.

13. The anti-CNX antibody for use according to any one of claims 1 to 12, wherein the antibody is to be administered together, separately, or sequentially with an anti-rheumatic drug.
